# EUROPEAN PATENT APPLICATION

(11) **EP 4 389 880 A1**
(43) Date of publication of application: **26.06.2024**
(21) Application number: 22858841.4
(22) Date of filing: 18.08.2022
(51) Int. Cl.: C12N 7/00, C12N 15/86, C12N 15/11, C07K 14/005, C12N 15/35

(54) **METHOD OF OBTAINING A MODIFIED ADENO-ASSOCIATED VIRUS CAPSID**

(30) Priority: 20.08.2021 RU 2021124731
(71) Applicant: Joint Stock Company "Biocad", 198515, Saint Petersburg (RU)
(72) Inventor: STRELKOVA, Anna Nikolaevna, Yaransk, Kirovskaya obl., 612261 (RU); LEGOTSKII, Sergei Aleksandrovich, Moscow, 107370 (RU); SHUGAEVA, Tatiana Evgenievna, Moscow, 119021 (RU); GERSHOVICH, Pavel Mikhailovich, Saint-Petersburg, 198328 (RU); NADOLINSKII, Alexandr Anatolevich, Saint-Petersburg, 198206 (RU); IAKOVLEV, Pavel Andreevich, Saint-Petersburg, 190068 (RU); MOROZOV, Dmitry Valentinovich, Saint-Petersburg, 190000 (RU)
(74) Representative: Meissner Bolte Partnerschaft mbB
(86) International application number: PCT/RU2022/050255
(87) International publication number: WO 2023/022631

(57) **Abstract**

The present application relates to the fields of gene therapy and molecular biology. More specifically, the present invention relates to a method for producing a modified adeno-associated virus (AAV) capsid and to a modified AAV capsid produced by said method, as well as to an isolated nucleic acid encoding said modified capsid and a vector based on recombinant adeno-associated virus for delivering a heterologous nucleic acid sequence to a subject, which includes said modified capsid.

## Description

### FIELD OF THE INVENTION

The present application relates to the fields of gene therapy and molecular biology. More specifically, the present invention relates to a method for producing a modified adeno-associated virus (AAV) capsid and to a modified AAV capsid produced by said method, as well as to an isolated nucleic acid encoding said modified capsid and a vector based on recombinant adeno-associated virus for delivering a heterologous nucleic acid sequence to a subject, which includes said modified capsid.

### BACKGROUND OF THE INVENTION

Adeno-associated virus (AAV) is a small (25 nm), independent replication-defective, nonenveloped virus. Many different AAV serotypes have been described in human and primates. The adeno-associated virus genome is composed of (+ or -) single-stranded DNA (ssDNA) being about 4,700 nucleotides long. At the ends of a genomic DNA molecule there are accommodated terminal inverted repeats (ITRs). The genome comprises two open reading frames (ORFs): Rep and Cap comprising several alternative reading frames encoding various protein products. The rep products are essential for AAV replication, whereas three capsid proteins (VP1, VP2, and VP3), along with other alternative products, are encoded by the Cap gene. VP1, VP2, and VP3 proteins are present at 1:1:10 ratio to form an icosahedral capsid (Xie Q. et al. The atomic structure of adeno-associated virus (AAV-2), a vector for human gene therapy. Proc Natl Acad Sci USA, 2002; 99:10405-10410). During recombinant AAV (rAAV) vector production, an expression cassette flanked by ITR is packaged into an AAV capsid. The genes required for AAV replication are not included in the cassette. Recombinant AAV is considered to be one of the safest and most widely used viral vectors for *in vivo* gene transfer. Vectors can infect cells of multiple tissue types to provide strong and sustained transgene expression. They are also non-pathogenic, and have a low immunogenicity profile (High KA et al., "rAAV human trial experience" Methods Mol Biol. 2011; 807:429-57).

One of the urgent purposes of research in the area of development of effective gene therapy is optimization of vectors to improve certain properties of the given vectors.

The various AAV serotypes are characterized by affinity for distinct host cell surface receptors, toward which they have tropism. Thus, the primary known receptor for AAV2 is heparan sulfate proteoglycan, the coreceptors are integrin heterodimer aVβ5, fibroblast growth factor receptor type 1, and hepatocyte growth factor receptor, c-Met. AAV12 binds to heparan sulfate proteoglycans and sialic acid. AAV4 and AAV5 bind to N- and O-linked sialic acids, respectively. AAV5 activates the platelet-derived growth factor receptor. Further, a relationship has been established between the amino acid sequence of AAV capsid proteins and the process of assembly thereof, encapsidation of the genome, affinity for different types of receptors represented on the surface of host cells (Govindasamy L. et. al. Structural insights into adeno-associated virus serotype 5. J Virol. 2013 Oct;87(20):11187-99).

The international application WO2012145601 describes adeno-associated virus (AAV) virions with variant capsid protein, wherein the AAV virions exhibit greater infectivity of retinal cells, when administered via intravitreal injection, compared to wild-type AAV.

The international application WO2013158879 describes an adeno-associated virus (AAV) vector for delivering to a subject a heterologous nucleic acid sequence comprising the capsid protein VP1 comprising one or more lysine substitutions, wherein one lysine substitution is K137R, wherein said lysine substitution is effective for inhibiting ubiquitination of said capsid protein, thereby increasing transduction efficiency of said AVV vector in target cells.

To date, there is a need for AAV with improved properties compared to wild-type AAV, for example, which have increased transduction ability, greater specific ability to transduce cells of target organs and tissues, increased capsid capacity, increased efficiency of packaging of AAV viral genomes, as well as increased efficiency of generation due to highly efficient production (assembly) of encapsidated viral vectors based on recombinant adeno-associated virus (rAAV).

### DESCRIPTION OF THE INVENTION

The authors of the invention have found that the method for producing a modified adeno-associated virus (AAV) capsid according to the invention surprisingly makes it possible to produce a modified AAV capsid having one or more improved properties as compared to AAV capsid free of these modifications (wild-type AAV capsid), which are selected from the group:
- increased efficiency of cell transduction,
- increased production of target protein,
- increased efficiency of generation due to highly efficient production (assembly) of encapsidated viral vectors based on recombinant adeno-associated virus (rAAV).

### Brief description of the invention

In one aspect, the present invention relates to a method for producing a modified AAV capsid, comprising:
a) determining amino acids in capsomere protein of the modified AAV, which are located in the interaction interface region of adjacent pentameric subunits of the capsid;
b) aligning structurally the modified AAV capsid to the template capsid to determine the pairwise correspondence between each amino acid of the modified AAV from the interaction interface region between adjacent pentameric subunits and the amino acid closest thereto of the template capsid, as a structural analogue of the initial amino acid that is considered a potential substitution,
   wherein the template capsid is understood to mean a structurally similar capsid of a virus selected from the family Parvoviridae, which is other than AAV;
c) comparing pairwisely amino acid residues, determined in step b), of the AAV capsid and the template capsid to identify structural differences between the modified AAV capsid and the template capsid in the interaction interface region of adjacent pentameric subunits of the capsid;
d) selecting positions for mutagenesis, wherein the amino acid residues for mutagenesis are located in the interaction interface region of adjacent pentameric subunits of the modified AAV capsid and have a structural difference between the modified AAV capsid and the template capsid in the interaction interface region of adjacent pentameric subunits of the capsid;
e) selecting an amino acid residue for mutagenesis at position selected in step d) using one of the following principles:
   - substitution of the initial amino acid in the capsomere protein of the modified AAV for an amino acid that has a greater volume, other than cysteine and methionine;
   - substitution of the initial amino acid in the capsomere protein of the modified AAV for an amino acid that has an increased or decreased number of polar contacts between the capsomeres, other than cysteine and methionine;
f) introducing one or more amino acid substitutions selected in steps d)-e) in the interaction interface region of adjacent pentameric subunits of the modified AAV capsid.

In some embodiments of the invention, the method for producing a modified AAV capsid further comprises checking the modified AAV capsids produced in step f) with one or more amino acid substitutions for the presence of one or more improved properties as compared to AAV capsid free of these modifications, wherein the one or more improved properties are selected from the group:
- increased efficiency of cell transduction,
- increased production of target protein,
- increased efficiency of generation due to highly efficient production (assembly) of encapsidated viral vectors based on recombinant adeno-associated virus (rAAV).

In some embodiments of the invention, the method for producing a modified AAV capsid further comprises checking the modified AAV capsids produced in step f) for the presence of one or more improved properties as compared to AAV capsid free of these modifications and selecting modified AAV capsids that have one or more improved properties as compared to AAV capsid free of these modifications, wherein the one or more improved properties are selected from the group:
- increased efficiency of cell transduction,
- increased production of target protein,
- increased efficiency of generation due to highly efficient production (assembly) of encapsidated viral vectors based on recombinant adeno-associated virus (rAAV).

In some embodiments of the method for producing an AAV capsid, the modified AAV capsid is selected from the group comprising: human AAV, simian AAV, or avian AAV.

In some embodiments of the method for producing an AAV capsid, the modified AAV capsid is selected from the group comprising the following AAV serotypes: AAV1, AAV2, AAV3, AAV4, AAV5, AAV6, AAV7, AAV8, AAV9, AAV10 AAV11, AAV12, AAV13, AAV14, AAV15 or AAV16.

In some embodiments of the method for producing an AAV capsid, the structurally similar template capsid is selected from the group comprising: parvovirus B19, human bocavirus 1 (HBoV1), bovine parvovirus (BVP).

In one aspect, the present invention relates to a modified AAV capsid for producing viral vectors based on recombinant adeno-associated virus produced by a method comprising:
a) determining amino acids in capsomere protein of the modified AAV, which are located in the interaction interface region of adjacent pentameric subunits of the capsid;
b) aligning structurally the modified AAV capsid to the template capsid to determine the pairwise correspondence between each amino acid of the modified AAV from the interaction interface region between adjacent pentameric subunits and the amino acid closest thereto of the template capsid, as a structural analogue of the initial amino acid that is considered a potential substitution,
   wherein the template capsid is understood to mean a structurally similar capsid of a virus selected from the family Parvoviridae, which is other than AAV;
c) comparing pairwisely amino acid residues, determined in step b), of the AAV capsid and the template capsid to identify structural differences between the modified AAV capsid and the template capsid in the interaction interface region of adjacent pentameric subunits of the capsid;
d) selecting positions for mutagenesis, wherein the amino acid residues for mutagenesis are located in the interaction interface region of adjacent pentameric subunits of the modified AAV capsid and have a structural difference between the modified AAV capsid and the template capsid in the interaction interface region of adjacent pentameric subunits of the capsid;
e) selecting an amino acid residue for mutagenesis at position selected in step d) using one of the following principles:
   - substitution of the initial amino acid in the capsomere protein of the modified AAV for an amino acid that has a greater volume, other than cysteine and methionine;
   - substitution of the initial amino acid in the capsomere protein of the modified AAV for an amino acid that has an increased or decreased number of polar contacts between the capsomeres, other than cysteine and methionine;
f) introducing one or more amino acid substitutions selected in steps d)-e) in the interaction interface region of adjacent pentameric subunits of the modified AAV capsid;
   and, if necessary, further comprising
g) checking the modified AAV capsids produced in step f) for the presence of one or more improved properties as compared to AAV capsid free of these modifications and selecting modified AAV capsids that have one or more improved properties as compared to AAV capsid free of these modifications, wherein the one or more improved properties are selected from the group:
   - increased efficiency of cell transduction,
   - increased production of target protein,
   - increased efficiency of generation due to highly efficient production (assembly) of encapsidated viral vectors based on recombinant adeno-associated virus (rAAV).

In some embodiments of the modified AAV capsid, the modified AAV capsid is selected from the group comprising: human AAV, simian AAV, or avian AAV.

In some embodiments of the modified AAV capsid, the modified AAV capsid is selected from the group comprising the following AAV serotypes: AAV1, AAV2, AAV3, AAV4, AAV5, AAV6, AAV7, AAV8, AAV9, AA V10 AAV11, AAV12, AAV 13, AAV14, AAV15 or AAV16.

In one aspect, the present invention relates to a modified AAV capsid for producing viral vectors based on recombinant adeno-associated virus, which includes a modified AAV capsid protein VP1 having an amino acid sequence that is selected from the group: SEQ ID No: 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, or 368.

In some embodiments of the invention, the modified AAV capsid comprises:
a) a modified AAV capsid protein VP1 having an amino acid sequence that is selected from the group: SEQ ID No: 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, or 368;
b) a modified AAV capsid protein VP2 corresponding to protein VP1 thereof, said modified AAV capsid protein VP2 having an amino acid sequence that is selected from the group: SEQ ID No: 16, 22, 28, 34, 40, 46, 52, 58, 64, 70, 76, 82, 88, 94, 100, 106, 112, 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, or 370;
c) a modified AAV capsid protein VP3 corresponding to protein VP1 thereof, said modified AAV capsid protein VP3 having an amino acid sequence that is selected from the group: SEQ ID No: 18, 24, 30, 36, 42, 48, 54, 60, 66, 72, 78, 84, 90, 96, 102, 108, 114, 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, or 372.

In one aspect, the present invention relates to an isolated nucleic acid that encodes any of the above modified capsids.

In one aspect, the present invention relates to a vector based on recombinant adeno-associated virus for delivering to a subject a heterologous nucleic acid sequence, which includes:
1) any of the above modified capsids, and
2) a heterologous nucleic acid sequence comprising regulatory sequences that promote the expression of the product encoded by the heterologous nucleic acid sequence, in the target cells.

In some embodiments, the vector based on recombinant adeno-associated virus has a product of expression of the heterologous nucleic acid sequence, which is a therapeutic polypeptide or a reporter polypeptide.

### Brief description of drawings

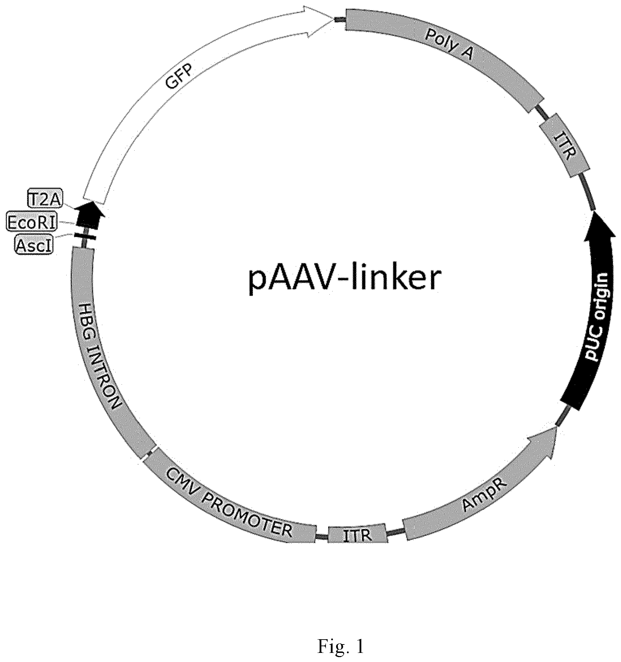

Figure 1 is a circular scheme of plasmid pAAV-linker intended for cloning the libraries of random variants of AAV capsid gene.
   GFP is a sequence encoding green fluorescent protein,
   PolyA is a polyadenylation signal,
   ITR is an inverted terminal repeat of adeno-associated virus,
   T2A is a sequence encoding a self-cleaving peptide from a polypeptide chain from the virus Thosea asigna,
   HBG intron is human beta-globin intron,
   CMVpromoter is human cytomegalovirus promoter,
   AmpR is a sequence of the beta-lactamase gene that provides E.coli resistance to ampicillin,
   pUC origin is a high-copy bacterial replication origin,
   EcoRI is EcoRI restriction endonuclease recognition site,
   AscI is AscI restriction endonuclease recognition site.

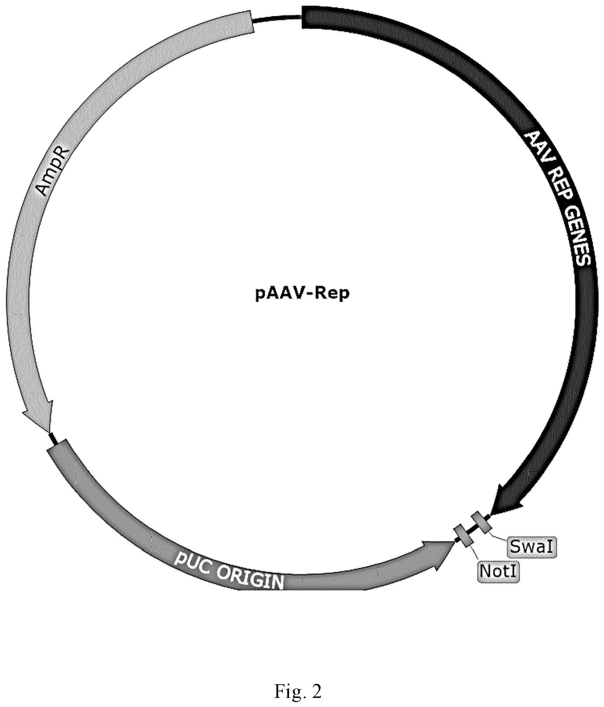

Figure 2 is a circular scheme of plasmid pAAV-Rep intended for producing recombinant viral products of wild-type AAV from the library of random variants.
   AmpR is a sequence of the beta-lactamase gene that provides *E.coli* resistance to ampicillin,
   pUC origin is a high-copy bacterial replication origin,
   AAV Rep genes are a sequence encoding Rep proteins required for the virus life cycle,
   SwaI is SwaI restriction endonuclease recognition site,
   NotI is NotI restriction endonuclease recognition site.

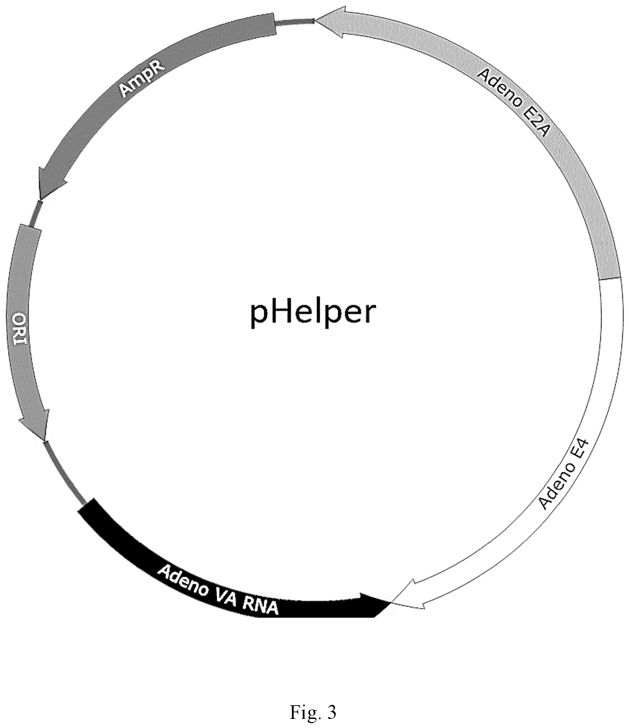

Figure 3 is a circular scheme of plasmid pHelper intended for producing recombinant viral products of wild-type AAV from the library of random variants.
   AmpR is a beta-lactamase gene that provides resistance to ampicillin,
   Ori is a replication origin in bacteria,
   Adeno E2A is a helper adenovirus gene sequence involved in viral DNA replication,
   Adeno E4 is a helper adenovirus gene sequence involved in viral DNA replication,
   Adeno VARNA is a helper adenovirus gene sequence responsible for the translation of both early and late viral genes.

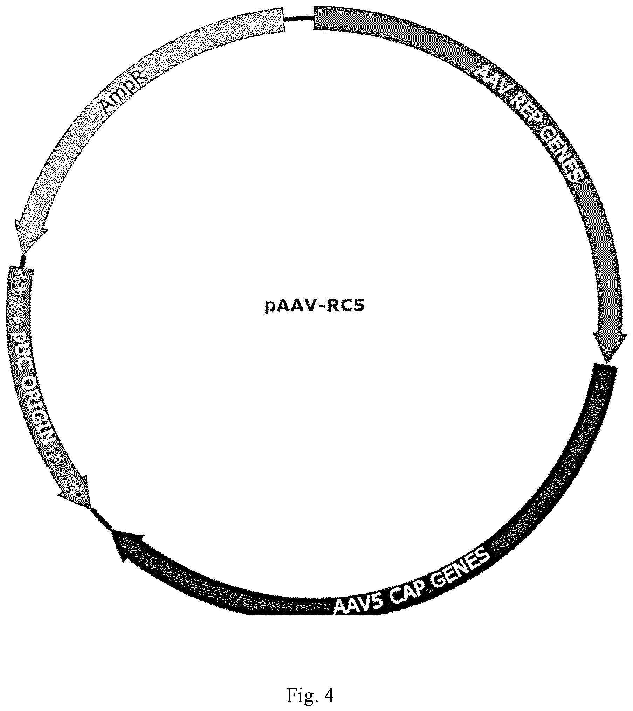

Figure 4 is a circular diagram of the plasmid pAAV-RC5 intended for generating recombinant AAV serotype 5 viral products.
   AmpR is a sequence of the beta-lactamase gene that provides *E.coli* resistance to ampicillin,
   pUC origin is a high-copy prokaryotic replication origin.
   AAV Rep genes are a sequence encoding Rep proteins required for the adeno-associaed virus life cycle,
   AAV5 Cap genes is a sequence encoding overlapping nucleotide sequences of the adeno-associated virus serotype 5 capsid proteins: VP1, VP2 and VP3.

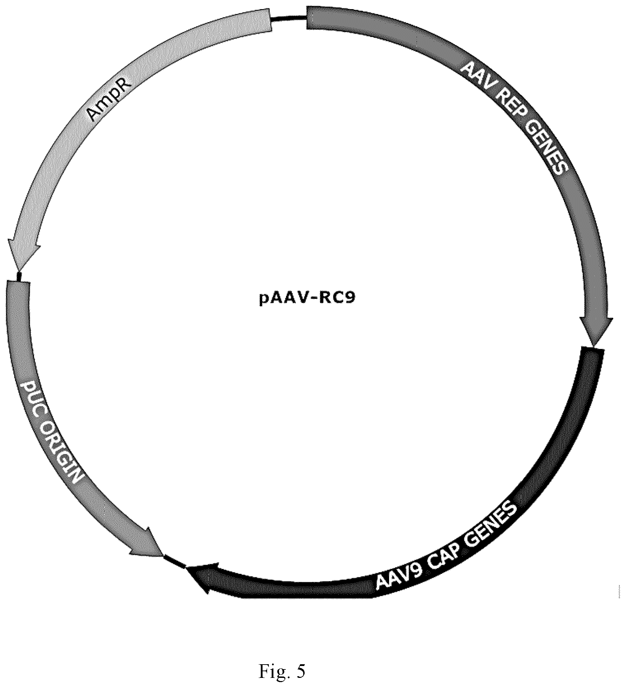

Figure 5 is a circular diagram of the plasmid pAAV-RC9 intended for generating recombinant AAV serotype 9 viral products.
   AmpR is a sequence of the beta-lactamase gene that provides *E.coli* resistance to ampicillin,
   pUC origin is a high-copy prokaryotic replication origin.
   AAV Rep genes are a sequence encoding Rep proteins required for the adeno-associaed virus life cycle,
   AAV9 Cap genes is a sequence encoding overlapping nucleotide sequences of the adeno-associated virus serotype 9 capsid proteins: VP1, VP2 and VP3.

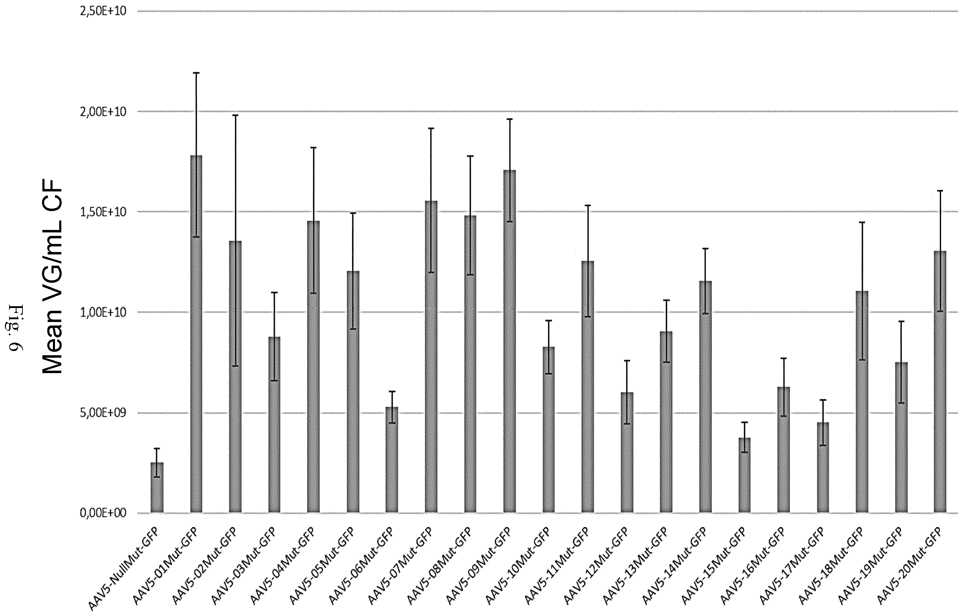

Figure 6 is a graph showing generation efficiency for vectors based on modified adeno-associated virus serotype 5 (rAAV5).
   Vg/ml cf refers to viral genome count per milliliter of culture fluid.
   AAV5-NullMut-GFP refers to a vector based on rAAV5 with a wild-type capsid protein VP1.
   AAV5-01Mut-GFP refers to a vector based on modified adeno-associated virus serotype 5 (rAAV5) comprising a modified AAV5 capsid protein VP1 with the mutations S2A and T711S.
   AAV5-02Mut-GFP refers to a vector based on modified adeno-associated virus serotype 5 (rAAV5) comprising a modified AAV5 capsid protein VP1 with the mutations S2A, G226A and T711S.
   AAV5-03Mut-GFP refers to a vector based on modified adeno-associated virus serotype 5 (rAAV5) comprising a modified AAV5 capsid protein VP1 with the mutations S2A, D286E and T711S.
   AAV5-04Mut-GFP refers to a vector based on modified adeno-associated virus serotype 5 (rAAV5) comprising a modified AAV5 capsid protein VP1 with the mutations S2A, L341Y and T711S.
   AAV5-05Mut-GFP refers to a vector based on modified adeno-associated virus serotype 5 (rAAV5) comprising a modified AAV5 capsid protein VP1 with the mutations S2A, C387V and T711S.
   AAV5-06Mut-GFP refers to a vector based on modified adeno-associated virus serotype 5 (rAAV5) comprising a modified AAV5 capsid protein VP1 with the mutations S2A, Q421H and T711S.
   AAV5-07Mut-GFP refers to a vector based on modified adeno-associated virus serotype 5 (rAAV5) comprising a modified AAV5 capsid protein VP1 with the mutations S2A, P466T and T711S.
   AAV5-08Mut-GFP refers to a vector based on modified adeno-associated virus serotype 5 (rAAV5) comprising a modified AAV5 capsid protein VP1 with the mutations S2A, S594Q and T711S.
   AAV5-09Mut-GFP refers to a vector based on modified adeno-associated virus serotype 5 (rAAV5) comprising a modified AAV5 capsid protein VP1 with the mutations S2A, T614L and T711S.
   AAV5-10Mut-GFP refers to a vector based on modified adeno-associated virus serotype 5 (rAAV5) comprising a modified AAV5 capsid protein VP1 with the mutations S2A, N679K and T711S.
   AAV5-11Mut-GFP refers to a vector based on modified adeno-associated virus serotype 5 (rAAV5) comprising a modified AAV5 capsid protein VP1 with the mutations S2A, P723V and T711S.
   AAV5-12Mut-GFP refers to a vector based on modified adeno-associated virus serotype 5 (rAAV5) comprising a modified AAV5 capsid protein VP1 with the mutations S2A, V431Y and T711S.
   AAV5-13Mut-GFP refers to a vector based on modified adeno-associated virus serotype 5 (rAAV5) comprising a modified AAV5 capsid protein VP1 with the mutations S2A, A616D and T711S.
   AAV5-14Mut-GFP refers to a vector based on modified adeno-associated virus serotype 5 (rAAV5) comprising a modified AAV5 capsid protein VP1 with the mutations S2A, W683R and T711S.
   AAV5-15Mut-GFP refers to a vector based on modified adeno-associated virus serotype 5 (rAAV5) comprising a modified AAV5 capsid protein VP1 with the mutations S2A, S222A and T711S.
   AAV5-16Mut-GFP refers to a vector based on modified adeno-associated virus serotype 5 (rAAV5) comprising a modified AAV5 capsid protein VP1 with the mutations S2A, R285L and T711S.
   AAV5-17Mut-GFP refers to a vector based on modified adeno-associated virus serotype 5 (rAAV5) comprising a modified AAV5 capsid protein VP1 with the mutations S2A, Q340A and T711S.
   AAV5-18Mut-GFP refers to a vector based on modified adeno-associated virus serotype 5 (rAAV5) comprising a modified AAV5 capsid protein VP1 with the mutations S2A, S420F and T711S.
   AAV5-19Mut-GFP refers to a vector based on modified adeno-associated virus serotype 5 (rAAV5) comprising a modified AAV5 capsid protein VP1 with the mutations S2A, S680A and T711S.
   AAV5-20Mut-GFP refers to a vector based on modified adeno-associated virus serotype 5 (rAAV5) comprising a modified AAV5 capsid protein VP1 with the mutations S2A, T721V and T711S.

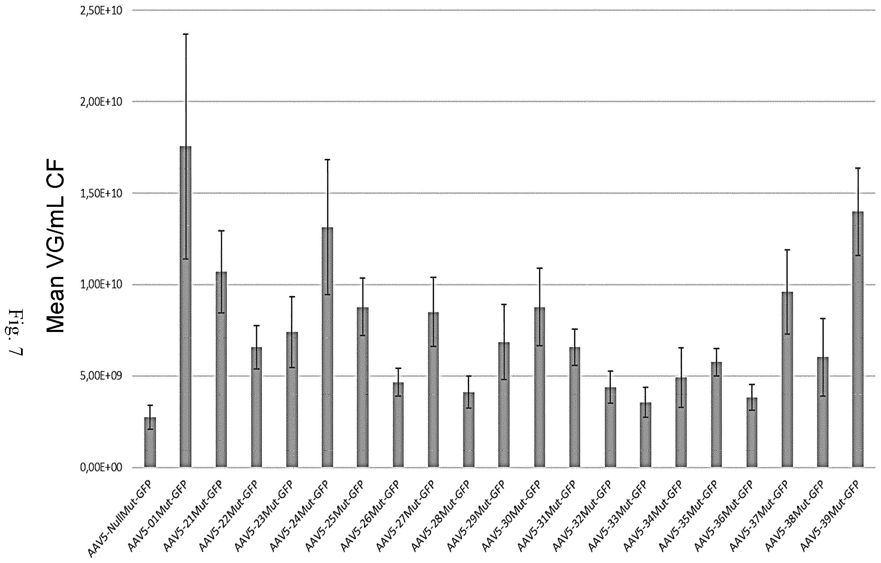

Figure 7 is a graph showing generation efficiency for vectors based on modified adeno-associated virus serotype 5 (rAAV5).
   Vg/ml cf refers to viral genome count per milliliter of culture fluid.
   AAV5-NullMut-GFP refers to a vector based on rAAV5 with a wild-type capsid protein VP1.
   AAV5-01Mut-GFP refers to a vector based on modified adeno-associated virus serotype 5 (rAAV5) comprising a modified AAV5 capsid protein VP1 with the mutations S2A and T711S.
   AAV5-21Mut-GFP refers to a vector based on modified adeno-associated virus serotype 5 (rAAV5) comprising a modified AAV5 capsid protein VP1 with the mutation G226A.
   AAV5-22Mut-GFP refers to a vector based on modified adeno-associated virus serotype 5 (rAAV5) comprising a modified AAV5 capsid protein VP1 with the mutation D286E.
   AAV5-23Mut-GFP refers to a vector based on modified adeno-associated virus serotype 5 (rAAV5) comprising a modified AAV5 capsid protein VP1 with the mutation L341Y.
   AAV5-24Mut-GFP refers to a vector based on modified adeno-associated virus serotype 5 (rAAV5) comprising a modified AAV5 capsid protein VP1 with the mutation C387V.
   AAV5-25Mut-GFP refers to a vector based on modified adeno-associated virus serotype 5 (rAAV5) comprising a modified AAV5 capsid protein VP1 with the mutation Q421H.
   AAV5-26Mut-GFP refers to a vector based on modified adeno-associated virus serotype 5 (rAAV5) comprising a modified AAV5 capsid protein VP1 with the mutation P466T.
   AAV5-27Mut-GFP refers to a vector based on modified adeno-associated virus serotype 5 (rAAV5) comprising a modified AAV5 capsid protein VP1 with the mutation S594Q.
   AAV5-28Mut-GFP refers to a vector based on modified adeno-associated virus serotype 5 (rAAV5) comprising a modified AAV5 capsid protein VP1 with the mutation T614L.
   AAV5-29Mut-GFP refers to a vector based on modified adeno-associated virus serotype 5 (rAAV5) comprising a modified AAV5 capsid protein VP1 with the mutation N679K.
   AAV5-30Mut-GFP refers to a vector based on modified adeno-associated virus serotype 5 (rAAV5) comprising a modified AAV5 capsid protein VP1 with the mutation P723V.
   AAV5-31Mut-GFP refers to a vector based on modified adeno-associated virus serotype 5 (rAAV5) comprising a modified AAV5 capsid protein VP1 with the mutation V431Y.
   AAV5-32Mut-GFP refers to a vector based on modified adeno-associated virus serotype 5 (rAAV5) comprising a modified AAV5 capsid protein VP1 with the mutation A616D.
   AAV5-33Mut-GFP refers to a vector based on modified adeno-associated virus serotype 5 (rAAV5) comprising a modified AAV5 capsid protein VP1 with the mutation W683R.
   AAV5-34Mut-GFP refers to a vector based on modified adeno-associated virus serotype 5 (rAAV5) comprising a modified AAV5 capsid protein VP1 with the mutation S222A.
   AAV5-35Mut-GFP refers to a vector based on modified adeno-associated virus serotype 5 (rAAV5) comprising a modified AAV5 capsid protein VP1 with the mutation R285L.
   AAV5-36Mut-GFP refers to a vector based on modified adeno-associated virus serotype 5 (rAAV5) comprising a modified AAV5 capsid protein VP1 with the mutation Q340A.
   AAV5-37Mut-GFP refers to a vector based on modified adeno-associated virus serotype 5 (rAAV5) comprising a modified AAV5 capsid protein VP1 with the mutation S420F.
   AAV5-38Mut-GFP refers to a vector based on modified adeno-associated virus serotype 5 (rAAV5) comprising a modified AAV5 capsid protein VP1 with the mutation S680A.
   AAV5-39Mut-GFP refers to a vector based on modified adeno-associated virus serotype 5 (rAAV5) comprising a modified AAV5 capsid protein VP1 with the mutation T721V.

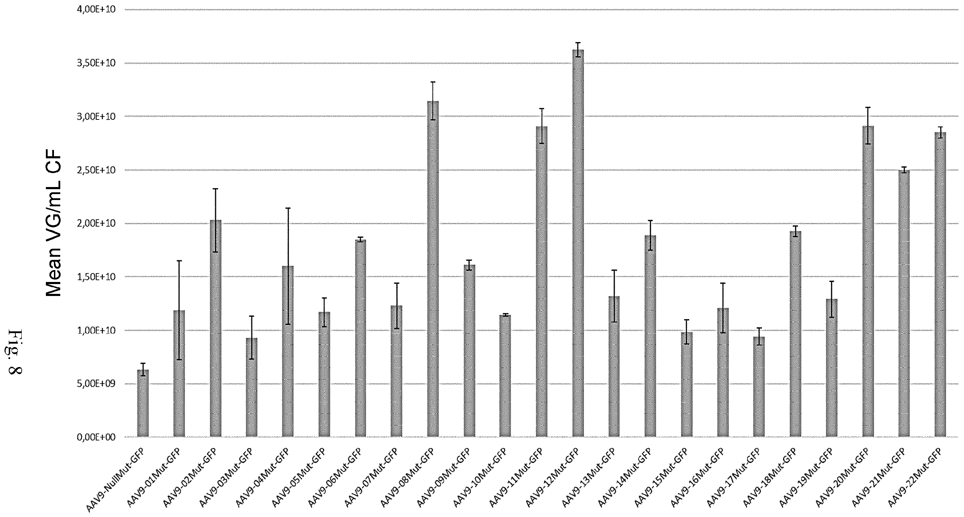

Figure 8 is a graph showing generation efficiency for vectors based on modified adeno-associated virus serotype 9 (rAAV9).
   Vg/ml cf refers to viral genome count per milliliter of culture fluid.
   AAV9-NullMut-GFP refers to a vector based on rAAV9 with a wild-type capsid protein VP1.
   AAV9-01Mut-GFP refers to a vector based on modified adeno-associated virus serotype 9 (rAAV9) comprising a modified AAV9 capsid protein VP1 with the mutation S232T.
   AAV9-02Mut-GFP refers to a vector based on modified adeno-associated virus serotype 9 (rAAV9) comprising a modified AAV9 capsid protein VP1 with the mutation D297E.
   AAV9-03Mut-GFP refers to a vector based on modified adeno-associated virus serotype 9 (rAAV9) comprising a modified AAV9 capsid protein VP1 with the mutation Q351K.
   AAV9-04Mut-GFP refers to a vector based on modified adeno-associated virus serotype 9 (rAAV9) comprising a modified AAV9 capsid protein VP1 with the mutation Q351R.
   AAV9-05Mut-GFP refers to a vector based on modified adeno-associated virus serotype 9 (rAAV9) comprising a modified AAV9 capsid protein VP1 with the mutation C396V.
   AAV9-06Mut-GFP refers to a vector based on modified adeno-associated virus serotype 9 (rAAV9) comprising a modified AAV9 capsid protein VP1 with the mutation D433Y.
   AAV9-07Mut-GFP refers to a vector based on modified adeno-associated virus serotype 9 (rAAV9) comprising a modified AAV9 capsid protein VP1 with the mutation L444R.
   AAV9-08Mut-GFP refers to a vector based on modified adeno-associated virus serotype 9 (rAAV9) comprising a modified AAV9 capsid protein VP1 with the mutation Y478F.
   AAV9-09Mut-GFP refers to a vector based on modified adeno-associated virus serotype 9 (rAAV9) comprising a modified AAV9 capsid protein VP1 with the mutation G604N.
   AAV9-10Mut-GFP refers to a vector based on modified adeno-associated virus serotype 9 (rAAV9) comprising a modified AAV9 capsid protein VP1 with the mutation G627K.
   AA V9-11Mut-GFP refers to a vector based on modified adeno-associated virus serotype 9 (rAAV9) comprising a modified AAV9 capsid protein VP1 with the mutation T625L
   AAV9-12Mut-GFP refers to a vector based on modified adeno-associated virus serotype 9 (rAAV9) comprising a modified AAV9 capsid protein VP1 with the mutation T625V.
   AAV9-13Mut-GFP refers to a vector based on modified adeno-associated virus serotype 9 (rAAV9) comprising a modified AAV9 capsid protein VP1 with the mutation S692T.
   AAV9-14Mut-GFP refers to a vector based on modified adeno-associated virus serotype 9 (rAAV9) comprising a modified AAV9 capsid protein VP1 with the mutation T733V.
   AAV9-15Mut-GFP refers to a vector based on modified adeno-associated virus serotype 9 (rAAV9) comprising a modified AAV9 capsid protein VP1 with the mutation A427R.
   AAV9-16Mut-GFP refers to a vector based on modified adeno-associated virus serotype 9 (rAAV9) comprising a modified AAV9 capsid protein VP1 with the mutation M635D.
   AAV9-17Mut-GFP refers to a vector based on modified adeno-associated virus serotype 9 (rAAV9) comprising a modified AAV9 capsid protein VP1 with the mutation W695R.
   AAV9-18Mut-GFP refers to a vector based on modified adeno-associated virus serotype 9 (rAAV9) comprising a modified AAV9 capsid protein VP1 with the mutation R296L.
   AAV9-19Mut-GFP refers to a vector based on modified adeno-associated virus serotype 9 (rAAV9) comprising a modified AAV9 capsid protein VP1 with the mutation Q351A.
   AAV9-20Mut-GFP refers to a vector based on modified adeno-associated virus serotype 9 (rAAV9) comprising a modified AAV9 capsid protein VP1 with the mutation Y395F.
   AAV9-21Mut-GFP refers to a vector based on modified adeno-associated virus serotype 9 (rAAV9) comprising a modified AAV9 capsid protein VP1 with the mutation R434L.
   AAV9-22Mut-GFP refers to a vector based on modified adeno-associated virus serotype 9 (rAAV9) comprising a modified AAV9 capsid protein VP1 with the mutation N691A.

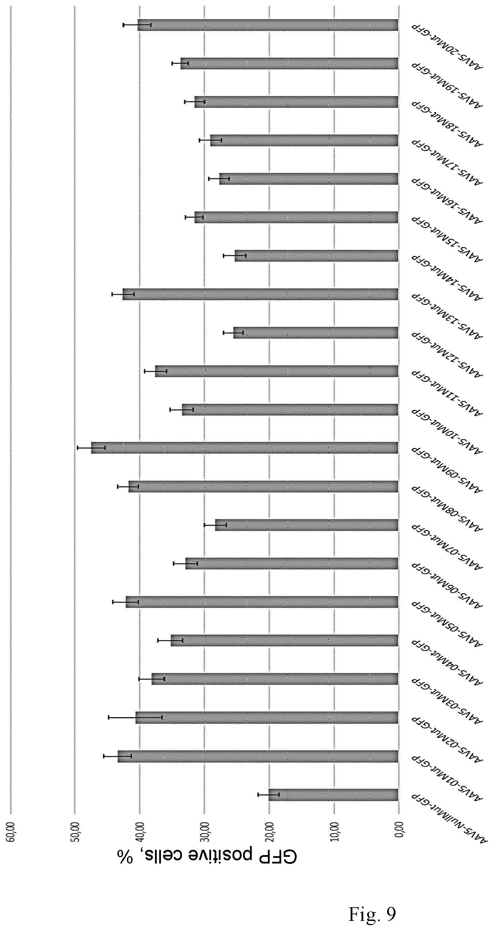

Figure 9 is a graph showing the efficiency of cell transduction with rAAV5-based products comprising point mutations in the wild-type rAAV5 capsid protein VP1 or in the rAAV5 capsid protein VP1 already comprising the mutations S2A and T711S.
   AAV5-NullMut-GFP refers to a vector based on rAAV5 with a wild-type capsid protein VP1
   AAV5-01Mut-GFP refers to a vector based on modified adeno-associated virus serotype 5 (rAAV5) comprising a modified AAV5 capsid protein VP1 with the mutations S2A and T711S.
   AAV5-02Mut-GFP refers to a vector based on modified adeno-associated virus serotype 5 (rAAV5) comprising a modified AAV5 capsid protein VP1 with the mutations S2A, G226A and T711S.
   AAV5-03Mut-GFP refers to a vector based on modified adeno-associated virus serotype 5 (rAAV5) comprising a modified AAV5 capsid protein VP1 with the mutations S2A, D286E and T711S.
   AAV5-04Mut-GFP refers to a vector based on modified adeno-associated virus serotype 5 (rAAV5) comprising a modified AAV5 capsid protein VP1 with the mutations S2A, L341Y and T711S.
   AAV5-05Mut-GFP refers to a vector based on modified adeno-associated virus serotype 5 (rAAV5) comprising a modified AAV5 capsid protein VP1 with the mutations S2A, C387V and T711S.
   AAV5-06Mut-GFP refers to a vector based on modified adeno-associated virus serotype 5 (rAAV5) comprising a modified AAV5 capsid protein VP1 with the mutations S2A, Q421H and T711S.
   AAV5-07Mut-GFP refers to a vector based on modified adeno-associated virus serotype 5 (rAAV5) comprising a modified AAV5 capsid protein VP1 with the mutations S2A, P466T and T711S.
   AAV5-08Mut-GFP refers to a vector based on modified adeno-associated virus serotype 5 (rAAV5) comprising a modified AAV5 capsid protein VP1 with the mutations S2A, S594Q and T711S.
   AAV5-09Mut-GFP refers to a vector based on modified adeno-associated virus serotype 5 (rAAV5) comprising a modified AAV5 capsid protein VP1 with the mutations S2A, T614L and T711S.
   AAV5-10Mut-GFP refers to a vector based on modified adeno-associated virus serotype 5 (rAAV5) comprising a modified AAV5 capsid protein VP1 with the mutations S2A, N679K and T711S.
   AAV5-11Mut-GFP refers to a vector based on modified adeno-associated virus serotype 5 (rAAV5) comprising a modified AAV5 capsid protein VP1 with the mutations S2A, P723V and T711S.
   AAV5-12Mut-GFP refers to a vector based on modified adeno-associated virus serotype 5 (rAAV5) comprising a modified AAV5 capsid protein VP1 with the mutations S2A, V431Y and T711S.
   AAV5-13Mut-GFP refers to a vector based on modified adeno-associated virus serotype 5 (rAAV5) comprising a modified AAV5 capsid protein VP1 with the mutations S2A, A616D and T711S.
   AAV5-14Mut-GFP refers to a vector based on modified adeno-associated virus serotype 5 (rAAV5) comprising a modified AAV5 capsid protein VP1 with the mutations S2A, W683R and T711S.
   AAV5-15Mut-GFP refers to a vector based on modified adeno-associated virus serotype 5 (rAAV5) comprising a modified AAV5 capsid protein VP1 with the mutations S2A, S222A and T711S.
   AAV5-16Mut-GFP refers to a vector based on modified adeno-associated virus serotype 5 (rAAV5) comprising a modified AAV5 capsid protein VP1 with the mutations S2A, R285L and T711S.
   AAV5-17Mut-GFP refers to a vector based on modified adeno-associated virus serotype 5 (rAAV5) comprising a modified AAV5 capsid protein VP1 with the mutations S2A, Q340A and T711S.
   AAV5-18Mut-GFP refers to a vector based on modified adeno-associated virus serotype 5 (rAAV5) comprising a modified AAV5 capsid protein VP1 with the mutations S2A, S420F and T711S.
   AAV5-19Mut-GFP refers to a vector based on modified adeno-associated virus serotype 5 (rAAV5) comprising a modified AAV5 capsid protein VP1 with the mutations S2A, S680A and T711S.
   AAV5-20Mut-GFP refers to a vector based on modified adeno-associated virus serotype 5 (rAAV5) comprising a modified AAV5 capsid protein VP1 with the mutations S2A, T721V and T711S.

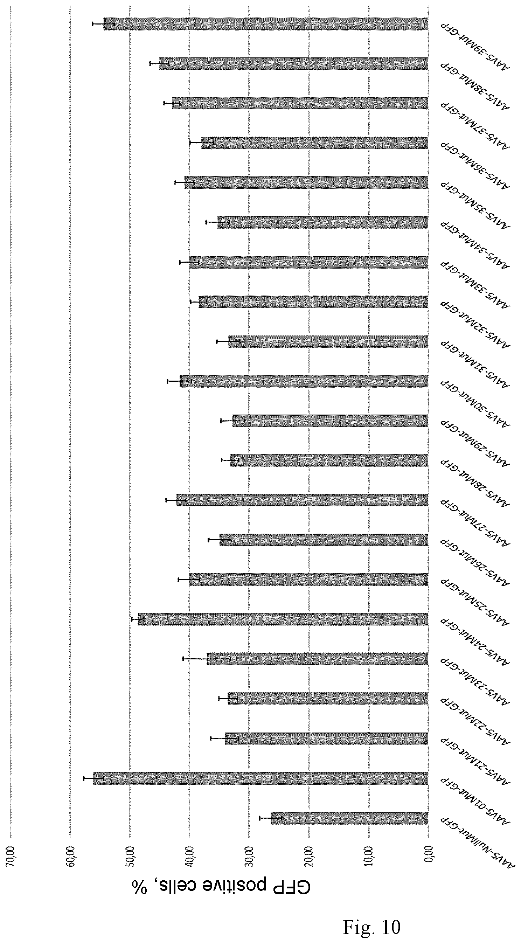

Figure 10 is a graph showing cell transduction efficiency with rAAV5-based products comprising point mutations in the wild-type rAAV5 capsid protein VP1
   AAV5-NullMut-GFP refers to a vector based on rAAV5 with a wild-type capsid protein VP1.
   AAV5-01Mut-GFP refers to a vector based on modified adeno-associated virus serotype 5 (rAAV5) comprising a modified AAV5 capsid protein VP1 with the mutations S2A and T711S.
   AAV5-21Mut-GFP refers to a vector based on modified adeno-associated virus serotype 5 (rAAV5) comprising a modified AAV5 capsid protein VP1 with the mutation G226A.
   AAV5-22Mut-GFP refers to a vector based on modified adeno-associated virus serotype 5 (rAAV5) comprising a modified AAV5 capsid protein VP1 with the mutation D286E.
   AAV5-23Mut-GFP refers to a vector based on modified adeno-associated virus serotype 5 (rAAV5) comprising a modified AAV5 capsid protein VP1 with the mutation L341Y.
   AAV5-24Mut-GFP refers to a vector based on modified adeno-associated virus serotype 5 (rAAV5) comprising a modified AAV5 capsid protein VP1 with the mutation C387V
   AAV5-25Mut-GFP refers to a vector based on modified adeno-associated virus serotype 5 (rAAV5) comprising a modified AAV5 capsid protein VP1 with the mutation Q421H.
   AAV5-26Mut-GFP refers to a vector based on modified adeno-associated virus serotype 5 (rAAV5) comprising a modified AAV5 capsid protein VP1 with the mutation P466T.
   AAV5-27Mut-GFP refers to a vector based on modified adeno-associated virus serotype 5 (rAAV5) comprising a modified AAV5 capsid protein VP1 with the mutation S594Q.
   AAV5-28Mut-GFP refers to a vector based on modified adeno-associated virus serotype 5 (rAAV5) comprising a modified AAV5 capsid protein VP1 with the mutation T614L.
   AAV5-29Mut-GFP refers to a vector based on modified adeno-associated virus serotype 5 (rAAV5) comprising a modified AAV5 capsid protein VP1 with the mutation N679K.
   AAV5-30Mut-GFP refers to a vector based on modified adeno-associated virus serotype 5 (rAAV5) comprising a modified AAV5 capsid protein VP1 with the mutation P723V.
   AAV5-31Mut-GFP refers to a vector based on modified adeno-associated virus serotype 5 (rAAV5) comprising a modified AAV5 capsid protein VP1 with the mutation V431Y.
   AAV5-32Mut-GFP refers to a vector based on modified adeno-associated virus serotype 5 (rAAV5) comprising a modified AAV5 capsid protein VP1 with the mutation A616D.
   AAV5-33Mut-GFP refers to a vector based on modified adeno-associated virus serotype 5 (rAAV5) comprising a modified AAV5 capsid protein VP1 with the mutation W683R.
   AAV5-34Mut-GFP refers to a vector based on modified adeno-associated virus serotype 5 (rAAV5) comprising a modified AAV5 capsid protein VP1 with the mutation S222A.
   AAV5-35Mut-GFP refers to a vector based on modified adeno-associated virus serotype 5 (rAAV5) comprising a modified AAV5 capsid protein VP1 with the mutation R285L.
   AAV5-36Mut-GFP refers to a vector based on modified adeno-associated virus serotype 5 (rAAV5) comprising a modified AAV5 capsid protein VP1 with the mutation Q340A.
   AAV5-37Mut-GFP refers to a vector based on modified adeno-associated virus serotype 5 (rAAV5) comprising a modified AAV5 capsid protein VP1 with the mutation S420F.
   AAV5-38Mut-GFP refers to a vector based on modified adeno-associated virus serotype 5 (rAAV5) comprising a modified AAV5 capsid protein VP1 with the mutation S680A.
   AAV5-39Mut-GFP refers to a vector based on modified adeno-associated virus serotype 5 (rAAV5) comprising a modified AAV5 capsid protein VP1 with the mutation T721V.

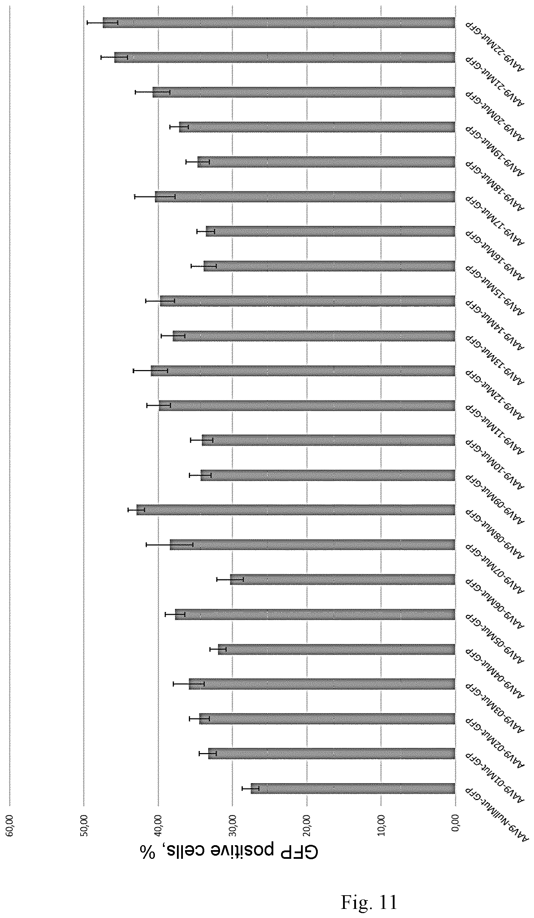

Figure 11 is a graph showing cell transduction efficiency by rAAV9-based products comprising point mutations in the wild-type rAAV9 capsid protein VP1.
   AAV9-NullMut-GFP refers to a vector based on rAAV9 with a wild-type capsid protein VP1.
   AAV9-01Mut-GFP refers to a vector based on modified adeno-associated virus serotype 9 (rAAV9) comprising a modified AAV9 capsid protein VP1 with the mutation S232T.
   AAV9-02Mut-GFP refers to a vector based on modified adeno-associated virus serotype 9 (rAAV9) comprising a modified AAV9 capsid protein VP1 with the mutation D297E.
   AAV9-03Mut-GFP refers to a vector based on modified adeno-associated virus serotype 9 (rAAV9) comprising a modified AAV9 capsid protein VP1 with the mutation Q351K.
   AAV9-04Mut-GFP refers to a vector based on modified adeno-associated virus serotype 9 (rAAV9) comprising a modified AAV9 capsid protein VP1 with the mutation Q351R.
   AAV9-05Mut-GFP refers to a vector based on modified adeno-associated virus serotype 9 (rAAV9) comprising a modified AAV9 capsid protein VP1 with the mutation C396V.
   AAV9-06Mut-GFP refers to a vector based on modified adeno-associated virus serotype 9 (rAAV9) comprising a modified AAV9 capsid protein VP1 with the mutation D433Y.
   AAV9-07Mut-GFP refers to a vector based on modified adeno-associated virus serotype 9 (rAAV9) comprising a modified AAV9 capsid protein VP1 with the mutation L444R.
   AAV9-08Mut-GFP refers to a vector based on modified adeno-associated virus serotype 9 (rAAV9) comprising a modified AAV9 capsid protein VP1 with the mutation Y478F.
   AAV9-09Mut-GFP refers to a vector based on modified adeno-associated virus serotype 9 (rAAV9) comprising a modified AAV9 capsid protein VP1 with the mutation G604N.
   AAV9-10Mut-GFP refers to a vector based on modified adeno-associated virus serotype 9 (rAAV9) comprising a modified AAV9 capsid protein VP1 with the mutation G627K.
   AA V9-11Mut-GFP refers to a vector based on modified adeno-associated virus serotype 9 (rAAV9) comprising a modified AAV9 capsid protein VP1 with the mutation T625L.
   AAV9-12Mut-GFP refers to a vector based on modified adeno-associated virus serotype 9 (rAAV9) comprising a modified AAV9 capsid protein VP1 with the mutation T625V.
   AAV9-13Mut-GFP refers to a vector based on modified adeno-associated virus serotype 9 (rAAV9) comprising a modified AAV9 capsid protein VP1 with the mutation S692T.
   AAV9-14Mut-GFP refers to a vector based on modified adeno-associated virus serotype 9 (rAAV9) comprising a modified AAV9 capsid protein VP1 with the mutation T733V.
   AAV9-15Mut-GFP refers to a vector based on modified adeno-associated virus serotype 9 (rAAV9) comprising a modified AAV9 capsid protein VP1 with the mutation A427R.
   AAV9-16Mut-GFP refers to a vector based on modified adeno-associated virus serotype 9 (rAAV9) comprising a modified AAV9 capsid protein VP1 with the mutation M635D.
   AAV9-17Mut-GFP refers to a vector based on modified adeno-associated virus serotype 9 (rAAV9) comprising a modified AAV9 capsid protein VP1 with the mutation W695R.
   AAV9-18Mut-GFP refers to a vector based on modified adeno-associated virus serotype 9 (rAAV9) comprising a modified AAV9 capsid protein VP1 with the mutation R296L.
   AAV9-19Mut-GFP refers to a vector based on modified adeno-associated virus serotype 9 (rAAV9) comprising a modified AAV9 capsid protein VP1 with the mutation Q351A.
   AAV9-20Mut-GFP refers to a vector based on modified adeno-associated virus serotype 9 (rAAV9) comprising a modified AAV9 capsid protein VP1 with the mutation Y395F.
   AAV9-21Mut-GFP refers to a vector based on modified adeno-associated virus serotype 9 (rAAV9) comprising a modified AAV9 capsid protein VP1 with the mutation R434L.
   AAV9-22Mut-GFP refers to a vector based on modified adeno-associated virus serotype 9 (rAAV9) comprising a modified AAV9 capsid protein VP1 with the mutation N691A.

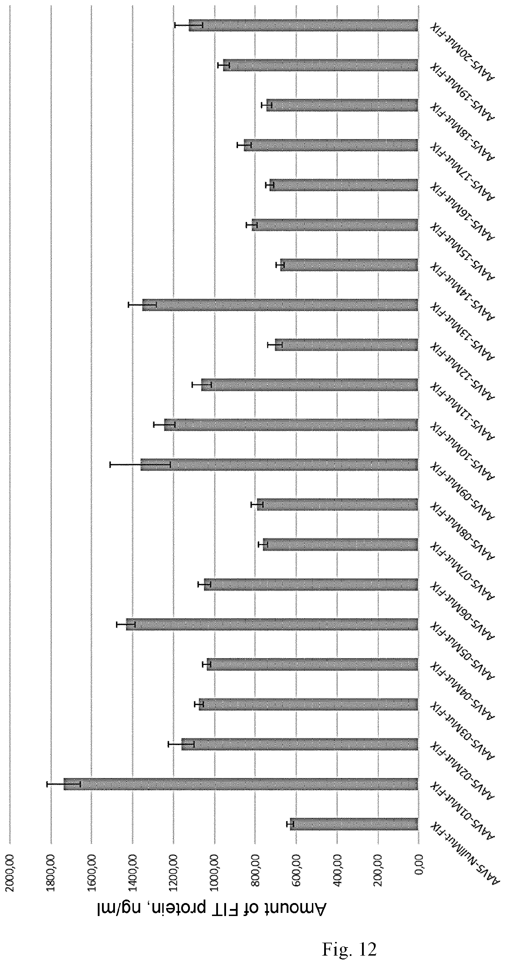

Figure 12 is a graph showing the efficiency of production of target protein encoded by transgene following cell transduction with rAAV5-based products comprising single mutations in wild-type rAAV5 capsid protein VP1 or in rAAV5 capsid protein VP1 already comprising the mutations S2A and T711S.
   AAV5-NullMut-FIX refers to a vector based on rAAV5 with a wild-type capsid protein VP1.
   AAV5-01Mut-FIX refers to a vector based on modified adeno-associated virus serotype 5 (rAAV5) comprising a modified AAV5 capsid protein VP1 with the mutations S2A and T711S.
   AAV5-02Mut-FIX refers to a vector based on modified adeno-associated virus serotype 5 (rAAV5) comprising a modified AAV5 capsid protein VP1 with the mutations S2A, G226A and T711S.
   AAV5-03Mut-FIX refers to a vector based on modified adeno-associated virus serotype 5 (rAAV5) comprising a modified AAV5 capsid protein VP1 with the mutations S2A, D286E and T711S.
   AAV5-04Mut-FIX refers to a vector based on modified adeno-associated virus serotype 5 (rAAV5) comprising a modified AAV5 capsid protein VP1 with the mutations S2A, L341Y and T711S.
   AAV5-05Mut-FIX refers to a vector based on modified adeno-associated virus serotype 5 (rAAV5) comprising a modified AAV5 capsid protein VP1 with the mutations S2A, C387V and T711S
   AAV5-06Mut-FIX refers to a vector based on modified adeno-associated virus serotype 5 (rAAV5) comprising a modified AAV5 capsid protein VP1 with the mutations S2A, Q421H and T711S.
   AAV5-07Mut-FIX refers to a vector based on modified adeno-associated virus serotype 5 (rAAV5) comprising a modified AAV5 capsid protein VP1 with the mutations S2A, P466T and T711S.
   AAV5-08Mut-FIX refers to a vector based on modified adeno-associated virus serotype 5 (rAAV5) comprising a modified AAV5 capsid protein VP1 with the mutations S2A, S594Q and T711S.
   AAV5-09Mut-FIX refers to a vector based on modified adeno-associated virus serotype 5 (rAAV5) comprising a modified AAV5 capsid protein VP1 with the mutations S2A, T614L and T711S.
   AAV5-10Mut-FIX refers to a vector based on modified adeno-associated virus serotype 5 (rAAV5) comprising a modified AAV5 capsid protein VP1 with the mutations S2A, N679K and T711S.
   AAV5-11Mut-FIX refers to a vector based on modified adeno-associated virus serotype 5 (rAAV5) comprising a modified AAV5 capsid protein VP1 with the mutations S2A, P723V and T711S.
   AAV5-12Mut-FIX refers to a vector based on modified adeno-associated virus serotype 5 (rAAV5) comprising a modified AAV5 capsid protein VP1 with the mutations S2A, V431Y and T711S.
   AAV5-13Mut-FIX refers to a vector based on modified adeno-associated virus serotype 5 (rAAV5) comprising a modified AAV5 capsid protein VP1 with the mutations S2A, A616D and T711S.
   AAV5-14Mut-FIX refers to a vector based on modified adeno-associated virus serotype 5 (rAAV5) comprising a modified AAV5 capsid protein VP1 with the mutations S2A, W683R and T711S.
   AAV5-15Mut-FIX refers to a vector based on modified adeno-associated virus serotype 5 (rAAV5) comprising a modified AAV5 capsid protein VP1 with the mutations S2A, S222A and T711S.
   AAV5-16Mut-FIX refers to a vector based on modified adeno-associated virus serotype 5 (rAAV5) comprising a modified AAV5 capsid protein VP1 with the mutations S2A, R285L and T711S.
   AAV5-17Mut-FIX refers to a vector based on modified adeno-associated virus serotype 5 (rAAV5) comprising a modified AAV5 capsid protein VP1 with the mutations S2A, Q340A and T711S.
   AAV5-18Mut-FIX refers to a vector based on modified adeno-associated virus serotype 5 (rAAV5) comprising a modified AAV5 capsid protein VP1 with the mutations S2A, S420F and T711S
   AAV5-19Mut-FIX refers to a vector based on modified adeno-associated virus serotype 5 (rAAV5) comprising a modified AAV5 capsid protein VP1 with the mutations S2A, S680A and T711S.
   AAV5-20Mut-FIX refers to a vector based on modified adeno-associated virus serotype 5 (rAAV5) comprising a modified AAV5 capsid protein VP1 with the mutations S2A, T721V and T711S.

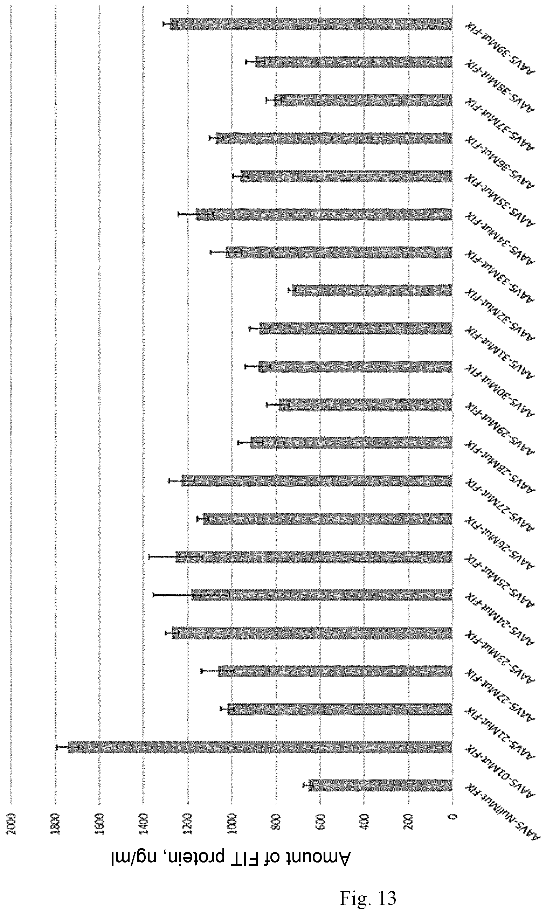

Figure 13 is a graph showing the efficiency of the production of the target protein encoded by the transgene following cell transduction with rAAV5-based products comprising single mutations in the wild-type rAAV5 capsid protein VP1.
   AAV5-NullMut-FIX refers to a vector based on rAAV5 with a wild-type capsid protein VP1.
   AAV5-01Mut-FIX refers to a vector based on modified adeno-associated virus serotype 5 (rAAV5) comprising a modified AAV5 capsid protein VP1 with the mutations S2A and T711S.
   AAV5-21Mut-FIX refers to a vector based on modified adeno-associated virus serotype 5 (rAAV5) comprising a modified AAV5 capsid protein VP1 with the mutation G226A.
   AAV5-22Mut-FIX refers to a vector based on modified adeno-associated virus serotype 5 (rAAV5) comprising a modified AAV5 capsid protein VP1 with the mutation D286E.
   AAV5-23Mut-FIX refers to a vector based on modified adeno-associated virus serotype 5 (rAAV5) comprising a modified AAV5 capsid protein VP1 with the mutation L341Y.
   AAV5-24Mut-FIX refers to a vector based on modified adeno-associated virus serotype 5 (rAAV5) comprising a modified AAV5 capsid protein VP1 with the mutation C387V.
   AAV5-25Mut-FIX refers to a vector based on modified adeno-associated virus serotype 5 (rAAV5) comprising a modified AAV5 capsid protein VP1 with the mutation Q421H.
   AAV5-26Mut-FIX refers to a vector based on modified adeno-associated virus serotype 5 (rAAV5) comprising a modified AAV5 capsid protein VP1 with the mutation P466T.
   AAV5-27Mut-FIX refers to a vector based on modified adeno-associated virus serotype 5 (rAAV5) comprising a modified AAV5 capsid protein VP1 with the mutation S594Q.
   AAV5-28Mut-FIX refers to a vector based on modified adeno-associated virus serotype 5 (rAAV5) comprising a modified AAV5 capsid protein VP1 with the mutation T614L.
   AAV5-29Mut-FIX refers to a vector based on modified adeno-associated virus serotype 5 (rAAV5) comprising a modified AAV5 capsid protein VP1 with the mutation N679K.
   AAV5-30Mut-FIX refers to a vector based on modified adeno-associated virus serotype 5 (rAAV5) comprising a modified AAV5 capsid protein VP1 with the mutation P723V.
   AAV5-31Mut-FIX refers to a vector based on modified adeno-associated virus serotype 5 (rAAV5) comprising a modified AAV5 capsid protein VP1 with the mutation V431Y.
   AAV5-32Mut-FIX refers to a vector based on modified adeno-associated virus serotype 5 (rAAV5) comprising a modified AAV5 capsid protein VP1 with the mutation A616D.
   AAV5-33Mut-FIX refers to a vector based on modified adeno-associated virus serotype 5 (rAAV5) comprising a modified AAV5 capsid protein VP1 with the mutation W683R.
   AAV5-34Mut-FIX refers to a vector based on modified adeno-associated virus serotype 5 (rAAV5) comprising a modified AAV5 capsid protein VP1 with the mutation S222A.
   AAV5-35Mut-FIX refers to a vector based on modified adeno-associated virus serotype 5 (rAAV5) comprising a modified AAV5 capsid protein VP1 with the mutation R285L.
   AAV5-36Mut-FIX refers to a vector based on modified adeno-associated virus serotype 5 (rAAV5) comprising a modified AAV5 capsid protein VP1 with the mutation Q340A.
   AAV5-37Mut-FIX refers to a vector based on modified adeno-associated virus serotype 5 (rAAV5) comprising a modified AAV5 capsid protein VP1 with the mutation S420F.
   AAV5-38Mut-FIX refers to a vector based on modified adeno-associated virus serotype 5 (rAAV5) comprising a modified AAV5 capsid protein VP1 with the mutation S680A.
   AAV5-39Mut-FIX refers to a vector based on modified adeno-associated virus serotype 5 (rAAV5) comprising a modified AAV5 capsid protein VP1 with the mutation T721V.

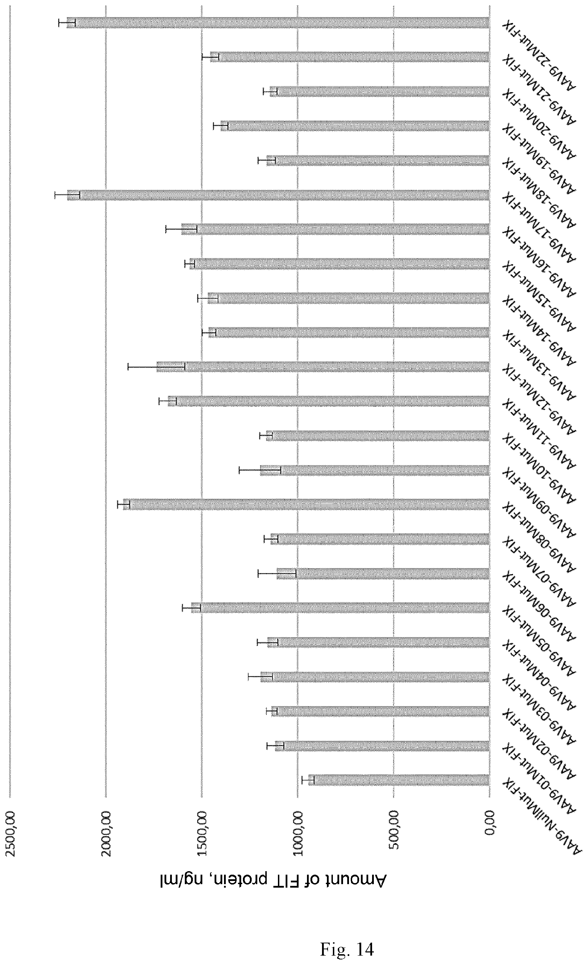

Figure 14 is a graph showing the efficiency of the production of the target protein encoded by the transgene following cell transduction with rAAV9-based products comprising mutations in the wild-type rAAV9 capsid protein VP1
   AAV9-NullMut-FIX refers to a vector based on rAAV9 with a wild-type capsid protein VP1
   AAV9-01Mut-FIX refers to a vector based on modified adeno-associated virus serotype 9 (rAAV9) comprising a modified AAV9 capsid protein VP1 with the mutation S232T.
   AAV9-02Mut-FIX refers to a vector based on modified adeno-associated virus serotype 9 (rAAV9) comprising a modified AAV9 capsid protein VP1 with the mutation D297E.
   AAV9-03Mut-FIX refers to a vector based on modified adeno-associated virus serotype 9 (rAAV9) comprising a modified AAV9 capsid protein VP1 with the mutation Q351K.
   AAV9-04Mut-FIX refers to a vector based on modified adeno-associated virus serotype 9 (rAAV9) comprising a modified AAV9 capsid protein VP1 with the mutation Q351R.
   AAV9-05Mut-FIX refers to a vector based on modified adeno-associated virus serotype 9 (rAAV9) comprising a modified AAV9 capsid protein VP1 with the mutation C396V.
   AAV9-06Mut-FIX refers to a vector based on modified adeno-associated virus serotype 9 (rAAV9) comprising a modified AAV9 capsid protein VP1 with the mutation D433Y.
   AAV9-07Mut-FIX refers to a vector based on modified adeno-associated virus serotype 9 (rAAV9) comprising a modified AAV9 capsid protein VP1 with the mutation L444R.
   AAV9-08Mut-FIX refers to a vector based on modified adeno-associated virus serotype 9 (rAAV9) comprising a modified AAV9 capsid protein VP1 with the mutation Y478F.
   AAV9-09Mut-FIX refers to a vector based on modified adeno-associated virus serotype 9 (rAAV9) comprising a modified AAV9 capsid protein VP1 with the mutation G604N.
   AAV9-10Mut-FIX refers to a vector based on modified adeno-associated virus serotype 9 (rAAV9) comprising a modified AAV9 capsid protein VP1 with the mutation G627K.
   AAV9-11Mut-FIX refers to a vector based on modified adeno-associated virus serotype 9 (rAAV9) comprising a modified AAV9 capsid protein VP1 with the mutation T625L
   AAV9-12Mut-FIX refers to a vector based on modified adeno-associated virus serotype 9 (rAAV9) comprising a modified AAV9 capsid protein VP1 with the mutation T625V.
   AAV9-13Mut-FIX refers to a vector based on modified adeno-associated virus serotype 9 (rAAV9) comprising a modified AAV9 capsid protein VP1 with the mutation S692T
   AAV9-14Mut-FIX refers to a vector based on modified adeno-associated virus serotype 9 (rAAV9) comprising a modified AAV9 capsid protein VP1 with the mutation T733V.
   AAV9-15Mut-FIX refers to a vector based on modified adeno-associated virus serotype 9 (rAAV9) comprising a modified AAV9 capsid protein VP1 with the mutation A427R.
   AAV9-16Mut-FIX refers to a vector based on modified adeno-associated virus serotype 9 (rAAV9) comprising a modified AAV9 capsid protein VP1 with the mutation M635D.
   AAV9-17Mut-FIX refers to a vector based on modified adeno-associated virus serotype 9 (rAAV9) comprising a modified AAV9 capsid protein VP1 with the mutation W695R.
   AAV9-18Mut-FIX refers to a vector based on modified adeno-associated virus serotype 9 (rAAV9) comprising a modified AAV9 capsid protein VP1 with the mutation R296L.
   AAV9-19Mut-FIX refers to a vector based on modified adeno-associated virus serotype 9 (rAAV9) comprising a modified AAV9 capsid protein VP1 with the mutation Q351A.
   AAV9-20Mut-FIX refers to a vector based on modified adeno-associated virus serotype 9 (rAAV9) comprising a modified AAV9 capsid protein VP1 with the mutation Y395F.
   AAV9-21Mut-FIX refers to a vector based on modified adeno-associated virus serotype 9 (rAAV9) comprising a modified AAV9 capsid protein VP1 with the mutation R434L.
   AAV9-22Mut-FIX refers to a vector based on modified adeno-associated virus serotype 9 (rAAV9) comprising a modified AAV9 capsid protein VP1 with the mutation N691A.

### Definitions and general methods

Unless defined otherwise herein, all technical and scientific terms used in connection with the present invention will have the same meaning as is commonly understood by those skilled in the art.

Furthermore, unless otherwise required by context, singular terms shall include plural terms, and the plural terms shall include the singular terms. Typically, the present classification and methods of cell culture, molecular biology, immunology, microbiology, genetics, analytical chemistry, organic synthesis chemistry, medical and pharmaceutical chemistry, as well as hybridization and chemistry of protein and nucleic acids described herein are well known by those skilled and widely used in the art. Enzyme reactions and purification methods are performed according to the manufacturer's guidelines, as is common in the art, or as described herein.

The terms "naturally occurring", "native", or "wild-type" are used to describe an object that can be found in nature as distinct from being artificially produced. For example, a protein or nucleotide sequence present in an organism (including a virus), which can be isolated from a source in nature and that has not been intentionally modified by a person in the laboratory, is naturally occurring.

As used in the present description and claims that follow, unless otherwise dictated by the context, the words "include" and "comprise", or variations thereof such as "includes", "including", "comprises", or "comprising", will be understood to imply the inclusion of a stated integer or group of integers but not the exclusion of any other integer or group of integers.

### Adeno-associated virus (AAV)

Viruses of the family Parvoviridae are small DNA-containing animal viruses. The Parvoviridae family may be divided into two subfamilies: the Parvovirinae, which members infect vertebrates, and the Densovirinae, which members infect insects. By 2006, there have been 11 serotypes of adeno-associated virus described (Mori, S. ET AL., 2004, "Two novel adeno-associated viruses from cynomolgus monkey: pseudotyping characterization of capsid protein", Virology, T. 330 (2): 375-83). Serotype 12 of the adeno-associated virus was described in 2008 (Michael Schmidt ET AL., Adeno-associated virus type 12 (AAV12): a novel AAV serotype with sialic acid- and heparan sulfate proteoglycan-independent transduction activity, J Virol. 2008 Feb;82(3): 1399-406. doi: 10.1128/JVI.02012-07). All of the known serotypes can infect cells from multiple tissue types. Tissue specificity is determined by the capsid protein serotype; therefore, the adeno-associated virus-based vectors are constructed by assigning the desired serotype. Further information on parvoviruses and other members of the Parvoviridae has been described in the literature (Kenneth I. Berns, «Parvoviridae: The Viruses and Their Replication», Chapter 69 in Fields Virology (3d Ed. 1996)).

The genomic organization of all known AAV serotypes is very similar. The genome of AAV is a linear, single-stranded DNA molecule that is less than about 5000 nucleotides (nt) in length. Inverted terminal repeats (ITR) flank unique coding nucleotide sequences of proteins (Rep) that are essential for the virus life cycle, as well as sequences of overlapping capsid proteins (Cap). The Cap gene encodes VP proteins (VP1, VP2 and VP3), which form a capsid, as well as AAP (protein activating adeno-associated virus (AAV) assembly (Sonntag F, Köther K, Schmidt K, et al. The assembly-activating protein promotes capsid assembly of different adeno-associated virus serotypes. J Virol. 2011;85(23):12686-12697. doi:10.1128/JVI.05359-11) and MAAP (membrane-associated accessory protein (Ogden PJ, Kelsic ED, Sinai S, Church GM. Comprehensive AAV capsid fitness landscape reveals a viral gene and enables machine-guided design. Science. 2019;366(6469):1139-1143. doi:10.1126/science.aaw2900). The AAV genome flanking sequences which are 145 nucleotides long are self-complementary and are organized such that an energetically stable intramolecular duplex forming a T-shaped hairpin may be formed. Such hairpin structures function as an origin for virus DNA replication, serving as primers for the cellular DNA polymerase complex. Following wild-type AAV (wtAAV) infection in mammalian cells, the Rep genes (e.g. Rep78 and Rep52) are expressed using the P5 promoter and the P19 promoter, respectively, and the both Rep proteins have a certain function in the replication of the viral genome. A splicing event in the Rep open reading frame (Rep ORF) results in the expression of actually four Rep proteins (e.g. Rep78, Rep68, Rep52, and Rep40). However, it has been shown that the unspliced mRNA encoding Rep78 and Rep52 proteins is sufficient for AAV vector production in mammalian cells.

### Recombinant adeno-associated virus (rAAV)-based vector

The term "vector" as used herein means a nucleic acid molecule capable of transporting another nucleic acid to which it has been linked. Furthermore, the term "vector" herein refers to a viral particle capable of transporting a nucleic acid.

As used in the present description, the term "expression" is defined as the transcription and/or translation of a particular nucleotide sequence driven by its promoter.

### Use

"Delivering to a subject a heterologous nucleic acid sequence" is the insertion of genes into the cells and/or tissues of the subject.

The terms "subject", "patient", "individual", and the like are used interchangeably in the present description, and they refer to any animal which is amenable to the methods described in the present description. In certain non-limiting embodiments, the subject, patient or individual is a human. Said subject may be either male or female, of any age.

### Detailed description of the invention

### Method for producing modified adeno-associated virus capsid

In one aspect, the present invention relates to a method for producing a modified AAV capsid, comprising:
a) determining amino acids in capsomere protein of the modified AAV, which are located in the interaction interface region of adjacent pentameric subunits of the capsid;
b) aligning structurally the modified AAV capsid to the template capsid to determine the pairwise correspondence between each amino acid of the modified AAV from the interaction interface region between adjacent pentameric subunits and the amino acid closest thereto of the template capsid, as a structural analogue of the initial amino acid that is considered a potential substitution,
   wherein the template capsid is understood to mean a structurally similar capsid of a virus selected from the family Parvoviridae, which is other than AAV;
c) comparing pairwisely amino acid residues, determined in step b), of the AAV capsid and the template capsid to identify structural differences between the modified AAV capsid and the template capsid in the interaction interface region of adjacent pentameric subunits of the capsid;
d) selecting positions for mutagenesis, wherein the amino acid residues for mutagenesis are located in the interaction interface region of adjacent pentameric subunits of the modified AAV capsid and have a structural difference between the modified AAV capsid and the template capsid in the interaction interface region of adjacent pentameric subunits of the capsid;
e) selecting an amino acid residue for mutagenesis at position selected in step d) using one of the following principles:
   - substitution of the initial amino acid in the capsomere protein of the modified AAV for an amino acid that has a greater volume, other than cysteine and methionine;
   - substitution of the initial amino acid in the capsomere protein of the modified AAV for an amino acid that has an increased or decreased number of polar contacts between the capsomeres, other than cysteine and methionine;
f) introducing one or more amino acid substitutions selected in steps d)-e) in the interaction interface region of adjacent pentameric subunits of the modified AAV capsid.

Interaction interface (or surface) refers to a set of capsomere protein polypeptide chain fragments interacting with the amino acids of an adjacent pentameric subunit, wherein the pentameric subunit refers to a capsid structural element having the shape of a regular pentagon and consisting of 5 capsomere proteins located around the central pore (L. M. Drouin and M. Agbandje-McKenna. Adeno-associated virus structural biology as a tool in vector development. Future Virol., vol. 8, no. 12, pp. 1183-1199, 2013).

Interaction interface for a given subunit is intended to refer to all amino acids, at least one atom of which is present at distance lower than 5 Å from any atom of adjacent pentameric subunits.

For AAV5, the calculation was made based on the capsid structure PDB ID: 6JCT.

According to the calculation, its interaction interface between adjacent pentameric AAV5 subunits includes the following ranges of AAV5 capsid protein VP1 residues: 220 - 226, 283 - 293, 339 - 342, 385 - 392, 412 - 435, 462 - 466, 590 - 598, 611 - 626, 677 - 685, 721-724.

For AAV9, a similar calculation was made based on the structure PDB ID: 3UX1.

According to the calculation, its interaction interface between adjacent pentameric subunits of AAV9 includes the following ranges of AAV9 capsid protein VP1 residues: 230 - 236, 294 - 304, 350 - 353, 394 - 401, 421 - 444, 476 - 480, 601 - 609, 622 - 637, 689 - 697, 733 - 736.

"One or more amino acid substitutions" refers to one, two, three, four, five, six, seven, eight, nine or ten amino acid substitutions. For example, from 1 to 10 amino acid substitutions, from 1 to 9 amino acid substitutions, from 1 to 8 amino acid substitutions, from 1 to 7 amino acid substitutions, from 1 to 6 amino acid substitutions, from 1 to 5 amino acid substitutions, from 1 to 4 amino acid substitutions, from 1 to 3 amino acid substitutions or from 1 to 2 amino acid substitutions.

In some embodiments of the invention, the method for producing a modified AAV capsid further comprises checking the modified AAV capsids produced in step f) with one or more amino acid substitutions for the presence of one or more improved properties as compared to AAV capsid free of these modifications, wherein the one or more improved properties are selected from the group:
- increased efficiency of cell transduction,
- increased production of target protein,
- increased efficiency of generation due to highly efficient production (assembly) of encapsidated viral vectors based on recombinant adeno-associated virus (rAAV).

In some embodiments of the invention, the method for producing a modified AAV capsid further comprises checking the modified AAV capsids produced in step f) for the presence of one or more improved properties as compared to AAV capsid free of these modifications and selecting modified AAV capsids that have one or more improved properties as compared to AAV capsid free of these modifications, wherein the one or more improved properties are selected from the group:
- increased efficiency of cell transduction,
- increased production of target protein,
- increased efficiency of generation due to highly efficient production (assembly) of encapsidated viral vectors based on recombinant adeno-associated virus (rAAV).

In some embodiments of the method for producing an AAV capsid, the modified AAV capsid is selected from the group comprising: human AAV, simian AAV, or avian AAV.

In some embodiments of the method for producing an AAV capsid, the modified AAV capsid is selected from the group comprising the following AAV serotypes: AAV1, AAV2, AAV3, AAV4, AAV5, AAV6, AAV7, AAV8, AAV9, AAV10 AAV11, AAV12, AAV13, AAV14, AAV15, AAV16, rAAV.rh8, rAAV.rhlO, rAAV.rh20, rAAV.rh39, rAAV.Rh74, rAAV.RHM4-l, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15 or AAV.HSC16.

In some embodiments of the method for producing an AAV capsid, the modified AAV capsid may be a wild-type AAV capsid or an AAV capsid comprising one or more amino acid substitutions in proteins VP1, VP2 and/or VP3.

In some embodiments of the method for producing an AAV capsid, the modified AAV capsid may be a wild-type AAV5 capsid or an AAV5 capsid comprising one or more amino acid substitutions in proteins VP1, VP2 and/or VP3.

In some embodiments of the method for producing an AAV capsid, the modified AAV capsid may be an AAV5 capsid comprising the amino acid substitutions S2A and T711S in the protein VP1.

In some embodiments of the method for producing an AAV capsid, the modified AAV capsid may be a wild-type AAV9 capsid or an AAV9 capsid comprising one or more amino acid substitutions in proteins VP1, VP2 and/or VP3.

In some embodiments of the method for producing an AAV capsid, the structurally similar template capsid is selected from the group comprising: parvovirus B19, human bocavirus 1 (HBoV1), bovine parvovirus (BVP).

### Modified AAV capsid according to the invention

In one aspect, the present invention relates to a modified AAV capsid for producing viral vectors based on recombinant adeno-associated virus produced by a method comprising:
a) determining amino acids in capsomere protein of the modified AAV, which are located in the interaction interface region of adjacent pentameric subunits of the capsid;
b) aligning structurally the modified AAV capsid to the template capsid to determine the pairwise correspondence between each amino acid of the modified AAV from the interaction interface region between adjacent pentameric subunits and the amino acid closest thereto of the template capsid, as a structural analogue of the initial amino acid that is considered a potential substitution,
   wherein the template capsid is understood to mean a structurally similar capsid of a virus selected from the family Parvoviridae, which is other than AAV;
c) comparing pairwisely amino acid residues, determined in step b), of the AAV capsid and the template capsid to identify structural differences between the modified AAV capsid and the template capsid in the interaction interface region of adjacent pentameric subunits of the capsid;
d) selecting positions for mutagenesis, wherein the amino acid residues for mutagenesis are located in the interaction interface region of adjacent pentameric subunits of the modified AAV capsid and have a structural difference between the modified AAV capsid and the template capsid in the interaction interface region of adjacent pentameric subunits of the capsid;
e) selecting an amino acid residue for mutagenesis at position selected in step d) using one of the following principles:
   - substitution of the initial amino acid in the capsomere protein of the modified AAV for an amino acid that has a greater volume, other than cysteine and methionine;
   - substitution of the initial amino acid in the capsomere protein of the modified AAV for an amino acid that has an increased or decreased number of polar contacts between the capsomeres, other than cysteine and methionine;
f) introducing one or more amino acid substitutions selected in steps d)-e) in the interaction interface region of adjacent pentameric subunits of the modified AAV capsid;
   and, if necessary, further comprising
g) checking the modified AAV capsids produced in step f) for the presence of one or more improved properties as compared to AAV capsid free of these modifications and selecting modified AAV capsids that have one or more improved properties as compared to AAV capsid free of these modifications, wherein the one or more improved properties are selected from the group:
   - increased efficiency of cell transduction,
   - increased production of target protein,
   - increased efficiency of generation due to highly efficient production (assembly) of encapsidated viral vectors based on recombinant adeno-associated virus (rAAV).

In some embodiments of the modified AAV capsid, the modified AAV capsid is selected from the group comprising: human AAV, simian AAV, or avian AAV.

In some embodiments of the modified AAV capsid, the modified AAV capsid is selected from the group comprising the following AAV serotypes: AAV: AAV1, AAV2, AAV3, AAV4, AAV5, AAV6, AAV7, AAV8, AAV9, AAV10 AAV11, AAV12, AAV13, AAV14, AAV15, AAV16, rAAV.rh8, rAAV.rhlO, rAAV.rh20, rAAV.rh39, rAAV.Rh74, rAAV.RHM4-l, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15 or AAV.HSC16.

In some embodiments of the modified AAV capsid, the modified AAV capsid may be a wild-type AAV capsid or an AAV capsid comprising one or more amino acid substitutions in proteins VP1, VP2 and/or VP3.

In some embodiments of the modified AAV capsid, the modified AAV capsid may be a wild-type AAV5 capsid or an AAV5 capsid comprising one or more amino acid substitutions in proteins VP1, VP2 and/or VP3.

In some embodiments of the modified AAV capsid, the modified AAV capsid may be an AAV5 capsid comprising the amino acid substitutions S2A and T711S in the protein VP1.

In some embodiments of the modified AAV capsid, the modified AAV capsid may be a wild-type AAV9 capsid or an AAV9 capsid comprising one or more amino acid substitutions in proteins VP1, VP2 and/or VP3.

The "right-hand side" of a (+)-chain of genomic DNA of adeno-associated virus comprises overlapping sequences encoding three capsid proteins: VP1, VP2 and VP3. Transcription of these genes starts from a single promoter, p40. The molecular weights of the corresponding proteins are 87, 72, and 62 kDa, respectively. All of the three proteins are translated from a single mRNA. Following transcription, pre-mRNA can be spliced in two different manners, where either longer or shorter intron is excised to form mRNAs of 2300 or 2600 nucleotide long.

Thus, the introduction of mutations into the *Cap* gene will affect not only the AAV capsid protein VP1, but also the AAV VP2 and VP3 capsid proteins.

Below are the positions of AAV5 capsid structural proteins in the Cap AAV5 gene sequence:
1-725 aa - VP1,
137-725 aa - VP2,
193-725 aa - VP3.

Below are the positions of AAV9 capsid structural proteins in the Cap AAV9 gene sequence:
1-737 aa - VP1,
138-737 aa - VP2,
203-737 aa - VP3.

In some embodiments of the invention, the modified AAV capsid includes a modified AAV capsid protein VP1 having an amino acid sequence selected from the group: SEQ ID No: 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362 or 368.

In some embodiments of the invention, the modified AAV capsid includes a modified AAV capsid protein VP2 having an amino acid sequence selected from the group: SEQ ID No: 16, 22, 28, 34, 40, 46, 52, 58, 64, 70, 76, 82, 88, 94, 100, 106, 112, 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364 or 370.

In some embodiments of the invention, the modified AAV capsid includes a modified AAV capsid protein VP3 having an amino acid sequence selected from the group: SEQ ID No: 18, 24, 30, 36, 42, 48, 54, 60, 66, 72, 78, 84, 90, 96, 102, 108, 114, 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366 or 372.

In some embodiments of the invention, the modified AAV capsid comprises:
a) a modified AAV capsid protein VP1 having an amino acid sequence that is selected from the group: SEQ ID No: 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, or 368;
b) a modified AAV capsid protein VP2 corresponding to protein VP1 thereof, said modified AAV capsid protein VP2 having an amino acid sequence that is selected from the group: SEQ ID No: 16, 22, 28, 34, 40, 46, 52, 58, 64, 70, 76, 82, 88, 94, 100, 106, 112, 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, or 370;
c) a modified AAV capsid protein VP3 corresponding to protein VP1 thereof, said modified AAV capsid protein VP3 having an amino acid sequence that is selected from the group: SEQ ID No: 18, 24, 30, 36, 42, 48, 54, 60, 66, 72, 78, 84, 90, 96, 102, 108, 114, 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, or 372.

In some embodiments of the invention, the modified AAV capsid includes a modified AAV5 capsid protein VP1 comprising the substitution G226A (AAV5 Capsid VP1 G226A) and having the amino acid sequence of SEQ ID No: 14.

In some embodiments of the invention, the modified AAV capsid includes a modified AAV5 capsid protein VP2 comprising the substitution G90A (AAV5 Capsid VP2 G90A) and having the amino acid sequence of SEQ ID No: 16.

In some embodiments of the invention, the modified AAV capsid includes a modified AAV5 capsid protein VP3 comprising the substitution G34A (AAV5 Capsid VP3 G34A) and having the amino acid sequence of SEQ ID No: 18

In some embodiments of the invention, the modified AAV capsid includes a modified AAV5 capsid protein VP1 comprising the substitutions S2A, G226A and T711S (AAV5 Capsid VP1 S2A, G226A and T711S) and having the amino acid sequence of SEQ ID No: 20.

In some embodiments of the invention, the modified AAV capsid includes a modified AAV5 capsid protein VP2 comprising the substitutions G90A and T575S (AAV5 Capsid VP2 G90A and T575S) and having the amino acid sequence of SEQ ID No: 22.

In some embodiments of the invention, the modified AAV capsid includes a modified AAV5 capsid protein VP3 comprising the substitutions G34A T519S (AAV5 Capsid VP3 G34A T519S) and having the amino acid sequence of SEQ ID No: 24.

In some embodiments of the invention, the modified AAV capsid includes a modified AAV5 capsid protein VP1 comprising the substitution D286E (AAV5 Capsid VP1 D286E) and having the amino acid sequence of SEQ ID No: 26.

In some embodiments of the invention, the modified AAV capsid includes a modified AAV5 capsid protein VP2 comprising the substitution D150E (AAV5 Capsid VP2 D150E) and having the amino acid sequence of SEQ ID No: 28.

In some embodiments of the invention, the modified AAV capsid includes a modified AAV5 capsid protein VP3 comprising the substitution D94E (AAV5 Capsid VP3 D94E) and having the amino acid sequence of SEQ ID No: 30.

In some embodiments of the invention, the modified AAV capsid includes a modified AAV5 capsid protein VP1 comprising the substitutions S2A, D286E and T711S (AAV5 Capsid VP1 S2A, D286E and T711S) and having the amino acid sequence of SEQ ID No: 32.

In some embodiments of the invention, the modified AAV capsid includes a modified AAV5 capsid protein VP2 comprising the substitutions D150E and T575S (AAV5 Capsid VP2 D150E and T575S) and having the amino acid sequence of SEQ ID No: 34

In some embodiments of the invention, the modified AAV capsid includes a modified AAV5 capsid protein VP3 comprising the substitutions D94E and T519S (AAV5 Capsid VP3 D94E and T519S) and having the amino acid sequence of SEQ ID No: 36.

In some embodiments of the invention, the modified AAV capsid includes a modified AAV5 capsid protein VP1 comprising the substitution L341Y (AAV5 Capsid VP1 L341Y) and having the amino acid sequence of SEQ ID No: 38.

In some embodiments of the invention, the modified AAV capsid includes a modified AAV5 capsid protein VP2 comprising the substitution L205Y (AAV5 Capsid VP2 L205Y) and having the amino acid sequence of SEQ ID No: 40.

In some embodiments of the invention, the modified AAV capsid includes a modified AAV5 capsid protein VP3 comprising the substitution L149Y (AAV5 Capsid VP3 L149Y) and having the amino acid sequence of SEQ ID No: 42.

In some embodiments of the invention, the modified AAV capsid includes a modified AAV5 capsid protein VP1 comprising the substitutions S2A, L341Y, T711S (AAV5 Capsid VP1 S2A, L341Y, T711S) and having the amino acid sequence of SEQ ID No: 44.

In some embodiments of the invention, the modified AAV capsid includes a modified AAV5 capsid protein VP2 comprising the substitutions L205Y, T575S (AAV5 Capsid VP2 L205Y, T575S) and having the amino acid sequence of SEQ ID No: 46.

In some embodiments of the invention, the modified AAV capsid includes a modified AAV5 capsid protein VP3 comprising the substitutions L149Y, T519S (AAV5 Capsid VP3 L149Y, T519S) and having the amino acid sequence of SEQ ID No: 48.

In some embodiments of the invention, the modified AAV capsid includes a modified AAV5 capsid protein VP1 comprising the substitution C387V (AAV5 Capsid VP1 C387V) and having the amino acid sequence of SEQ ID No: 50.

In some embodiments of the invention, the modified AAV capsid includes a modified AAV5 capsid protein VP2 comprising the substitution C251V (AAV5 Capsid VP2 C251V) and having the amino acid sequence of SEQ ID No: 52.

In some embodiments of the invention, the modified AAV capsid includes a modified AAV5 capsid protein VP3 comprising the substitution C195V (AAV5 Capsid VP3 C195V) and having the amino acid sequence of SEQ ID No: 54.

In some embodiments of the invention, the modified AAV capsid includes a modified AAV5 capsid protein VP1 comprising the substitutions S2A, C387V, T711S (AAV5 Capsid VP1 S2A, C387V, T711S) and having the amino acid sequence of SEQ ID No: 56.

In some embodiments of the invention, the modified AAV capsid includes a modified AAV5 capsid protein VP2 comprising the substitutions C251V, T575S (AAV5 Capsid VP2 C251V, T575S) and having the amino acid sequence of SEQ ID No: 58.

In some embodiments of the invention, the modified AAV capsid includes a modified AAV5 capsid protein VP3 comprising the substitutions C195V, T519S (AAV5 Capsid VP3 C195V, T519S) and having the amino acid sequence of SEQ ID No: 60.

In some embodiments of the invention, the modified AAV capsid includes a modified AAV5 capsid protein VP1 comprising the substitution Q421H (AAV5 Capsid VP1 Q421H) and having the amino acid sequence of SEQ ID No: 62.

In some embodiments of the invention, the modified AAV capsid includes a modified AAV5 capsid protein VP2 comprising the substitution Q285H (AAV5 Capsid VP2 Q285H) and having the amino acid sequence of SEQ ID No: 64.

In some embodiments of the invention, the modified AAV capsid includes a modified AAV5 capsid protein VP3 comprising the substitution Q229H (AAV5 Capsid VP3 Q229H) and having the amino acid sequence of SEQ ID No: 66.

In some embodiments of the invention, the modified AAV capsid includes a modified AAV5 capsid protein VP1 comprising the substitutions S2A Q421H T711S (AAV5 Capsid VP1 S2A Q421H T711S) and having the amino acid sequence of SEQ ID No: 68.

In some embodiments of the invention, the modified AAV capsid includes a modified AAV5 capsid protein VP2 comprising the substitutions Q285H T575S (AAV5 Capsid VP2 Q285H T575S) and having the amino acid sequence of SEQ ID No: 70.

In some embodiments of the invention, the modified AAV capsid includes a modified AAV5 capsid protein VP3 comprising the substitutions Q229H T519S (AAV5 Capsid VP3 Q229H T519S) and having the amino acid sequence of SEQ ID No: 72.

In some embodiments of the invention, the modified AAV capsid includes a modified AAV5 capsid protein VP1 comprising the substitution P466T (AAV5 Capsid VP1 P466T) and having the amino acid sequence of SEQ ID No: 74.

In some embodiments of the invention, the modified AAV capsid includes a modified AAV5 capsid protein VP2 comprising the substitution P330T (AAV5 Capsid VP2 P330T) and having the amino acid sequence of SEQ ID No: 76.

In some embodiments of the invention, the modified AAV capsid includes a modified AAV5 capsid protein VP3 comprising the substitution P274T (AAV5 Capsid VP3 P274T) and having the amino acid sequence of SEQ ID No: 78.

In some embodiments of the invention, the modified AAV capsid includes a modified AAV5 capsid protein VP1 comprising the substitutions S2A P466T T711S (AAV5 Capsid VP1 S2A P466T T711S) and having the amino acid sequence of SEQ ID No: 80.

In some embodiments of the invention, the modified AAV capsid includes a modified AAV5 capsid protein VP2 comprising the substitutions P330T T575S (AAV5 Capsid VP2 P330T T575S) and having the amino acid sequence of SEQ ID No: 82.

In some embodiments of the invention, the modified AAV capsid includes a modified AAV5 capsid protein VP3 comprising the substitutions P274T T519S (AAV5 Capsid VP3 P274T T519S) and having the amino acid sequence of SEQ ID No: 84.

In some embodiments of the invention, the modified AAV capsid includes a modified AAV5 capsid protein VP1 comprising the substitution S594Q (AAV5 Capsid VP1 S594Q) and having the amino acid sequence of SEQ ID No: 86.

In some embodiments of the invention, the modified AAV capsid includes a modified AAV5 capsid protein VP2 comprising the substitution S458Q (AAV5 Capsid VP2 S458Q) and having the amino acid sequence of SEQ ID No: 88.

In some embodiments of the invention, the modified AAV capsid includes a modified AAV5 capsid protein VP3 comprising the substitution S402Q (AAV5 Capsid VP3 S402Q) and having the amino acid sequence of SEQ ID No: 90.

In some embodiments of the invention, the modified AAV capsid includes a modified AAV5 capsid protein VP1 comprising the substitutions S2A S594Q T711S (AAV5 Capsid VP1 S2A S594Q T711S) and having the amino acid sequence of SEQ ID No: 92.

In some embodiments of the invention, the modified AAV capsid includes a modified AAV5 capsid protein VP2 comprising the substitutions S458Q T575S (AAV5 Capsid VP2 S458Q T575S) and having the amino acid sequence of SEQ ID No: 94.

In some embodiments of the invention, the modified AAV capsid includes a modified AAV5 capsid protein VP3 comprising the substitutions S402Q T519S (AAV5 Capsid VP3 S402Q T519S) and having the amino acid sequence of SEQ ID No: 96.

In some embodiments of the invention, the modified AAV capsid includes a modified AAV5 capsid protein VP1 comprising the substitutions T614L (AAV5 Capsid VP1 T614L) and having the amino acid sequence of SEQ ID No: 98.

In some embodiments of the invention, the modified AAV capsid includes a modified AAV5 capsid protein VP2 comprising the substitutions T478L (AAV5 Capsid VP2 T478L) and having the amino acid sequence of SEQ ID No: 100.

In some embodiments of the invention, the modified AAV capsid includes a modified AAV5 capsid protein VP3 comprising the substitutions T422L (AAV5 Capsid VP3 T422L) and having the amino acid sequence of SEQ ID No: 102.

In some embodiments of the invention, the modified AAV capsid includes a modified AAV5 capsid protein VP1 comprising the substitutions S2A T614L T711S (AAV5 Capsid VP1 S2A T614L T711S) and having the amino acid sequence of SEQ ID No: 104.

In some embodiments of the invention, the modified AAV capsid includes a modified AAV5 capsid protein VP2 comprising the substitutions T478L T575S (AAV5 Capsid VP2 T478L T575S) and having the amino acid sequence of SEQ ID No: 106.

In some embodiments of the invention, the modified AAV capsid includes a modified AAV5 capsid protein VP3 comprising the substitutions (AAV5 Capsid VP3 T422L T519S) and having the amino acid sequence of SEQ ID No: 108.

In some embodiments of the invention, the modified AAV capsid includes a modified AAV5 capsid protein VP1 comprising the substitution N679K (AAV5 Capsid VP1 N679K) and having the amino acid sequence of SEQ ID No: 110.

In some embodiments of the invention, the modified AAV capsid includes a modified AAV5 capsid protein VP2 comprising the substitution N543K (AAV5 Capsid VP2 N543K) and having the amino acid sequence of SEQ ID No: 112.

In some embodiments of the invention, the modified AAV capsid includes a modified AAV5 capsid protein VP3 comprising the substitution N487K (AAV5 Capsid VP3 N487K) and having the amino acid sequence of SEQ ID No: 114.

In some embodiments of the invention, the modified AAV capsid includes a modified AAV5 capsid protein VP1 comprising the substitutions S2A N679K T711S (AAV5 Capsid VP1 S2A N679K T711S) and having the amino acid sequence of SEQ ID No: 116.

In some embodiments of the invention, the modified AAV capsid includes a modified AAV5 capsid protein VP2 comprising the substitutions N543K T575S (AAV5 Capsid VP2 N543K T575S) and having the amino acid sequence of SEQ ID No: 118.

In some embodiments of the invention, the modified AAV capsid includes a modified AAV5 capsid protein VP3 comprising the substitutions N487K T519S (AAV5 Capsid VP3 N487K T519S) and having the amino acid sequence of SEQ ID No: 120.

In some embodiments of the invention, the modified AAV capsid includes a modified AAV5 capsid protein VP1 comprising the substitution P723V (AAV5 Capsid VP1 P723V) and having the amino acid sequence of SEQ ID No: 122.

In some embodiments of the invention, the modified AAV capsid includes a modified AAV5 capsid protein VP2 comprising the substitution P587V (AAV5 Capsid VP2 P587V) and having the amino acid sequence of SEQ ID No: 124.

In some embodiments of the invention, the modified AAV capsid includes a modified AAV5 capsid protein VP3 comprising the substitution P531V (AAV5 Capsid VP3 P531V) and having the amino acid sequence of SEQ ID No: 126.

In some embodiments of the invention, the modified AAV capsid includes a modified AAV5 capsid protein VP1 comprising the substitutions P723V T711S (AAV5 Capsid VP1 S2A P723V T711S) and having the amino acid sequence of SEQ ID No: 128.

In some embodiments of the invention, the modified AAV capsid includes a modified AAV5 capsid protein VP2 comprising the substitutions P587V T575S (AAV5 Capsid VP2 P587V T575S) and having the amino acid sequence of SEQ ID No: 130.

In some embodiments of the invention, the modified AAV capsid includes a modified AAV5 capsid protein VP3 comprising the substitutions P531V T519S (AAV5 Capsid VP3 P531V T519S) and having the amino acid sequence of SEQ ID No: 132.

In some embodiments of the invention, the modified AAV capsid includes a modified AAV5 capsid protein VP1 comprising the substitution V431Y (AAV5 Capsid VP1 V431Y) and having the amino acid sequence of SEQ ID No: 134.

In some embodiments of the invention, the modified AAV capsid includes a modified AAV5 capsid protein VP2 comprising the substitution V295Y (AAV5 Capsid VP2 V295Y) and having the amino acid sequence of SEQ ID No: 136.

In some embodiments of the invention, the modified AAV capsid includes a modified AAV5 capsid protein VP3 comprising the substitution V239Y (AAV5 Capsid VP3 V239Y) and having the amino acid sequence of SEQ ID No: 138.

In some embodiments of the invention, the modified AAV capsid includes a modified AAV5 capsid protein VP1 comprising the substitutions S2A V431Y T711S (AAV5 Capsid VP1 S2A V431Y T711S) and having the amino acid sequence of SEQ ID No: 140.

In some embodiments of the invention, the modified AAV capsid includes a modified AAV5 capsid protein VP2 comprising the substitutions V295Y T575S (AAV5 Capsid VP2 V295Y T575S) and having the amino acid sequence of SEQ ID No: 142.

In some embodiments of the invention, the modified AAV capsid includes a modified AAV5 capsid protein VP3 comprising the substitutions V239Y T519S (AAV5 Capsid VP3 V239Y T519S) and having the amino acid sequence of SEQ ID No: 144.

In some embodiments of the invention, the modified AAV capsid includes a modified AAV5 capsid protein VP1 comprising the substitution A616D (AAV5 Capsid VP1 A616D) and having the amino acid sequence of SEQ ID No: 146.

In some embodiments of the invention, the modified AAV capsid includes a modified AAV5 capsid protein VP2 comprising the substitution A480D (AAV5 Capsid VP2 A480D) and having the amino acid sequence of SEQ ID No: 148.

In some embodiments of the invention, the modified AAV capsid includes a modified AAV5 capsid protein VP3 comprising the substitution A424D (AAV5 Capsid VP3 A424D) and having the amino acid sequence of SEQ ID No: 150.

In some embodiments of the invention, the modified AAV capsid includes a modified AAV5 capsid protein VP1 comprising the substitutions S2A A616D T711S (AAV5 Capsid VP1 S2A A616D T711S) and having the amino acid sequence of SEQ ID No: 152.

In some embodiments of the invention, the modified AAV capsid includes a modified AAV5 capsid protein VP2 comprising the substitutions A480D T575S (AAV5 Capsid VP2 A480D T575S) and having the amino acid sequence of SEQ ID No: 154.

In some embodiments of the invention, the modified AAV capsid includes a modified AAV5 capsid protein VP3 comprising the substitutions A424D T519S (AAV5 Capsid VP3 A424D T519S) and having the amino acid sequence of SEQ ID No: 156.

In some embodiments of the invention, the modified AAV capsid includes a modified AAV5 capsid protein VP1 comprising the substitution W683R (AAV5 Capsid VP1 W683R) and having the amino acid sequence of SEQ ID No: 158.

In some embodiments of the invention, the modified AAV capsid includes a modified AAV5 capsid protein VP2 comprising the substitution W547R (AAV5 Capsid VP2 W547R) and having the amino acid sequence of SEQ ID No: 160.

In some embodiments of the invention, the modified AAV capsid includes a modified AAV5 capsid protein VP3 comprising the substitution W491R (AAV5 Capsid VP3 W491R) and having the amino acid sequence of SEQ ID No: 162.

In some embodiments of the invention, the modified AAV capsid includes a modified AAV5 capsid protein VP1 comprising the substitutions S2A W683R T711S (AAV5 Capsid VP1 S2A W683R T711S) and having the amino acid sequence of SEQ ID No: 164.

In some embodiments of the invention, the modified AAV capsid includes a modified AAV5 capsid protein VP2 comprising the substitutions W547R T575S (AAV5 Capsid VP2 W547R T575S) and having the amino acid sequence of SEQ ID No: 166.

In some embodiments of the invention, the modified AAV capsid includes a modified AAV5 capsid protein VP3 comprising the substitutions W491R T519S (AAV5 Capsid VP3 W491R T519S) and having the amino acid sequence of SEQ ID No: 168.

In some embodiments of the invention, the modified AAV capsid includes a modified AAV5 capsid protein VP1 comprising the substitution S222A (AAV5 Capsid VP1 S222A) and having the amino acid sequence of SEQ ID No: 170.

In some embodiments of the invention, the modified AAV capsid includes a modified AAV5 capsid protein VP2 comprising the substitution S86A (AAV5 Capsid VP2 S86A) and having the amino acid sequence of SEQ ID No: 172.

In some embodiments of the invention, the modified AAV capsid includes a modified AAV5 capsid protein VP3 comprising the substitution S30A (AAV5 Capsid VP3 S30A) and having the amino acid sequence of SEQ ID No: 174.

In some embodiments of the invention, the modified AAV capsid includes a modified AAV5 capsid protein VP1 comprising the substitutions S2A S222A T711S (AAV5 Capsid VP1 S2A S222A T711S) and having the amino acid sequence of SEQ ID No: 176.

In some embodiments of the invention, the modified AAV capsid includes a modified AAV5 capsid protein VP2 comprising the substitutions S86A T575S (AAV5 Capsid VP2 S86A T575S) and having the amino acid sequence of SEQ ID No: 178.

In some embodiments of the invention, the modified AAV capsid includes a modified AAV5 capsid protein VP3 comprising the substitutions S30A T519S (AAV5 Capsid VP3 S30A T519S) and having the amino acid sequence of SEQ ID No: 180.

In some embodiments of the invention, the modified AAV capsid includes a modified AAV5 capsid protein VP1 comprising the substitution R285L (AAV5 Capsid VP1 R285L) and having the amino acid sequence of SEQ ID No: 182.

In some embodiments of the invention, the modified AAV capsid includes a modified AAV5 capsid protein VP2 comprising the substitution R149L (AAV5 Capsid VP2 R149L) and having the amino acid sequence of SEQ ID No: 184.

In some embodiments of the invention, the modified AAV capsid includes a modified AAV5 capsid protein VP3 comprising the substitution R93L (AAV5 Capsid VP3 R93L) and having the amino acid sequence of SEQ ID No: 186.

In some embodiments of the invention, the modified AAV capsid includes a modified AAV5 capsid protein VP1 comprising the substitutions S2A R285L T711S (AAV5 Capsid VP1 S2A R285L T711S) and having the amino acid sequence of SEQ ID No: 188.

In some embodiments of the invention, the modified AAV capsid includes a modified AAV5 capsid protein VP2 comprising the substitutions R149L T575S (AAV5 Capsid VP2 R149L T575S) and having the amino acid sequence of SEQ ID No: 190.

In some embodiments of the invention, the modified AAV capsid includes a modified AAV5 capsid protein VP3 comprising the substitutions R93L T519S (AAV5 Capsid VP3 R93L T519S) and having the amino acid sequence of SEQ ID No: 192.

In some embodiments of the invention, the modified AAV capsid includes a modified AAV5 capsid protein VP1 comprising the substitution Q340A (AAV5 Capsid VP1 Q340A) and having the amino acid sequence of SEQ ID No: 194.

In some embodiments of the invention, the modified AAV capsid includes a modified AAV5 capsid protein VP2 comprising the substitution (AAV5 Capsid VP2 Q204A) and having the amino acid sequence of SEQ ID No: 196.

In some embodiments of the invention, the modified AAV capsid includes a modified AAV5 capsid protein VP3 comprising the substitution Q148A (AAV5 Capsid VP3 Q148A) and having the amino acid sequence of SEQ ID No: 198.

In some embodiments of the invention, the modified AAV capsid includes a modified AAV5 capsid protein VP1 comprising the substitutions S2A Q340A T711S (AAV5 Capsid VP1 S2A Q340A T711S) and having the amino acid sequence of SEQ ID No: 200.

In some embodiments of the invention, the modified AAV capsid includes a modified AAV5 capsid protein VP2 comprising the substitutions Q204A T575S (AAV5 Capsid VP2 Q204A T575S) and having the amino acid sequence of SEQ ID No: 202.

In some embodiments of the invention, the modified AAV capsid includes a modified AAV5 capsid protein VP3 comprising the substitutions Q148A T519S (AAV5 Capsid VP3 Q148A T519S) and having the amino acid sequence of SEQ ID No: 204.

In some embodiments of the invention, the modified AAV capsid includes a modified AAV5 capsid protein VP1 comprising the substitution S420F (AAV5 Capsid VP1 S420F) and having the amino acid sequence of SEQ ID No: 206.

In some embodiments of the invention, the modified AAV capsid includes a modified AAV5 capsid protein VP2 comprising the substitution S284F (AAV5 Capsid VP2 S284F) and having the amino acid sequence of SEQ ID No: 208.

In some embodiments of the invention, the modified AAV capsid includes a modified AAV5 capsid protein VP3 comprising the substitution S228F (AAV5 Capsid VP3 S228F) and having the amino acid sequence of SEQ ID No: 210.

In some embodiments of the invention, the modified AAV capsid includes a modified AAV5 capsid protein VP1 comprising the substitutions S2A S420F T711S (AAV5 Capsid VP1 S2A S420F T711S) and having the amino acid sequence of SEQ ID No: 212.

In some embodiments of the invention, the modified AAV capsid includes a modified AAV5 capsid protein VP2 comprising the substitutions S284F T575S (AAV5 Capsid VP2 S284F T575S) and having the amino acid sequence of SEQ ID No: 214.

In some embodiments of the invention, the modified AAV capsid includes a modified AAV5 capsid protein VP3 comprising the substitutions S228F T519S (AAV5 Capsid VP3 S228F T519S) and having the amino acid sequence of SEQ ID No: 216.

In some embodiments of the invention, the modified AAV capsid includes a modified AAV5 capsid protein VP1 comprising the substitution S680A (AAV5 Capsid VP1 S680A) and having the amino acid sequence of SEQ ID No: 218.

In some embodiments of the invention, the modified AAV capsid includes a modified AAV5 capsid protein VP2 comprising the substitution S544A (AAV5 Capsid VP2 S544A) and having the amino acid sequence of SEQ ID No: 220.

In some embodiments of the invention, the modified AAV capsid includes a modified AAV5 capsid protein VP3 comprising the substitution S488A (AAV5 Capsid VP3 S488A) and having the amino acid sequence of SEQ ID No: 222.

In some embodiments of the invention, the modified AAV capsid includes a modified AAV5 capsid protein VP1 comprising the substitutions S2A S680A T711S (AAV5 Capsid VP1 S2A S680A T711S) and having the amino acid sequence of SEQ ID No: 224.

In some embodiments of the invention, the modified AAV capsid includes a modified AAV5 capsid protein VP2 comprising the substitutions S544A T575S (AAV5 Capsid VP2 S544A T575S) and having the amino acid sequence of SEQ ID No: 226.

In some embodiments of the invention, the modified AAV capsid includes a modified AAV5 capsid protein VP3 comprising the substitutions S488A T519S (AAV5 Capsid VP3 S488A T519S) and having the amino acid sequence of SEQ ID No: 228.

In some embodiments of the invention, the modified AAV capsid includes a modified AAV5 capsid protein VP1 comprising the substitution T721V (AAV5 Capsid VP1 T721V) and having the amino acid sequence of SEQ ID No: 230.

In some embodiments of the invention, the modified AAV capsid includes a modified AAV5 capsid protein VP2 comprising the substitution T585V (AAV5 Capsid VP2 T585V) and having the amino acid sequence of SEQ ID No: 232.

In some embodiments of the invention, the modified AAV capsid includes a modified AAV5 capsid protein VP3 comprising the substitution T529V (AAV5 Capsid VP3 T529V) and having the amino acid sequence of SEQ ID No: 234.

In some embodiments of the invention, the modified AAV capsid includes a modified AAV5 capsid protein VP1 comprising the substitutions S2A T721V T711S (AAV5 Capsid VP1 S2A T721V T711S) and having the amino acid sequence of SEQ ID No: 236.

In some embodiments of the invention, the modified AAV capsid includes a modified AAV5 capsid protein VP2 comprising the substitutions T585V T575S (AAV5 Capsid VP2 T585V T575S) and having the amino acid sequence of SEQ ID No: 238.

In some embodiments of the invention, the modified AAV capsid includes a modified AAV5 capsid protein VP3 comprising the substitutions T529V T519S (AAV5 Capsid VP3 T529V T519S) and having the amino acid sequence of SEQ ID No: 240.

In some embodiments of the invention, the modified AAV capsid includes a modified AAV9 capsid protein VP1 comprising the substitution Q351K (AAV9 Capsid VP1 Q351K) and having the amino acid sequence of SEQ ID No: 242.

In some embodiments of the invention, the modified AAV capsid includes a modified AAV9 capsid protein VP2 comprising the substitution Q214K (AAV9 Capsid VP2 Q214K) and having the amino acid sequence of SEQ ID No: 244.

In some embodiments of the invention, the modified AAV capsid includes a modified AAV9 capsid protein VP3 comprising the substitution Q149K (AAV9 Capsid VP3 Q149K) and having the amino acid sequence of SEQ ID No: 246.

In some embodiments of the invention, the modified AAV capsid includes a modified AAV9 capsid protein VP1 comprising the substitution Q351R (AAV9 Capsid VP1 Q351R) and having the amino acid sequence of SEQ ID No: 248.

In some embodiments of the invention, the modified AAV capsid includes a modified AAV9 capsid protein VP2 comprising the substitution Q214R (AAV9 Capsid VP2 Q214R) and having the amino acid sequence of SEQ ID No: 250.

In some embodiments of the invention, the modified AAV capsid includes a modified AAV9 capsid protein VP3 comprising the substitution Q149R (AAV9 Capsid VP3 Q149R) and having the amino acid sequence of SEQ ID No: 252.

In some embodiments of the invention, the modified AAV capsid includes a modified AAV9 capsid protein VP1 comprising the substitution S232T (AAV9 Capsid VP1 S232T) and having the amino acid sequence of SEQ ID No: 254.

In some embodiments of the invention, the modified AAV capsid includes a modified AAV9 capsid protein VP2 comprising the substitution S95T (AAV9 Capsid VP2 S95T) and having the amino acid sequence of SEQ ID No: 256.

In some embodiments of the invention, the modified AAV capsid includes a modified AAV9 capsid protein VP3 comprising the substitution S30T (AAV9 Capsid VP3 S30T) and having the amino acid sequence of SEQ ID No: 258.

In some embodiments of the invention, the modified AAV capsid includes a modified AAV9 capsid protein VP1 comprising the substitution D297E (AAV9 Capsid VP1 D297E) and having the amino acid sequence of SEQ ID No: 260.

In some embodiments of the invention, the modified AAV capsid includes a modified AAV9 capsid protein VP2 comprising the substitution D160E (AAV9 Capsid VP2 D160E) and having the amino acid sequence of SEQ ID No: 262.

In some embodiments of the invention, the modified AAV capsid includes a modified AAV9 capsid protein VP3 comprising the substitution D95E (AAV9 Capsid VP3 D95E) and having the amino acid sequence of SEQ ID No: 264.

In some embodiments of the invention, the modified AAV capsid includes a modified AAV9 capsid protein VP1 comprising the substitution S692T (AAV9 Capsid VP1 S692T) and having the amino acid sequence of SEQ ID No: 266.

In some embodiments of the invention, the modified AAV capsid includes a modified AAV9 capsid protein VP2 comprising the substitution S555T (AAV9 Capsid VP2 S555T) and having the amino acid sequence of SEQ ID No: 268.

In some embodiments of the invention, the modified AAV capsid includes a modified AAV9 capsid protein VP3 comprising the substitution S490T (AAV9 Capsid VP3 S490T) and having the amino acid sequence of SEQ ID No: 270.

In some embodiments of the invention, the modified AAV capsid includes a modified AAV9 capsid protein VP1 comprising the substitution D433Y (AAV9 Capsid VP1 D433Y) and having the amino acid sequence of SEQ ID No: 272.

In some embodiments of the invention, the modified AAV capsid includes a modified AAV9 capsid protein VP2 comprising the substitution D296Y (AAV9 Capsid VP2 D296Y) and having the amino acid sequence of SEQ ID No: 274.

In some embodiments of the invention, the modified AAV capsid includes a modified AAV9 capsid protein VP3 comprising the substitution D231Y (AAV9 Capsid VP3 D231Y) and having the amino acid sequence of SEQ ID No: 276.

In some embodiments of the invention, the modified AAV capsid includes a modified AAV9 capsid protein VP1 comprising the substitution T625L (AAV9 Capsid VP1 T625L) and having the amino acid sequence of SEQ ID No: 278.

In some embodiments of the invention, the modified AAV capsid includes a modified AAV9 capsid protein VP2 comprising the substitution T488L (AAV9 Capsid VP2 T488L) and having the amino acid sequence of SEQ ID No: 280.

In some embodiments of the invention, the modified AAV capsid includes a modified AAV9 capsid protein VP3 comprising the substitution T423L (AAV9 Capsid VP3 T423L) and having the amino acid sequence of SEQ ID No: 282.

In some embodiments of the invention, the modified AAV capsid includes a modified AAV9 capsid protein VP1 comprising the substitution T625V (AAV9 Capsid VP1 T625V) and having the amino acid sequence of SEQ ID No: 284.

In some embodiments of the invention, the modified AAV capsid includes a modified AAV9 capsid protein VP2 comprising the substitution T488V (AAV9 Capsid VP2 T488V) and having the amino acid sequence of SEQ ID No: 286.

In some embodiments of the invention, the modified AAV capsid includes a modified AAV9 capsid protein VP3 comprising the substitution T423V (AAV9 Capsid VP3 T423V) and having the amino acid sequence of SEQ ID No: 288.

In some embodiments of the invention, the modified AAV capsid includes a modified AAV9 capsid protein VP1 comprising the substitution N691A (AAV9 Capsid VP1 N691A) and having the amino acid sequence of SEQ ID No: 290.

In some embodiments of the invention, the modified AAV capsid includes a modified AAV9 capsid protein VP2 comprising the substitution N554A (AAV9 Capsid VP2 N554A) and having the amino acid sequence of SEQ ID No: 292.

In some embodiments of the invention, the modified AAV capsid includes a modified AAV9 capsid protein VP3 comprising the substitution N489A (AAV9 Capsid VP3 N489A) and having the amino acid sequence of SEQ ID No: 294.

In some embodiments of the invention, the modified AAV capsid includes a modified AAV9 capsid protein VP1 comprising the substitution C396V (AAV9 Capsid VP1 C396V) and having the amino acid sequence of SEQ ID No: 296.

In some embodiments of the invention, the modified AAV capsid includes a modified AAV9 capsid protein VP2 comprising the substitution C259V (AAV9 Capsid VP2 C259V) and having the amino acid sequence of SEQ ID No: 298.

In some embodiments of the invention, the modified AAV capsid includes a modified AAV9 capsid protein VP3 comprising the substitution C194V (AAV9 Capsid VP3 C194V) and having the amino acid sequence of SEQ ID No: 300.

In some embodiments of the invention, the modified AAV capsid includes a modified AAV9 capsid protein VP1 comprising the substitution Y478F (AAV9 Capsid VP1 Y478F) and having the amino acid sequence of SEQ ID No: 302.

In some embodiments of the invention, the modified AAV capsid includes a modified AAV9 capsid protein VP2 comprising the substitution Y341F (AAV9 Capsid VP2 Y341F) and having the amino acid sequence of SEQ ID No: 304.

In some embodiments of the invention, the modified AAV capsid includes a modified AAV9 capsid protein VP3 comprising the substitution Y276F (AAV9 Capsid VP3 Y276F) and having the amino acid sequence of SEQ ID No: 306.

In some embodiments of the invention, the modified AAV capsid includes a modified AAV9 capsid protein VP1 comprising the substitution R296L (AAV9 Capsid VP1 R296L) and having the amino acid sequence of SEQ ID No: 308.

In some embodiments of the invention, the modified AAV capsid includes a modified AAV9 capsid protein VP2 comprising the substitution R159L (AAV9 Capsid VP2 R159L) and having the amino acid sequence of SEQ ID No: 310.

In some embodiments of the invention, the modified AAV capsid includes a modified AAV9 capsid protein VP3 comprising the substitution R94L (AAV9 Capsid VP3 R94L) and having the amino acid sequence of SEQ ID No: 312.

In some embodiments of the invention, the modified AAV capsid includes a modified AAV9 capsid protein VP1 comprising the substitution T733V (AAV9 Capsid VP1 T733V) and having the amino acid sequence of SEQ ID No: 314.

In some embodiments of the invention, the modified AAV capsid includes a modified AAV9 capsid protein VP2 comprising the substitution T596V (AAV9 Capsid VP2 T596V) and having the amino acid sequence of SEQ ID No: 316.

In some embodiments of the invention, the modified AAV capsid includes a modified AAV9 capsid protein VP3 comprising the substitution T531V (AAV9 Capsid VP3 T531V) and having the amino acid sequence of SEQ ID No: 318.

In some embodiments of the invention, the modified AAV capsid includes a modified AAV9 capsid protein VP1 comprising the substitution A427R (AAV9 Capsid VP1 A427R) and having the amino acid sequence of SEQ ID No: 320.

In some embodiments of the invention, the modified AAV capsid includes a modified AAV9 capsid protein VP2 comprising the substitution A290R (AAV9 Capsid VP2 A290R) and having the amino acid sequence of SEQ ID No: 322.

In some embodiments of the invention, the modified AAV capsid includes a modified AAV9 capsid protein VP3 comprising the substitution A225R (AAV9 Capsid VP3 A225R) and having the amino acid sequence of SEQ ID No: 324.

In some embodiments of the invention, the modified AAV capsid includes a modified AAV9 capsid protein VP1 comprising the substitution M635D (AAV9 Capsid VP1 M635D) and having the amino acid sequence of SEQ ID No: 326.

In some embodiments of the invention, the modified AAV capsid includes a modified AAV9 capsid protein VP2 comprising the substitution M498D (AAV9 Capsid VP2 M498D) and having the amino acid sequence of SEQ ID No: 328.

In some embodiments of the invention, the modified AAV capsid includes a modified AAV9 capsid protein VP3 comprising the substitution M433D (AAV9 Capsid VP3 M433D) and having the amino acid sequence of SEQ ID No: 330.

In some embodiments of the invention, the modified AAV capsid includes a modified AAV9 capsid protein VP1 comprising the substitution L444R (AAV9 Capsid VP1 L444R) and having the amino acid sequence of SEQ ID No: 332.

In some embodiments of the invention, the modified AAV capsid includes a modified AAV9 capsid protein VP2 comprising the substitution L307R (AAV9 Capsid VP2 L307R) and having the amino acid sequence of SEQ ID No: 334.

In some embodiments of the invention, the modified AAV capsid includes a modified AAV9 capsid protein VP3 comprising the substitution L242R (AAV9 Capsid VP3 L242R) and having the amino acid sequence of SEQ ID No: 336.

In some embodiments of the invention, the modified AAV capsid includes a modified AAV9 capsid protein VP1 comprising the substitution G604N (AAV9 Capsid VP1 G604N) and having the amino acid sequence of SEQ ID No: 338.

In some embodiments of the invention, the modified AAV capsid includes a modified AAV9 capsid protein VP2 comprising the substitution G467N (AAV9 Capsid VP2 G467N) and having the amino acid sequence of SEQ ID No: 340.

In some embodiments of the invention, the modified AAV capsid includes a modified AAV9 capsid protein VP3 comprising the substitution G402N (AAV9 Capsid VP3 G402N) and having the amino acid sequence of SEQ ID No: 342.

In some embodiments of the invention, the modified AAV capsid includes a modified AAV9 capsid protein VP1 comprising the substitution G627K (AAV9 Capsid VP1 G627K) and having the amino acid sequence of SEQ ID No: 344.

In some embodiments of the invention, the modified AAV capsid includes a modified AAV9 capsid protein VP2 comprising the substitution G490K (AAV9 Capsid VP2 G490K) and having the amino acid sequence of SEQ ID No: 346.

In some embodiments of the invention, the modified AAV capsid includes a modified AAV9 capsid protein VP3 comprising the substitution G425K (AAV9 Capsid VP3 G425K) and having the amino acid sequence of SEQ ID No: 348.

In some embodiments of the invention, the modified AAV capsid includes a modified AAV9 capsid protein VP1 comprising the substitution W695R (AAV9 Capsid VP1 W695R) and having the amino acid sequence of SEQ ID No: 350.

In some embodiments of the invention, the modified AAV capsid includes a modified AAV9 capsid protein VP2 comprising the substitution W558R (AAV9 Capsid VP2 W558R) and having the amino acid sequence of SEQ ID No: 352.

In some embodiments of the invention, the modified AAV capsid includes a modified AAV9 capsid protein VP3 comprising the substitution W493R (AAV9 Capsid VP3 W493R) and having the amino acid sequence of SEQ ID No: 354.

In some embodiments of the invention, the modified AAV capsid includes a modified AAV9 capsid protein VP1 comprising the substitution Q351A (AAV9 Capsid VP1 Q351A) and having the amino acid sequence of SEQ ID No: 356.

In some embodiments of the invention, the modified AAV capsid includes a modified AAV9 capsid protein VP2 comprising the substitution Q214A (AAV9 Capsid VP2 Q214A) and having the amino acid sequence of SEQ ID No: 358.

In some embodiments of the invention, the modified AAV capsid includes a modified AAV9 capsid protein VP3 comprising the substitution Q149A (AAV9 Capsid VP3 Q149A) and having the amino acid sequence of SEQ ID No: 360.

In some embodiments of the invention, the modified AAV capsid includes a modified AAV9 capsid protein VP1 comprising the substitution Y395F (AAV9 Capsid VP1 Y395F) and having the amino acid sequence of SEQ ID No: 362.

In some embodiments of the invention, the modified AAV capsid includes a modified AAV9 capsid protein VP2 comprising the substitution Y258F (AAV9 Capsid VP2 Y258F) and having the amino acid sequence of SEQ ID No: 364.

In some embodiments of the invention, the modified AAV capsid includes a modified AAV9 capsid protein VP3 comprising the substitution Y193F (AAV9 Capsid VP3 Y193F) and having the amino acid sequence of SEQ ID No: 366.

In some embodiments of the invention, the modified AAV capsid includes a modified AAV9 capsid protein VP1 comprising the substitution R434L (AAV9 Capsid VP1 R434L) and having the amino acid sequence of SEQ ID No: 368.

In some embodiments of the invention, the modified AAV capsid includes a modified AAV9 capsid protein VP2 comprising the substitution R297L (AAV9 Capsid VP2 R297L) and having the amino acid sequence of SEQ ID No: 370.

In some embodiments of the invention, the modified AAV capsid includes a modified AAV9 capsid protein VP3 comprising the substitution (AAV9 Capsid VP3 R232L) and having the amino acid sequence of SEQ ID No: 372.

All of the above one or more substitutions in the modified AAV capsid are calculated relative to the corresponding wild-type capsid.

The naturally-occurring sequence of wild-type AAV5 capsid protein VP1 (AAV5 Capsid VP1 WT) is represented by the amino acid sequence of SEQ ID No: 2, the naturally-occurring sequence of wild-type AAV5 capsid protein VP2 (AAV5 Capsid VP2 WT) is represented by the amino acid sequence of SEQ ID No: 4, the naturally-occurring sequence of wild-type AAV5 capsid protein VP3 (AAV5 Capsid VP3 WT) is represented by the amino acid sequence of SEQ ID No: 6.

The naturally-occurring sequence of wild-type AAV9 capsid protein VP1 (AAV9 Capsid VP1 WT) is represented by the amino acid sequence of SEQ ID No: 373, the naturally-occurring sequence of wild-type AAV9 capsid protein VP2 (AAV9 Capsid VP2 WT) is represented by the amino acid sequence of SEQ ID No: 375, the naturally-occurring sequence of wild-type AAV9 capsid protein VP3 (AAV9 Capsid VP3 WT) is represented by the amino acid sequence of SEQ ID No: 377.

The authors of the invention have found that the above modified AAV capsids have one or more improved properties as compared to AAV capsid free of said modifications (wild-type AAV capsid), which are selected from the group:
- increased efficiency of cell transduction,
- increased production of target protein,
- increased efficiency of generation due to highly efficient production (assembly) of encapsidated viral vectors based on recombinant adeno-associated virus (rAAV).

### Isolated nucleic acid encoding modified capsid according to the invention

In one aspect, the present invention relates to a nucleic acid that encodes any of the above modified capsids.

In any embodiment of the invention, the nucleic acid molecule may be isolated.

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, determining a fragment or a region of a nucleic acid, containing unnatural nucleotides or not, and being either a double-strand DNA or RNA, a single-strand DNA or RNA, or transcription products of said DNAs.

As used in the present description, polynucleotides include, as non-limiting examples, all nucleic acid sequences which are obtained by any means available in the art, including, as non-limiting examples, recombinant means, i.e. the cloning of nucleic acid sequences from a recombinant library or a cell genome, using ordinary cloning technology and PCR and the like, and by synthetic means.

It should also be included here that the present invention does not relate to nucleotide sequences in their natural chromosomal environment, i.e. in a natural state. The sequences of the present invention have been isolated and/or purified, i.e. they were sampled directly or indirectly, for example by copying. Thus, isolated nucleic acids obtained by recombinant genetics, by means, for example, of host cells, or obtained by chemical synthesis should also be mentioned here.

A reference to a nucleotide sequence encompasses the complement thereof unless otherwise specified. Thus, a reference to a nucleic acid having a particular sequence should be understood as one which encompasses the complementary strand thereof with the complementary sequence thereof.

An "isolated" nucleic acid molecule is one which is identified and separated from at least one nucleic acid molecule-impurity, which the former is bound to in the natural source of antibody nucleic acid. An isolated nucleic acid molecule is different from the form or set in which it is found under natural conditions. Thus, an isolated nucleic acid molecule is different from a nucleic acid molecule that exists in cells under natural conditions.

In some embodiments of the invention, the isolated nucleic acid is DNA.

In some embodiments of the invention, the isolated nucleic acid encoding a modified capsid includes a nucleic acid encoding a modified AAV5 capsid protein VP1 comprising the substitution G226A (AAV5 Capsid VP1 G226A) that has the nucleotide sequence of SEQ ID No: 13 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution G90A (AAV5 Capsid VP2 G90A) that has the nucleotide sequence of SEQ ID No: 15 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution G34A (AAV5 Capsid VP3 G34A) that has the nucleotide sequence of SEQ ID No: 17 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A, G226A and T711S (AAV5 Capsid VP1 S2A, G226A and T711S) that has the nucleotide sequence of SEQ ID No: 19 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions G90A and T575S (AAV5 Capsid VP2 G90A and T575S) that has the nucleotide sequence of SEQ ID No: 21 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions G34A T519S (AAV5 Capsid VP3 G34A T519S) that has the nucleotide sequence of SEQ ID No: 23 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution D286E (AAV5 Capsid VP1 D286E) that has the nucleotide sequence of SEQ ID No: 25 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution D150E (AAV5 Capsid VP2 D150E) that has the nucleotide sequence of SEQ ID No: 27 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution D94E (AAV5 Capsid VP3 D94E) that has the nucleotide sequence of SEQ ID No: 29 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A, D286E and T711S (AAV5 Capsid VP1 S2A, D286E and T711S) that has the nucleotide sequence of SEQ ID No: 31 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions D150E and T575S (AAV5 Capsid VP2 D150E and T575S) that has the nucleotide sequence of SEQ ID No: 33 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions D94E and T519S (AAV5 Capsid VP3 D94E and T519S) that has the nucleotide sequence of SEQ ID No: 35 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution L341Y (AAV5 Capsid VP1 L341Y) that has the nucleotide sequence of SEQ ID No: 37 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution L205Y (AAV5 Capsid VP2 L205Y) that has the nucleotide sequence of SEQ ID No: 39 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution L149Y (AAV5 Capsid VP3 L149Y) that has the nucleotide sequence of SEQ ID No: 41 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A, L341Y, T711S (AAV5 Capsid VP1 S2A, L341Y, T711S) that has the nucleotide sequence of SEQ ID No: 43 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions L205Y, T575S (AAV5 Capsid VP2 L205Y, T575S) that has the nucleotide sequence of SEQ ID No: 45 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions L149Y, T519S (AAV5 Capsid VP3 L149Y, T519S) that has the nucleotide sequence of SEQ ID No: 47 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution C387V (AAV5 Capsid VP1 C387V) that has the nucleotide sequence of SEQ ID No: 49 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution C251V (AAV5 Capsid VP2 C251V) that has the nucleotide sequence of SEQ ID No: 51 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution C195V (AAV5 Capsid VP3 C195V) that has the nucleotide sequence of SEQ ID No: 53 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A, C387V, T711S (AAV5 Capsid VP1 S2A, C387V, T711S) that has the nucleotide sequence of SEQ ID No: 55 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions C251V, T575S (AAV5 Capsid VP2 C251V, T575S) that has the nucleotide sequence of SEQ ID No: 57 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions C195V, T519S (AAV5 Capsid VP3 C195V, T519S) that has the nucleotide sequence of SEQ ID No: 59 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution Q421H (AAV5 Capsid VP1 Q421H) that has the nucleotide sequence of SEQ ID No: 61 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution Q285H (AAV5 Capsid VP2 Q285H) that has the nucleotide sequence of SEQ ID No: 63 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution Q229H (AAV5 Capsid VP3 Q229H) that has the nucleotide sequence of SEQ ID No: 65 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A Q421H T711S (AAV5 Capsid VP1 S2A Q421H T711S) that has the nucleotide sequence of SEQ ID No: 67 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions Q285H T575S (AAV5 Capsid VP2 Q285H T575S) that has the nucleotide sequence of SEQ ID No: 69 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions Q229H T519S (AAV5 Capsid VP3 Q229H T519S) that has the nucleotide sequence of SEQ ID No: 71 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution P466T (AAV5 Capsid VP1 P466T) that has the nucleotide sequence of SEQ ID No: 73 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution P330T (AAV5 Capsid VP2 P330T) that has the nucleotide sequence of SEQ ID No: 75 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution P274T (AAV5 Capsid VP3 P274T) that has the nucleotide sequence of SEQ ID No: 77 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A P466T T711S (AAV5 Capsid VP1 S2A P466T T711S) that has the nucleotide sequence of SEQ ID No: 79 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions P330T T575S (AAV5 Capsid VP2 P330T T575S) that has the nucleotide sequence of SEQ ID No: 81 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions P274T T519S (AAV5 Capsid VP3 P274T T519S) that has the nucleotide sequence of SEQ ID No: 83 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution S594Q (AAV5 Capsid VP1 S594Q) that has the nucleotide sequence of SEQ ID No: 85 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution S458Q (AAV5 Capsid VP2 S458Q) that has the nucleotide sequence of SEQ ID No: 87 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution S402Q (AAV5 Capsid VP3 S402Q) that has the nucleotide sequence of SEQ ID No: 89 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A S594Q T711S (AAV5 Capsid VP1 S2A S594Q T711S) that has the nucleotide sequence of SEQ ID No: 91 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions S458Q T575S (AAV5 Capsid VP2 S458Q T575S) that has the nucleotide sequence of SEQ ID No: 93 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions S402Q T519S (AAV5 Capsid VP3 S402Q T519S) that has the nucleotide sequence of SEQ ID No: 95 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions T614L (AAV5 Capsid VP1 T614L) that has the nucleotide sequence of SEQ ID No: 97 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions T478L (AAV5 Capsid VP2 T478L) that has the nucleotide sequence of SEQ ID No: 99 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions T422L (AAV5 Capsid VP3 T422L) that has the nucleotide sequence of SEQ ID No: 101 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A T614L T711S (AAV5 Capsid VP1 S2A T614L T711S) that has the nucleotide sequence of SEQ ID No: 103 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions T478L T575S (AAV5 Capsid VP2 T478L T575S) that has the nucleotide sequence of SEQ ID No: 105 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions (AAV5 Capsid VP3 T422L T519S) that has the nucleotide sequence of SEQ ID No: 107 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution N679K (AAV5 Capsid VP1 N679K) that has the nucleotide sequence of SEQ ID No: 109 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution N543K (AAV5 Capsid VP2 N543K) that has the nucleotide sequence of SEQ ID No: 111 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution N487K (AAV5 Capsid VP3 N487K) that has the nucleotide sequence of SEQ ID No: 113 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A N679K T711S (AAV5 Capsid VP1 S2A N679K T711S) that has the nucleotide sequence of SEQ ID No: 115 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions N543K T575S (AAV5 Capsid VP2 N543K T575S) that has the nucleotide sequence of SEQ ID No: 117 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions N487K T519S (AAV5 Capsid VP3 N487K T519S) that has the nucleotide sequence of SEQ ID No: 119 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution P723V (AAV5 Capsid VP1 P723V) that has the nucleotide sequence of SEQ ID No: 121 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution P587V (AAV5 Capsid VP2 P587V) that has the nucleotide sequence of SEQ ID No: 123 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution P531V (AAV5 Capsid VP3 P531V) that has the nucleotide sequence of SEQ ID No: 125 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions P723V T711S (AAV5 Capsid VP1 S2A P723V T711S) that has the nucleotide sequence of SEQ ID No: 127 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions P587V T575S (AAV5 Capsid VP2 P587V T575S) that has the nucleotide sequence of SEQ ID No: 129 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions P531V T519S (AAV5 Capsid VP3 P531V T519S) that has the nucleotide sequence of SEQ ID No: 131 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution V431Y (AAV5 Capsid VP1 V431Y) that has the nucleotide sequence of SEQ ID No: 133 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution V295Y (AAV5 Capsid VP2 V295Y) that has the nucleotide sequence of SEQ ID No: 135 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution V239Y (AAV5 Capsid VP3 V239Y) that has the nucleotide sequence of SEQ ID No: 137 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A V431Y T711S (AAV5 Capsid VP1 S2A V431Y T711S) that has the nucleotide sequence of SEQ ID No: 139 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions V295Y T575S (AAV5 Capsid VP2 V295Y T575S) that has the nucleotide sequence of SEQ ID No: 141 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions V239Y T519S (AAV5 Capsid VP3 V239Y T519S) that has the nucleotide sequence of SEQ ID No: 143 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution A616D (AAV5 Capsid VP1 A616D) that has the nucleotide sequence of SEQ ID No: 145 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution A480D (AAV5 Capsid VP2 A480D) that has the nucleotide sequence of SEQ ID No: 147 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution A424D (AAV5 Capsid VP3 A424D) that has the nucleotide sequence of SEQ ID No: 149 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A A616D T711S (AAV5 Capsid VP1 S2A A616D T711S) that has the nucleotide sequence of SEQ ID No: 151 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions A480D T575S (AAV5 Capsid VP2 A480D T575S) that has the nucleotide sequence of SEQ ID No: 153 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions A424D T519S (AAV5 Capsid VP3 A424D T519S) that has the nucleotide sequence of SEQ ID No: 155 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution W683R (AAV5 Capsid VP1 W683R) that has the nucleotide sequence of SEQ ID No: 157 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution W547R (AAV5 Capsid VP2 W547R) that has the nucleotide sequence of SEQ ID No: 159 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution W491R (AAV5 Capsid VP3 W491R) that has the nucleotide sequence of SEQ ID No: 161 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A W683R T711S (AAV5 Capsid VP1 S2A W683R T711S) that has the nucleotide sequence of SEQ ID No: 163 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions W547R T575S (AAV5 Capsid VP2 W547R T575S) that has the nucleotide sequence of SEQ ID No: 165 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions W491R T519S (AAV5 Capsid VP3 W491R T519S) that has the nucleotide sequence of SEQ ID No: 167 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution S222A (AAV5 Capsid VP1 S222A) that has the nucleotide sequence of SEQ ID No: 169 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution S86A (AAV5 Capsid VP2 S86A) that has the nucleotide sequence of SEQ ID No: 171 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution S30A (AAV5 Capsid VP3 S30A) that has the nucleotide sequence of SEQ ID No: 173 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A S222A T711S (AAV5 Capsid VP1 S2A S222A T711S) that has the nucleotide sequence of SEQ ID No: 175 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions S86A T575S (AAV5 Capsid VP2 S86A T575S) that has the nucleotide sequence of SEQ ID No: 177 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions S30A T519S (AAV5 Capsid VP3 S30A T519S) that has the nucleotide sequence of SEQ ID No: 179 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution R285L (AAV5 Capsid VP1 R285L) that has the nucleotide sequence of SEQ ID No: 181 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution R149L (AAV5 Capsid VP2 R149L) that has the nucleotide sequence of SEQ ID No: 183 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution R93L (AAV5 Capsid VP3 R93L) that has the nucleotide sequence of SEQ ID No: 185 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A R285L T711S (AAV5 Capsid VP1 S2A R285L T711S) that has the nucleotide sequence of SEQ ID No: 187 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions R149L T575S (AAV5 Capsid VP2 R149L T575S) that has the nucleotide sequence of SEQ ID No: 189 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions R93L T519S (AAV5 Capsid VP3 R93L T519S) that has the nucleotide sequence of SEQ ID No: 191 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution Q340A (AAV5 Capsid VP1 Q340A) that has the nucleotide sequence of SEQ ID No: 193 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution (AAV5 Capsid VP2 Q204A) that has the nucleotide sequence of SEQ ID No: 195 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution Q148A (AAV5 Capsid VP3 Q148A) that has the nucleotide sequence of SEQ ID No: 197 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A Q340A T711S (AAV5 Capsid VP1 S2A Q340A T711S) that has the nucleotide sequence of SEQ ID No: 199 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions Q204A T575S (AAV5 Capsid VP2 Q204A T575S) that has the nucleotide sequence of SEQ ID No: 201 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions Q148A T519S (AAV5 Capsid VP3 Q148A T519S) that has the nucleotide sequence of SEQ ID No: 203 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution S420F (AAV5 Capsid VP1 S420F) that has the nucleotide sequence of SEQ ID No: 205 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution S284F (AAV5 Capsid VP2 S284F) that has the nucleotide sequence of SEQ ID No: 207 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution S228F (AAV5 Capsid VP3 S228F) that has the nucleotide sequence of SEQ ID No: 209 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A S420F T711S (AAV5 Capsid VP1 S2A S420F T711S) that has the nucleotide sequence of SEQ ID No: 211 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions S284F T575S (AAV5 Capsid VP2 S284F T575S) that has the nucleotide sequence of SEQ ID No: 213 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions S228F T519S (AAV5 Capsid VP3 S228F T519S) that has the nucleotide sequence of SEQ ID No: 215 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution S680A (AAV5 Capsid VP1 S680A) that has the nucleotide sequence of SEQ ID No: 217 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution S544A (AAV5 Capsid VP2 S544A) that has the nucleotide sequence of SEQ ID No: 219 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution S488A (AAV5 Capsid VP3 S488A) that has the nucleotide sequence of SEQ ID No: 221 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A S680A T711S (AAV5 Capsid VP1 S2A S680A T711S) that has the nucleotide sequence of SEQ ID No: 223 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions S544A T575S (AAV5 Capsid VP2 S544A T575S) that has the nucleotide sequence of SEQ ID No: 225 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions S488A T519S (AAV5 Capsid VP3 S488A T519S) that has the nucleotide sequence of SEQ ID No: 227 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitution T721V (AAV5 Capsid VP1 T721V) that has the nucleotide sequence of SEQ ID No: 229 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitution T585V (AAV5 Capsid VP2 T585V) that has the nucleotide sequence of SEQ ID No: 231 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitution T529V (AAV5 Capsid VP3 T529V) that has the nucleotide sequence of SEQ ID No: 233 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP1 comprising the substitutions S2A T721V T711S (AAV5 Capsid VP1 S2A T721V T711S) that has the nucleotide sequence of SEQ ID No: 235 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP2 comprising the substitutions T585V T575S (AAV5 Capsid VP2 T585V T575S) that has the nucleotide sequence of SEQ ID No: 237 or any other nucleotide sequence encoding the corresponding amino acid sequence of the given modified protein.

In some embodiments of the invention, the isolated nucleic acid encoding a modified capsid includes a nucleic acid encoding a modified AAV5 capsid protein VP3 comprising the substitutions T529V T519S (AAV5 Capsid VP3 T529V T519S) that has the nucleotide sequence of SEQ ID No: 239 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution Q351K (AAV9 Capsid VP1 Q351K) that has the nucleotide sequence of SEQ ID No: 241 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution Q214K (AAV9 Capsid VP2 Q214K) that has the nucleotide sequence of SEQ ID No: 243 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution Q149K (AAV9 Capsid VP3 Q149K) that has the nucleotide sequence of SEQ ID No: 245 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution Q351R (AAV9 Capsid VP1 Q351R) that has the nucleotide sequence of SEQ ID No: 247 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution Q214R (AAV9 Capsid VP2 Q214R) that has the nucleotide sequence of SEQ ID No: 249 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution Q149R (AAV9 Capsid VP3 Q149R) that has the nucleotide sequence of SEQ ID No: 251 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution S232T (AAV9 Capsid VP1 S232T) that has the nucleotide sequence of SEQ ID No: 253 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution S95T (AAV9 Capsid VP2 S95T) that has the nucleotide sequence of SEQ ID No: 255 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution S30T (AAV9 Capsid VP3 S30T) that has the nucleotide sequence of SEQ ID No: 257 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution D297E (AAV9 Capsid VP1 D297E) that has the nucleotide sequence of SEQ ID No: 259 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution D160E (AAV9 Capsid VP2 D160E) that has the nucleotide sequence of SEQ ID No: 261 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution D95E (AAV9 Capsid VP3 D95E) that has the nucleotide sequence of SEQ ID No: 263 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution S692T (AAV9 Capsid VP1 S692T) that has the nucleotide sequence of SEQ ID No: 265 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution S555T (AAV9 Capsid VP2 S555T) that has the nucleotide sequence of SEQ ID No: 267 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution S490T (AAV9 Capsid VP3 S490T) that has the nucleotide sequence of SEQ ID No: 269 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution D433Y (AAV9 Capsid VP1 D433Y) that has the nucleotide sequence of SEQ ID No: 271 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution D296Y (AAV9 Capsid VP2 D296Y) that has the nucleotide sequence of SEQ ID No: 273 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution D231Y (AAV9 Capsid VP3 D231Y) that has the nucleotide sequence of SEQ ID No: 275 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution T625L (AAV9 Capsid VP1 T625L) that has the nucleotide sequence of SEQ ID No: 277 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution T488L (AAV9 Capsid VP2 T488L) that has the nucleotide sequence of SEQ ID No: 279 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution T423L (AAV9 Capsid VP3 T423L) that has the nucleotide sequence of SEQ ID No: 281 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution T625V (AAV9 Capsid VP1 T625V) that has the nucleotide sequence of SEQ ID No: 283 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution T488V (AAV9 Capsid VP2 T488V) that has the nucleotide sequence of SEQ ID No: 285 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution T423V (AAV9 Capsid VP3 T423V) that has the nucleotide sequence of SEQ ID No: 287 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution N691A (AAV9 Capsid VP1 N691A) that has the nucleotide sequence of SEQ ID No: 289 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution N554A (AAV9 Capsid VP2 N554A) that has the nucleotide sequence of SEQ ID No: 291 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution N489A (AAV9 Capsid VP3 N489A) that has the nucleotide sequence of SEQ ID No: 293 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution C396V (AAV9 Capsid VP1 C396V) that has the nucleotide sequence of SEQ ID No: 295 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution C259V (AAV9 Capsid VP2 C259V) that has the nucleotide sequence of SEQ ID No: 297 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution C194V (AAV9 Capsid VP3 C194V) that has the nucleotide sequence of SEQ ID No: 299 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution Y478F (AAV9 Capsid VP1 Y478F) that has the nucleotide sequence of SEQ ID No: 301 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution Y341F (AAV9 Capsid VP2 Y341F) that has the nucleotide sequence of SEQ ID No: 303 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution Y276F (AAV9 Capsid VP3 Y276F) that has the nucleotide sequence of SEQ ID No: 305 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution R296L (AAV9 Capsid VP1 R296L) that has the nucleotide sequence of SEQ ID No: 307 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution R159L (AAV9 Capsid VP2 R159L) that has the nucleotide sequence of SEQ ID No: 309 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution R94L (AAV9 Capsid VP3 R94L) that has the nucleotide sequence of SEQ ID No: 311 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution T733V (AAV9 Capsid VP1 T733V) that has the nucleotide sequence of SEQ ID No: 313 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution T596V (AAV9 Capsid VP2 T596V) that has the nucleotide sequence of SEQ ID No: 315 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution T531V (AAV9 Capsid VP3 T531V) that has the nucleotide sequence of SEQ ID No: 317 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution A427R (AAV9 Capsid VP1 A427R) that has the nucleotide sequence of SEQ ID No: 319 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution A290R (AAV9 Capsid VP2 A290R) that has the nucleotide sequence of SEQ ID No: 321 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution A225R (AAV9 Capsid VP3 A225R) that has the nucleotide sequence of SEQ ID No: 323 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution M635D (AAV9 Capsid VP1 M635D) that has the nucleotide sequence of SEQ ID No: 325 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution M498D (AAV9 Capsid VP2 M498D) that has the nucleotide sequence of SEQ ID No: 327 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution M433D (AAV9 Capsid VP3 M433D) that has the nucleotide sequence of SEQ ID No: 329 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution L444R (AAV9 Capsid VP1 L444R) that has the nucleotide sequence of SEQ ID No: 331 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution L307R (AAV9 Capsid VP2 L307R) that has the nucleotide sequence of SEQ ID No: 333 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution L242R (AAV9 Capsid VP3 L242R) that has the nucleotide sequence of SEQ ID No: 335 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution G604N (AAV9 Capsid VP1 G604N) that has the nucleotide sequence of SEQ ID No: 337 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution G467N (AAV9 Capsid VP2 G467N) that has the nucleotide sequence of SEQ ID No: 339 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution G402N (AAV9 Capsid VP3 G402N) that has the nucleotide sequence of SEQ ID No: 341 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution G627K (AAV9 Capsid VP1 G627K) that has the nucleotide sequence of SEQ ID No: 343 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution G490K (AAV9 Capsid VP2 G490K) that has the nucleotide sequence of SEQ ID No: 345 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution G425K (AAV9 Capsid VP3 G425K) that has the nucleotide sequence of SEQ ID No: 347 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution W695R (AAV9 Capsid VP1 W695R) that has the nucleotide sequence of SEQ ID No: 349 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution W558R (AAV9 Capsid VP2 W558R) that has the nucleotide sequence of SEQ ID No: 351 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution W493R (AAV9 Capsid VP3 W493R) that has the nucleotide sequence of SEQ ID No: 353 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution Q351A (AAV9 Capsid VP1 Q351A) that has the nucleotide sequence of SEQ ID No: 355 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution Q214A (AAV9 Capsid VP2 Q214A) that has the nucleotide sequence of SEQ ID No: 357 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution Q149A (AAV9 Capsid VP3 Q149A) that has the nucleotide sequence of SEQ ID No: 359 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution Y395F (AAV9 Capsid VP1 Y395F) that has the nucleotide sequence of SEQ ID No: 361 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution Y258F (AAV9 Capsid VP2 Y258F) that has the nucleotide sequence of SEQ ID No: 363 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution Y193F (AAV9 Capsid VP3 Y193F) that has the nucleotide sequence of SEQ ID No: 365 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP1 comprising the substitution R434L (AAV9 Capsid VP1 R434L) that has the nucleotide sequence of SEQ ID No: SEQ ID No: 367 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP2 comprising the substitution R297L (AAV9 Capsid VP2 R297L) that has the nucleotide sequence of SEQ ID No: 369 or any other nucleotide sequence encoding the corresponding amino acid sequence of the given modified protein.

In some embodiments of the invention, the isolated nucleic acid encoding a modified capsid includes a nucleic acid encoding a modified AAV9 capsid protein VP3 comprising the substitution (AAV9 Capsid VP3 R232L) that has the nucleotide sequence of SEQ ID No: 371 or any other nucleotide sequence encoding the corresponding amino acid sequence of the given modified protein.

"Any other nucleotide sequence encoding the corresponding amino acid sequence of the given modified protein" refers to a nucleic sequence that is alternative to said nucleic sequence, as, due to the degeneracy of genetic code, a wide range of various DNA sequences can encode the same amino acid sequence. It is well within the skill of a person trained in the art to create these alternative DNA sequences encoding the same amino acid sequences. Such variant DNA sequences are within the scope of the present invention.

In some embodiments, the isolated nucleic acid encoding any of the above modified capsids comprises any of the above nucleic acid sequences or combinations thereof.

### Recombinant adeno-associated virus-based vector

In one aspect, the present invention relates to a vector based on recombinant adeno-associated virus for delivering to a subject a heterologous nucleic acid sequence, which includes:
1) any of the above modified capsids, and
2) a heterologous nucleic acid sequence comprising regulatory sequences that promote the expression of the product encoded by the heterologous nucleic acid sequence, in the target cells.

The terms "vector based on recombinant adeno-associated virus", "recombinant virus based on AAV", "virus-like particle based on AAV", "recombinant viral AAV strain", "recombinant AAV vector" or "rAAV vector" as used in the present description have the same meaning.

In some embodiments of the invention, the rAAV vector includes a heterologous nucleic acid sequence comprising regulatory sequences that provide product expression, wherein the product of expression of the heterologous nucleic acid sequence is a therapeutic polypeptide or reporter polypeptide.

The rAAV vector according to the invention does not comprise nucleotide sequences of genes encoding sequences of proteins (Rep) that are essential for the virus life cycle, as well as sequences of overlapping capsid proteins (Cap).

The capsid is characterized in detail in the above section of the description.

"Regulatory sequences that provide the expression of product encoded by the heterologous nucleic acid sequence in target cells", as used in the present invention, mean polynucleotide sequences that are necessary to influence the expression and processing of coding sequences to which they are cloned. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (i.e. Kozak consensus sequence); sequences that enhance protein stability; and when desired, sequences that enhance protein secretion. The nature of such control sequences differs depending upon the host organism; in prokaryotes, such control sequences generally include the promoter of ribosome binding site, and transcription termination sequences; in eukaryotes, typically, such control sequences include promoters and transcription termination sequences. The term "regulatory sequences" includes at least all components, the presence of which is essential for expression and processing, and can also include additional components, the presence of which is advantageous, for example, leader peptide sequences.

As used in the present description, the term "promoter" refers to a nucleic acid fragment that controls the transcription of one or more coding sequences and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art that directly or indirectly regulate the level of transcription with said promoter. A "constitutive" promoter is a promoter that is active in most tissues under typical physiological and developmental conditions. An "inducible" promoter is a promoter that is physiologically or developmentally regulated, e.g. under the influence of a chemical inducer. A "tissue specific" promoter is only active in specific types of tissues or cells.

Promoters that are used for high-level production of polypeptides in eukaryotic cells and, in particular, in mammalian cells, should be strong and preferably active in a wide range of cell types. Strong constitutive promoters which are capable of driving expression in many cell types are well known in the art and, therefore, it is not herein necessary to describe them in detail. In accordance with the idea of the present invention, it is preferable to use the cytomegalovirus (CMV) promoter. A promoter or promoter/enhancer derived from the immediate early (IE) region of human cytomegalovirus (hCMV) is particularly suitable as a promoter for the rAAV5 vector of the present invention. Human cytomegalovirus (hCMV) immediate early (IE) region and functional expression-inducing and/or functional expression-enhancing fragments derived therefrom, for example, have been disclosed in EP0173177 and EP0323997, and are well known in the art. Thus, several fragments of the hCMV immediate early (IE) region may be used as a promoter and/or promoter/enhancer.

The terms "enhancers" or "enhancer" as used herein may refer to a DNA sequence that is located adjacent to the DNA sequence that encodes a recombinant product. Enhancer elements are typically located in a 5' direction from a promoter element or can be located downstream of or within a coding DNA sequence (e.g. a DNA sequence transcribed or translated into a recombinant product or products). Hence, an enhancer element may be located 100 base pairs, 200 base pairs, or 300 or more base pairs upstream of a DNA sequence that encodes a recombinant product, or downstream of said sequence. Enhancer elements may increase the amount of a recombinant product being expressed from a DNA sequence above the level of expression associated with a single promoter element. Multiple enhancer elements are readily available to those of ordinary skill in the art.

In some embodiments of the invention, the heterologous nucleic acid sequence comprising regulatory sequences providing the expression of a product encoded by the heterologous nucleic acid sequence in target cells may include the following elements in the 5'-end to 3'-end direction:
a left-hand (first) ITR (inverted terminal repeats);
A CMV (cytomegalovirus) enhancer;
A CMV (cytomegalovirus) promoter;
an intron of the hBG1 gene (hemoglobin subunit gamma 1 gene);
a nucleic acid encoding the product;
an hGH1 polyadenylation signal (human growth hormone gene polyadenylation signal);
a right-hand (second) ITR.

In some embodiments, the nucleic acid encoding the product (transgene) is at least one gene encoding a protein. In some embodiments, the transgene encodes at least one small inhibitory nucleic acid. In some embodiments, the transgene encodes at least one reporter molecule. In some embodiments, the small inhibitory nucleic acid is miRNA. In some embodiments, the small inhibitory nucleic acid is sponge miRNA or TuD-RNA, which inhibits the activity of at least one miRNA in an animal. In some embodiments, miRNA is expressed in a cell of the target tissue. In some embodiments, the target tissue is the tissue of the liver, central nervous system (CNS), eyes, gastrointestinal tract, respiratory tract, breast, pancreas, urinary tract or uterine tissue.

In some embodiments, the rAAV vector has a product of expression of the heterologous nucleic acid sequence, which is a therapeutic polypeptide or a reporter polypeptide.

In some embodiments, the rAAV vector comprises a heterologous nucleic acid sequence encoding a product that is a therapeutic polypeptide, wherein the therapeutic polypeptide is a coagulation factor selected from the group consisting of Factor VIII, Factor IX, or a functional variant thereof.

In some embodiments, the rAAV vector comprises a heterologous nucleic acid sequence encoding a product that is Factor VIII or a functional variant thereof.

In some embodiments, the rAAV vector comprises a heterologous nucleic acid sequence encoding a product that is Factor IX or a functional variant thereof.

In some embodiments, the rAAV vector comprises a heterologous nucleic acid sequence encoding a product that is the SMN1 protein (survival motor neuron protein)

In some embodiments, the rAAV vector comprises a heterologous nucleic acid sequence encoding a product that is the RBD-S polypeptide (recombinant receptor-binding domain of S glycoprotein) of SARS-cov2 (severe acute respiratory syndrome coronavirus 2).

### Examples

The following examples are provided for better understanding of the invention. These examples are for purposes of illustration only and are not to be construed as limiting the scope of the invention in any manner.

All publications, patents, and patent applications cited in this specification are incorporated herein by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended embodiments.

### Materials and general methods

### Recombinant DNA techniques

Standard methods were used to manipulate DNA as described in Sambrook, J. et al, Molecular cloning: A laboratory manual; Cold Spring Harbor Laboratory Press, Cold Spring Harbor, New York, 2012. The molecular biological reagents were used according to the manufacturer protocols.

### Gene synthesis

Desired gene segments were prepared from oligonucleotides made by chemical synthesis. The gene segments of 300-4,000 bp long, which were flanked by singular restriction sites, were assembled by annealing and ligation of oligonucleotides including PCR amplification and subsequently cloned via the specified restriction sites. The DNA sequences of the subcloned gene fragments were confirmed by DNA sequencing.

### DNA sequence determination

DNA sequences were determined by Sanger sequencing.

### DNA and protein sequence analysis and sequence data management

The Infomax's Vector NTI Advance suite version 8.0 and SnapGene version 5.1.4.1 were used for sequence creation, mapping, analysis, annotation and illustration.

### Statistical data analysis

The results indicate an average value ± standard deviation (SD), analysis of variance (ANOVA) was employed to compare the test and control results, and they were determined to be statistically significant.

### Example 1.

### Selection of mutation variants in AAV5 capsid based on structural similarity of capsids of the family Parvoviridae

The design of AAV5 capsid mutations is based on a high degree of structural similarity of capsid proteins within the family Parvoviridae, including non-AAV viruses. The selection of a substitution consists of three steps of:
1) determining amino acids in AAV5 capsomere protein, which are located in the interaction interface region of adjacent pentameric subunits of the capsid;
2) aligning structurally the modified AAV5 capsid to a capsid of one of the non-AAV parvoviruses (hereinafter referred to as "template capsid");
3) comparing pairwisely amino acid residues of the AAV5 capsid and those of the template capsid.

The structures of the three-dimensional models of AAV5 and the template capsid are pre-prepared; the pre-preparation consists in completing the missing atoms in the structure using the Prepwizard utility from Schrodinger Suite.

In step 1, the interaction interface refers to a set of capsomere protein polypeptide chain fragments interacting with the amino acids of an adjacent pentameric subunit, wherein the pentameric subunit refers to a capsid structural element having the shape of a regular pentagon and consisting of 5 capsomere proteins located around the central pore (L. M. Drouin and M. Agbandje-McKenna. Adeno-associated virus structural biology as a tool in vector development. Future Virol., vol. 8, no. 12, pp. 1183-1199, 2013.).

The second step comprises aligning structurally the AAV5 capsid and the template capsid using the Structure Alignment utility from Schrodinger Suite. Following the results of alignment, for each AAV5 amino acid from the interaction interface of adjacent pentameric subunits, the closest thereto amino acid of the template capsid (structural analogue of the initial amino acid) is determined, which is considered a potential substitution. There is no structural analogue of the initial amino acid if in the vicinity of the nearest thereto amino acid of the template capsid there is present another AAV5 amino acid, the distance to which is less than that to the initial amino acid. Here, the distance between amino acids is estimated by that between alpha-carbon atoms.

After structural analogues have been determined for all amino acids of the interaction interface of adjacent pentameric subunits, the decision to substitute is made in accordance with Examples 2, 3 and 4.

### Example 2. Design of mutations based on the principle of increasing the volume of amino acid residue

After aligning structurally the AAV5 capsid to the template capsid, we get a pairwise structural correspondence between amino acid residues thereof. In one of the cases, the decision to substitute the initial amino acid for the corresponding amino acid of the template capsid is made following the results of comparing the van der Waals volumes of the amino acid side substituents (N. J. Darby, S. S. N. J. Darby, and T. E. Creighton, Protein Structure. IRL Press at Oxford University Press, 1993; Table 1). A mutation is introduced if the following conditions are met:
1. Volume of the amino acid side substituent from the template capsid is greater than that of the initial amino acid side substituent
2. An amino acid from the template capsid is not cysteine or methionine.

The exclusion of cysteine and methionine from the amino acids for potential substitution is due to their significant reactivity, in particular oxidizability.

**Table 1. Van der Waals volume for amino acid side substituents (N. J. Darby, S. S. N. J. Darby, and T. E. Creighton, Protein Structure. IRL Press at Oxford University Press, 1993.)**

| Three-letter code | One-letter code | Volume, Å³ |
|---|---|---|
| Ala | A | 67 |
| Arg | R | 148 |
| Asp | D | 96 |
| Asn | N | 91 |
| Cys | C | 86 |
| Gln | Q | 114 |
| Glu | E | 109 |
| Gly | G | 48 |
| His | H | 118 |
| Ile | I | 124 |
| Leu | L | 124 |
| Lys | K | 135 |
| Met | M | 124 |
| Phe | F | 135 |
| Pro | P | 90 |
| Ser | S | 73 |
| Thr | T | 93 |
| Trp | W | 163 |
| Tyr | Y | 141 |
| Val | V | 105 |

Table 2 shows the results of applying the subject method for designing mutations in the AAV5 capsid using three template capsids: HBoV1 (PDBid 5urf), B19 (PDBid 1s58), BPV (PDBid 4qc8). The first column contains amino acids from the interaction interface of adjacent pentameric subunits of the AAV5 capsid, the remaining columns contain amino acids produced after pairwise structural alignment of AAV5 to each one of the capsids: HBoV1, B19 and BPV. An asterisk indicates amino acids suitable for substitution in accordance with the size criterion for the side substituent and the prohibited substitution for cysteine and methionine.

Interaction interface for a given subunit is intended to refer to all amino acids, at least one atom of which is present at distance lower than 5 Å from any atom of adj acent pentameric subunits.

For AAV5, the calculation was made based on the capsid structure PDB ID: 6JCT.

According to the calculation, its interaction interface between adjacent pentameric AAV5 subunits includes the following ranges of AAV5 capsid protein VP1 residues: 220 - 226, 283 - 293, 339 - 342, 385 - 392, 412 - 435, 462 - 466, 590 - 598, 611 - 626, 677 - 685, 721-724.

**Table 2. Amino acids from the interaction interface of adjacent pentameric subunits of the AAV5 capsid and structurally corresponding thereto amino acids of the capsomeres B19, HBoV1 and BPV. An asterisk indicates amino acids whose side substituent is greater than that of the initial amino acid of the AAV5 capsomere. A dash indicates the fact that there is no structurally similar amino acid in the template capsid.**

| **AAV5** | **B19** | **HBoV1** | **BPV** |
|---|---|---|---|
| C220 | E* | G | G |
| D221 | G | G | G |
| S222 | A | T* | S |
| T223 | T | I* | H* |
| W224 | F | F | F |
| M225 | S | S | S |
| G226 | A* | E* | D* |
| S283 | S | S | S |
| P284 | P | P | P |
| R285 | L | N | Q |
| D286 | E* | D | D |
| W287 | F | W | W |
| Q288 | Q | Q | Q |
| R289 | H | H | R |
| L290 | L | L | L |
| I291 | I | V | T |
| N292 | E* | N | N |
| N293 | N | D* | E* |
| Y339 | Y | H | H |
| Q340 | K* | R* | A |
| L341 | Y* | Y* | Y* |
| P342 | P | P | P |
| F385 | F | L | F |
| F386 | Y* | Y* | F |
| C387 | V* | M | L* |
| L388 | L | L | L |
| E389 | E | E | E |
| Y390 | H | N | N |
| F391 | S | S | S |
| P392 | S | D* | D* |
| P412 | P | E* | E* |
| F413 | P | W* | W* |
| H414 | E | I* | V |
| S415 | N* | E* | N* |
| S416 | L* | N* | N* |
| F417 | E | N | E |
| A418 | G | I* | R* |
| P419 | C | T* | A |
| S420 | Q* | F* | Y* |
| Q421 | H* | S | I* |
| N422 | - | M | P |
| L423 | F* | P | P |
| F424 | Y* | Q | G |
| K425 | E | M | L |
| L426 | M | M | M |
| A427 | Y* | Y* | F* |
| N428 | N | N | N |
| P429 | P | P | P |
| L430 | L | L | K* |
| V431 | Y* | V | V |
| D432 | G | R* | P |
| Q433 | S | S | T |
| Y434 | R* | R* | R* |
| L435 | L | R* | R* |
| K462 | Q | S | T |
| N463 | N | N | S |
| W464 | F | W | W |
| F465 | M | M | M |
| P466 | P | S | T* |
| I590 | L | M | M |
| V591 | M | L* | F* |
| P592 | V* | P | P |
| G593 | G | N* | N* |
| S594 | S | Q* | Q* |
| V595 | V | M | V |
| W596 | W | w | w |
| M597 | N | D | D |
| E598 | R* | S | R* |
| I611 | I | V | K* |
| P612 | P | P | P |
| E613 | N | R* | R* |
| T614 | L* | V* | A |
| G615 | D* | N* | D* |
| A616 | D* | R* | K* |
| H617 | S | K* | H |
| F618 | F | T | T |
| H619 | K* | L* | I* |
| P620 | T* | L* | M |
| S621 | F* | D* | D* |
| P622 | A | T* | P |
| A623 | A | Q* | F* |
| M624 | L | D | D |
| G625 | G | G | G |
| G626 | G | S* | S* |
| K677 | P | K | R* |
| E678 | R* | R* | Y* |
| N679 | K* | G | A |
| S680 | A | T* | T* |
| K681 | T | K | K |
| R682 | G | N | N |
| W683 | R | W | W |
| N684 | N | R* | R* |
| P685 | P | P | P |
| T721 | V* | H* | N |
| R722 | H | K | K |
| P723 | P | V* | V* |
| L724 | L | L | L |

### Example 3. Design of mutations based on the principle of increasing the number of polar contacts between capsomeres.

In accordance with Example 1, after aligning structurally the AAV5 capsid and the template capsid, we get a pairwise structural correspondence between amino acid residues thereof. In one of the cases, the decision to substitute the initial amino acid for the corresponding amino acid of the template capsid is made based on the analysis of the microenvironment for both the initial amino acid and a structural analogue thereof. In particular, a positive decision to substitute is made if in a pair of corresponding amino acids in the template capsid there is disposed an amino acid with a polar side substituent, and in AAV5 there is disposed one with a non-polar substituent, wherein the both amino acids have at least one polar contact to an adjacent pentameric subunit of the capsid. An amino acid is assumed to have a polar contact if at a distance of no more than 5 Å from any atom of the side substituent other than hydrogen there is disposed at least one atom of the polar group of the side substituent of another amino acid of another capsomere (Table 3). By analogy with Example 2, substitutions for cysteine and methionine are also not allowed.

**Table 3. List of amino acids with polar side substituents and the atoms of polar groups thereof (name according to the PDB specification) with the exception of hydrogen atoms.**

| Amino acid | Atoms included in the polar side group (name according to the PDB specification) |
|---|---|
| Arg | NH1, NH2, CZ, NE |
| Asn | CG, OD1, ND2, |
| Asp | CG, OD1, OD2 |
| Cys | SG |
| Gln | CD, OE1, NE2 |
| Glu | CD, OE1, OE2 |
| His | ND1, CD2, CE1, NE2 |
| Lys | NZ |
| Ser | OG |
| Thr | OG1 |
| Tyr | OH |

Table 4 shows the results of applying the subject method for designing mutations in the AAV5 capsid using three template capsids: HBoV1 (PDBid 5urf), B19 (PDBid 1s58), BPV (4qc8). The first column contains amino acids from the interaction interface of adjacent pentameric subunits of the AAV5 capsid, for which suitable substitutions were found in one of the three capsids: HBoV1, B19 and BPV. The remaining columns contain amino acids produced after pairwise structural alignment of AAV5 to each of the capsids. An asterisk indicates amino acids suitable for substitution in accordance with the increased number criterion for the polar contacts and the prohibited substitution for cysteine and methionine.

**Table 4. AAV5 capsomere positions that were revealed to have suitable substitutions and corresponding thereto amino acids of the capsids: HBoV1, B19 and BPV. An asterisk indicates amino acids suitable for substitution in accordance with the increased number criterion for polar contacts between capsomeres.**

| **AAV5** | **B19** | **HBoV1** | **BPV** |
|---|---|---|---|
| 431V | Y* | V | V |
| 616A | D* | K | R |
| 683W | R* | W | W |

### Example 4. Design of mutations based on the principle of decreasing the number of polar contacts between capsomeres

In accordance with Example 1, after aligning structurally the AAV5 capsid and the template capsid, we get a pairwise structural correspondence between amino acid residues thereof. In one of the cases, the decision to substitute the initial amino acid for the corresponding amino acid of the template capsid is made based on the analysis of the microenvironment for both the initial amino acid and a structural analogue thereof. In particular, a positive decision to substitute is made if in a pair of corresponding amino acids in the AAV5 capsid there is present a polar amino acid residue that forms at least a single polar contact with an adjacent pentameric subunit, whereas in the template capsid there is disposed a non-polar amino acid residue. Further, by analogy with Example 2, cysteine or methionine residues are not considered for substitution.

Table 5 shows the results of applying the subject method for designing mutations in the AAV5 capsid using three template capsids: HBoV1 (PDBid 5urf), B19 (PDBid 1s58), BPV (4qc8). The first column contains amino acids from the interaction interface of adjacent pentameric subunits of the AAV5 capsid, for which suitable substitutions were found in one of the three capsids: HBoV1, B19 and BPV. The remaining columns contain amino acids produced after pairwise structural alignment of AAV5 to each of the template capsids. An asterisk indicates amino acids suitable for substitution in accordance with the increased number criterion for the polar contacts and the prohibited substitution for cysteine and methionine.

**Table 5. AAV5 capsomere positions that were revealed to have suitable substitutions and corresponding thereto amino acids of the capsids: HBoV1, B19 and BPV. An asterisk indicates amino acids suitable for substitution in accordance with the decreased number criterion for polar contacts between capsomeres.**

| **AAV5** | **B19** | **HBoV1** | **BPV** |
|---|---|---|---|
| S222 | A* | S | T |
| R285 | L* | Q | N |
| Q340 | K | A* | R |
| S420 | Q | Y | F* |
| K425 | E | L* | M |
| N679 | K | A* | G |
| S680 | A* | T | T |
| T721 | V* | N | H |

### Example 5.

### Selection of mutation variants in AAV9 capsid based on structural similarity of capsids of the family Parvoviridae

The design of AAV9 capsid mutations is based on a high degree of structural similarity of capsid proteins within the family Parvoviridae, including non-AAV viruses. The selection of a substitution consists of three steps of:
1) determining amino acids in AAV9 capsomere protein, which are located in the interaction interface region of adjacent pentameric subunits of the capsid;
2) aligning structurally the modified AAV9 capsid to a capsid of one of the non-AAV parvoviruses (hereinafter referred to as "template capsid");
3) comparing pairwisely amino acid residues of the AAV9 capsid and those of the template capsid.

The structures of the three-dimensional models of AAV9 and the template capsid are pre-prepared; the pre-preparation consists in completing the missing atoms in the structure using the Prepwizard utility from Schrodinger Suite.

In step 1, the interaction interface refers to a set of capsomere protein polypeptide chain fragments interacting with the amino acids of an adjacent pentameric subunit, wherein the pentameric subunit refers to a capsid structural element having the shape of a regular pentagon and consisting of 5 capsomere proteins located around the central pore (L. M. Drouin and M. Agbandje-McKenna. Adeno-associated virus structural biology as a tool in vector development. Future Virol., vol. 8, no. 12, pp. 1183-1199, 2013.).

The second step comprises aligning structurally the AAV9 capsid to the template capsid using the Structure Alignment utility from Schrodinger Suite. Following the results of alignment, for each AAV9 amino acid from the interaction interface of adjacent pentameric subunits, the closest thereto amino acid of the template capsid (structural analogue of the initial amino acid) is determined, which is considered a potential substitution. There is no structural analogue of the initial amino acid if in the vicinity of the nearest thereto amino acid of the template capsid there is present another AAV9 amino acid, the distance to which is less than that to the initial amino acid. Here, the distance between amino acids is estimated by that between alpha-carbon atoms.

After structural analogues have been determined for all amino acids of the interaction interface of adjacent pentameric subunits, the decision to substitute is made in accordance with Examples 6, 7 and 8.

### Example 6. Design of mutations based on the principle of increasing the volume of amino acid residue

After aligning structurally the AAV9 capsid to the template capsid, we get a pairwise structural correspondence between amino acid residues thereof. In one of the cases, the decision to substitute the initial amino acid for the corresponding amino acid of the template capsid is made following the results of comparing the van der Waals volumes of the amino acid side substituents (N. J. Darby, S. S. N. J. Darby, and T. E. Creighton, Protein Structure. IRL Press at Oxford University Press, 1993; Table 1). A mutation is introduced if the following conditions are met:
1. Volume of the amino acid side substituent from the template capsid is greater than that of the initial amino acid side substituent
2. An amino acid from the template capsid is not cysteine or methionine.

The exclusion of cysteine and methionine from the amino acids for potential substitution is due to their significant reactivity, in particular oxidizability.

Table 6 shows the results of applying the subject method for designing mutations in the AAV9 capsid using three template capsids: HBoV1 (PDBid 5urf), B19 (PDBid 1s58), BPV (PDBid 4qc8). The first column contains amino acids from the interaction interface of adjacent pentameric subunits of the AAV9 capsid, the remaining columns contain amino acids produced after pairwise structural alignment of AAV9 to each one of the capsids: HBoV1, B19 and BPV. An asterisk indicates amino acids suitable for substitution in accordance with the size criterion for the side substituent and the prohibited substitution for cysteine and methionine.

Interaction interface for a given subunit is intended to refer to all amino acids, at least one atom of which is present at distance lower than 5 Å from any atom of adj acent pentameric subunits.

For AAV9, the calculation was made based on the structure PDB ID: 3UX1.

According to the calculation, its interaction interface between adjacent pentameric subunits of AAV9 includes the following ranges of AAV9 capsid protein VP1 residues: 230 - 236, 294 - 304, 350 - 353, 394 - 401, 421 - 444, 476 - 480, 601 - 609, 622 - 637, 689 - 697, 733 - 736.

**Table 6. Amino acids from the interaction interface of adjacent pentameric subunits of the AAV9 capsid and structurally corresponding thereto amino acids of the capsomeres B19, HBoV1 and BPV. An asterisk indicates amino acids whose side substituent is greater than that of the initial amino acid of the AAV9 capsomere. A dash indicates the fact that there is no structurally similar amino acid in the template capsid.**

| **AAV9** | **B19** | **HBoV1** | **BPV** |
|---|---|---|---|
| C230 | E* | G | G |
| D231 | G | G | G |
| S232 | A | T* | S |
| Q233 | T | I* | H* |
| W234 | F | F | F |
| L235 | S | S | S |
| G236 | A* | E* | D* |
| S294 | S | S | S |
| P295 | P | P | P |
| R296 | L | N | Q |
| D297 | E* | D | D |
| W298 | F | W | W |
| Q299 | Q | Q | Q |
| R300 | H | H | R |
| L301 | L | L | L |
| I302 | I | V | T |
| N303 | E* | N | N |
| N304 | N | D* | E* |
| Y350 | Y | H | H |
| Q351 | K* | R* | A |
| L352 | Y* | Y* | Y* |
| P353 | P | P | P |
| F394 | F | L | F |
| Y395 | Y | Y | F |
| C396 | V* | M | L* |
| L397 | L | L | L |
| E398 | E | E | E |
| Y399 | H | N | N |
| F400 | S | S | S |
| P401 | S | D* | D* |
| P421 | P | E* | E* |
| F422 | P | W* | W* |
| H423 | E | I* | V |
| S424 | N* | E* | N* |
| S425 | L* | N* | N* |
| Y426 | E | N | E |
| A427 | G | I* | R* |
| H428 | C | T | A |
| S429 | Q* | F* | Y* |
| Q430 | H* | S | I* |
| S431 | F* | M | P* |
| L432 | - | P | P |
| D433 | Y* | Q* | G |
| R434 | E | M | L |
| L435 | M | M | M |
| M436 | Y* | Y* | F* |
| N437 | N | N | N |
| P438 | P | P | P |
| L439 | Y* | L | K* |
| I440 | G | V | V |
| D441 | S | R* | P |
| Q442 | R* | S | T |
| Y443 | - | R* | R* |
| L444 | - | R* | R* |
| R476 | Q | S | T |
| N477 | N | N | S |
| Y478 | F | W* | W* |
| I479 | M | M | M |
| P480 | P | S | T* |
| I601 | L | M | M |
| L602 | M | L | F* |
| P603 | V* | P | P |
| G604 | G | N* | N* |
| M605 | S | Q | Q |
| V606 | V | M | V |
| W607 | W | w | w |
| Q608 | N | D | D |
| D609 | R* | S | R* |
| I622 | I | V | K* |
| P623 | P | P | P |
| H624 | N | R* | R* |
| T625 | L* | V* | A |
| D626 | D | N | D |
| G627 | D* | R* | K* |
| N628 | S | K* | H* |
| F629 | F | T | T |
| H630 | K* | L* | I* |
| P631 | T* | L* | M |
| S632 | Q* | D* | D* |
| P633 | A | T* | - |
| L634 | A | Q | P |
| M635 | L | D | D |
| G636 | G | G | G |
| G637 | G | S* | S* |
| K689 | P | K | R* |
| E690 | R* | R* | Y* |
| N691 | K* | G | A |
| S692 | A | T* | T* |
| K693 | T | K | K |
| R694 | G | N | N |
| W695 | R | W | W |
| N696 | N | R* | R* |
| P697 | P | P | P |
| T733 | V* | H* | N |
| R734 | H | K | K |
| N735 | L* | V* | V* |
| L736 | - | L | L |

### Example 7. Design of mutations based on the principle of increasing the number of polar contacts between capsomeres.

In accordance with Example 5, following the results of aligning structurally the AAV9 capsid to the template capsid, we get a pairwise structural correspondence between amino acid residues thereof. In one of the cases, the decision to substitute the initial amino acid for the corresponding amino acid of the template capsid is made based on the analysis of the microenvironment for both the initial amino acid and a structural analogue thereof. In particular, a positive decision to substitute is made if in a pair of corresponding amino acids in the template capsid there is disposed an amino acid with a polar side substituent, and in AAV9 there is disposed one with a non-polar substituent, wherein the both amino acids have at least one polar contact with an adjacent pentameric subunit of the capsid. An amino acid is assumed to have a polar contact if at a distance of no more than 5 Å from any atom of the side substituent other than hydrogen there is disposed at least one atom of the polar group of the side substituent of another amino acid of another capsomere (Table 3). By analogy with Example 6, substitutions for cysteine and methionine are also not allowed.

Table 7 shows the results of applying the subject method for designing mutations in the AAV9 capsid using three template capsids: HBoV1 (PDBid 5urf), B19 (PDBid 1s58), BPV (4qc8). The first column contains amino acids from the interaction interface of adjacent pentameric subunits of the AAV9 capsid, for which suitable substitutions were found in one of the three capsids: HBoV1, B19 and BPV. The remaining columns contain amino acids produced after pairwise structural alignment of AAV9 to each of the capsids. An asterisk indicates amino acids suitable for substitution in accordance with the increased number criterion for the polar contacts and the prohibited substitution for cysteine and methionine.

**Table 7. AAV9 capsomere positions that were revealed to have suitable substitutions and corresponding thereto amino acids of the capsids: HBoV1, B19 and BPV. An asterisk indicates amino acids suitable for substitution in accordance with the increased number criterion for polar contacts between capsomeres.**

| **AAV9** | **B19** | **HBoV1** | **BPV** |
|---|---|---|---|
| A427 | G | R* | I |
| L439 | Y* | K* | L |
| M635 | L | D* | D |
| W695 | R* | W | W |

### Example 8. Design of mutations based on the principle of decreasing the number of polar contacts between capsomeres

In accordance with Example 5, following the results of aligning structurally the AAV9 capsid to the template capsid, we get a pairwise structural correspondence between amino acid residues thereof. In one of the cases, the decision to substitute the initial amino acid for the corresponding amino acid of the template capsid is made based on the analysis of the microenvironment for both the initial amino acid and a structural analogue thereof. In particular, a positive decision to substitute is made if in a pair of corresponding amino acids in the AAV9 capsid there is present a polar amino acid residue that forms at least a single polar contact with an adjacent pentameric subunit, whereas in the template capsid there is disposed a non-polar amino acid residue. Further, by analogy with Example 6, cysteine or methionine residues are not considered for substitution.

Table 8 shows the results of applying the subject method for designing mutations in the AAV9 capsid using three template capsids: HBoV1 (PDBid 5urf), B19 (PDBid 1s58), BPV (4qc8). The first column contains amino acids from the interaction interface of adjacent pentameric subunits of the AAV9 capsid, for which suitable substitutions were found in one of the three capsids: HBoV1, B19 and BPV. The remaining columns contain amino acids produced following the results of pairwise structural alignment of AAV9 to each of the template capsids. An asterisk indicates amino acids suitable for substitution in accordance with the increased number criterion for the polar contacts and the prohibited substitution for cysteine and methionine.

**Table 8. AAV9 capsomere positions that were revealed to have suitable substitutions and corresponding thereto amino acids of the capsids: HBoV1, B19 and BPV. An asterisk indicates amino acids suitable for substitution in accordance with the decreased number criterion for polar contacts between capsomeres.**

| **AAV9** | **B19** | **HBoV1** | **BPV** |
|---|---|---|---|
| R296 | L* | Q | N |
| Q351 | K | A* | R |
| Y395 | Y | F* | Y |
| S429 | Q | Y | F* |
| S431 | F* | P | M |
| R434 | E | L* | M |
| T625 | L* | A* | V* |
| N691 | K | A* | G |
| S692 | A* | T | T |
| T733 | V* | N | H |
| N735 | L* | V* | V* |

### Example 9. Production of libraries of AAV5 capsid variants

Libraries of AAV5 capsid variants were produced by random mutagenesis of the Cap gene sequence (Davidsson M. et al., 2016). Briefly, the wild-type sequence of the Cap gene serotype 5 (GenBank ID AF085716.1) was assembled *de novo,* the synthesized wild-type AAV5 capsid gene was thereafter fragmented using uracil-DNA glycosylase, the resulting fragments were assembled to a full-length Cap gene using DNA polymerase not having a proofreading activity (resulting in random mutations in the sequence). The full-length mutant variants were cloned into the carrier plasmid pAAV-linker (Fig. 1) at AscI/EcoRI restriction sites in a common reading frame with green fluorescent protein (GFP), thereby producing a diverse random library of AAV5 capsids, which library was thereafter used to select capsid variants having increased transduction activity.

Viral particles having increased transducing activity were positively selected in vitro on CHO-K1-S cells. Further, for transduction, we used particles that were purified using ultracentrifugation in a iodixanol gradient. After 48 hours, the cells were harvested and genomic DNA was isolated for subsequent amplification of the viral genome sequences capable of efficient transduction. The resulting sequences were thereafter re-cloned and re-produced for subsequent selection iterations to enrich the library with variants having the highest transduction efficiency. Following 5 rounds of selection, the capsid genes of 30 clones were sequenced to determine the most successful mutations and combinations thereof. The sequencing results showed that the predominant combinations of mutations were S2A, T711S in AAV5 VP1 and capsid variants containing S2A, T711S, S651A in AAV5 VP1, which were about 20% of the clones. Capsid variants comprising the mutation S651A in AAV5 VP1 were also selected. These capsid variants were cloned into vectors for producing viral particles and further used for visualizing and comparing the transduction profiles relative to wild-type AAV5.

### Example 10. Generation followed by selection of recombinant viral particles from resulting sequence library

To produce and subsequently select recombinant viral particles from the resulting sequence library, a series of plasmids was developed as follows: a carrier plasmid, a plasmid comprising the Rep gene sequence, as well as a construct comprising adenoviral genes that are required for the replication of viral particles.

The carrier plasmid pAAV-linker (Fig. 1), intended for cloning the libraries of random variants of the capsid gene of AAV serotype 5 into one reading frame with the reporter protein, was produced by substituting the sequence of a modified green fluorescent protein in the original construct pAAV-GFP, using the restrictase-ligase method of cloning at HindIII/EcoRI sites, for the sequence T2A-GFP synthesized *de novo* with the addition of an EcoRI restriction site from the 5' end and a HindIII restriction site from the 3' end.

The plasmid pAAV-Rep comprising the Rep gene sequence (Fig. 2) produced by *de novo* cloning of the synthesized Rep gene sequence of AAV serotype 2 (GenBank ID AF043303.1), with the addition of a PciI restriction site at the 5' end and a PsiI restriction site at the 3' end into the plasmid pGEM-T Easy (Promega, USA) that was also treated with PciI/PsiI restrictases (New England Biolabs, USA).

The adenoviral genes for producing the recombinant viral particles were sourced from the construct pHelper (Fig. 3) comprising AmpR - a beta-lactamase gene that provides resistance to ampicillin, Ori - a replication origin in bacteria, Adeno E2A - a helper adenovirus gene sequence involved in viral DNA replication, Adeno E4 - a helper adenovirus gene sequence involved in viral DNA replication, Adeno VARNA - a helper adenovirus gene sequence responsible for the translation of both early and late viral genes.

### Example 11. Production of mutant variants of AAV5 and AAV9 capsids

Gene sequences of wild-type AAV serotype 5 capsid (GenBank ID AF085716.1) and those of wild-type AAV serotype 9 capsid (GenBank ID AY530579.1) were synthesized *de novo* with the inclusion of SwaI site from the 5' end and NotI site from the 3' end into each one of the synthesized sequences, respectively; following treatment with Swal/Notl restriction endonucleases (New England Biolabs, USA), each of the sequences was cloned into the plasmid vector pAAV-Rep (Fig. 2) also pre-treated with Swal/Notl restrictases (New England Biolabs, USA), resulting in the following plasmids: pAAV-RC5 (Fig. 4) comprising the sequence of wild-type AAV serotype 5 capsid and further comprising the AAV Rep gene sequence, and pAAV-RC9 (Fig. 5) comprising the sequence of wild-type AAV serotype 9 capsid and further comprising the AAV Rep gene sequence.

Mutations were introduced into the sequence of wild-type AAV capsid using a commercial QuikChange II Site-directed mutagenesis kit (Agilent, USA) according to the manufacturer's instructions. PCR primers were developed using the QuikChange Primer Design software (Agilent, USA) (Table 9, Table 10). Briefly, the procedure based on site-directed mutagenesis was performed using the plasmids pAAV-RC5 or pAAV-RC9 as a template, respectively. To introduce mutations, the first step included a PCR reaction in individual test tubes for each mutant and control sample supplemented with corresponding oligonucleotides. The second step included treatment with restrictase according to the manufacturer's protocol to remove the template plasmid DNA, the competent *E.coli XL-1Blue* cells were transformed with the resulting mixture. The resulting clones were analyzed using PCR for specific regions in the target plasmid, the selected mutants were subject to sequencing before use.

**Table 9. Nucleotide sequences of primers used for site-directed mutagenesis of the gene sequence of AAV capsid serotype 5.**

| Substit ution | Forward sequence | Reverse sequence |
|---|---|---|
| G226A | cgattccacgtggatg**gcc**gacagagtcgtcacc | ggtgacgactctgtcg**gcc**atccacgtggaatcg |
| D286E | ccactggagcccccgagagtggcaaagactcatcaa c | gttgatgagtctttgc**cac**tctcgggggctccagtgg |
| L341Y | ggacgacgactaccag**tac**ccctacgtcgtcggc | gccgacgacgtaggg**gta**ctggtagtcgtcgtcc |
| C387V | cgagaggagcagcttcttcgtcctagagtactttcccag c | gctgggaaagtactctaggacgaagaagctgctcctctc g |
| Q421H | cagcttcgctcccagt**cac**aacctcttcaagctgg | ccagcttgaagaggtt**gtg**actgggagcgaagctg |
| P466T | ctacaaaaactggttc**acc**gggcccatgggccgaac | gttcggcccatgggccc**ggt**gaaccagtttttgtag |
| S594Q | caggaaatcgtgcccggccaggtgtggatggagagg gac | gtccctctccatccacac**ctg**gccgggcacgatttcctg |
| T614L | gccaagatcccagag**ctc**ggggcgcactttcac | gtgaaagtgcgcccc**gag**ctctgggatcttggc |
| N679K | gagatggagtgggagctcaagaaggaaaagtccaag aggtggaacc | ggttccacctcttggacttttccttcttgagctcccactcca tctc |
| P723V | ctatcggaacccgataccttacccgagtgctttaaccca ttcatg | catgaatgggttaaagcactcgggtaaggtatcgggttc cgatag |
| V431Y | gctggccaacccgctg**tac**gaccagtacttgtacc | ggtacaagtactggtc**gta**cagcgggttggccagc |
| A616D | gatcccagagacgggg**gac**cactttcacccctctc | gagaggggtgaaagtg**gtc**ccccgtctctgggatc |
| W683R | gaaaactccaagagg**aga**aacccagagatccagtac | gtactggatctctgggtt**tct**cctcttggagttttc |
| S222A | gagattggcattgcgat**gcc**acgtggatgggggacag | ctgtcccccatccacgt**ggc**atcgcaatgccaatctc |
| R285L | cagccactggagcccc**ctg**gactggcaaagactcatc | gatgagtctttgccagtc**cag**ggggctccagtggctg |
| Q340A | cggacgacgactac**gcc**ctgccctacgtcgtcg | cgacgacgtagggcag**ggc**gtagtcgtcgtccg |
| S420F | ctccagcttcgctccc**ttc**cagaacctcttcaagc | gcttgaagaggttctg**gaa**gggagcgaagctggag |
| S680A | gctcaagaaggaaaac**gcc**aagaggtggaacccag | ctgggttccacctctt**ggc**gttttccttcttgagc |
| T721V | ggaacccgatacctt**gtg**cgacccctttaacccattc | gaatgggttaaaggggtcg**cac**aaggtatcgggttcc |

Codons that bear a substitution are highlighted in bold.

In a similar fashion, mutations were introduced into the leader variant of AAV serotype 5 capsid simultaneously comprising the mutations S2A and T711S, produced by targeted evolution described in Examples 9 and 10.

**Table 10. Nucleotide sequences of primers used for site-directed mutagenesis of the gene sequence of AAV capsid serotype 9.**

| Substitution | Forwaid sequence | Reverse sequence |
|---|---|---|
| Q351K | cttcacggactcagactataagctcccgtacgtgct c | gagcacgtacgggag**ctt**atagtctgagtccgtgaag |
| Q351R | cttcacggactcagactatagactcccgtacgtgct c | gagcacgtacgggag**tct**atagtctgagtccgtgaag |
| S232T | gaaattggcattgcgatacccaatggctgggggac ag | ctgtcccccagccattg**ggt**atcgcaatgccaatttc |
| D297E | ccacttctcaccacgtgagtggcagcgactcatca ac | gttgatgagtcgctgcca**ctc**acgtggtgagaagtgg |
| S692T | gctgcagaaggaaaacactaagcgctggaaccc g | cgggttccagcgctt**agt**gttttccttctgcagc |
| D433Y | ctcacagccaaagcctgtatcgactaatgaatcca c | gtggattcattagtcg**ata**caggctttggctgtgag |
| T625L | gccaaaattcctcac**ctg**gacggcaactttcacc | ggtgaaagttgccgtc**cag**gtgaggaattttggc |
| T625V | ggccaaaattcctcac**gtt**gacggcaactttcac | gtgaaagttgccgtc**aac**gtgaggaattttggcc |
| N691A | gagctgcagaaggaagccagcaagcgctggaac c | ggttccagcgcttgct**ggc**ttccttctgcagctc |
| C396V | gtcgttcgtccttttac**gtg**ctggaatatttcccgtc | gacgggaaatattccag**cac**gtaaaaggacgaacgac |
| Y478F | ctgtccagggaagaaac**ttc**atacctggacccagc | gctgggtccaggtat**gaa**gtttcttccctggacag |
| R296L | ctgccacttctcacca**ctt**gactggcagcgactc | gagtcgctgccagtc**aag**tggtgagaagtggcag |
| T733V | gcaccagatacctg**gtc**cgtaatctgtaacgtac | gtacgttacagattacg**gac**caggtatctggtgc |
| A427R | ctttccatagcagctacagacacagccaaagcctg gac | gtccaggctttggctgtg**tct**gtagctgctatggaaag |
| M635D | cacccttctccgctg**gat**ggagggtttggaatg | cattccaaaccctcc**atc**cagcggagaagggtg |
| L444R | cactcatcgaccaatac**aga**tactatctctcaaag | ctttgagagatagta**tct**gtattggtcgatgagtg |
| G604N | ccaaggaatacttccg**aat**atggtttggcaggac | gtcctgccaaaccat**att**cggaagtattccttgg |
| G627K | caaaattcctcacacggacaagaactttcacccttc tc | gagaagggtgaaagtt**ctt**gtccgtgtgaggaattttg |
| W695R | ggaaaacagcaagcgcagaaacccggagatcca gtac | gtactggatctccgggtt**tct**gcgcttgctgttttcc |
| Q351A | cacggactcagactat**gct**ctcccgtacgtgctcg | cgagcacgtacgggag**agc**atagtctgagtccgtg |
| Y395F | gtgggtcgttcgtccttt**ttc**tgcctggaatatttcc | ggaaatattccaggca**gaa**aaaggacgaacgacccac |
| R434L | cagccaaagcctggacctgctaatgaatccactca tc | gatgagtggattcattag**cag**gtccaggctttggctg |

Codons that bear a substitution are highlighted in bold.

### Example 11. Efficiency of generation of vectors based on modified adeno-associated virus serotype 5 (rAAV5)

To produce rAAV particles with the modified serotype 5 capsid, HEK293 producer cells were transfected using polyethylenimine (PEI, linear, MW 25000, Polyscinces, Inc.) simultaneously with 3 plasmids as follows:
1) With a plasmid comprising adenovirus nucleotide sequences encoding proteins and RNAs required for assembly of rAAV particles (helper plasmid);
2) With a plasmid comprising the natural nucleotide sequence of the Rep gene of adeno-associated virus serotype 2, as well as the sequence of the modified Cap gene, which is selected from the group comprising: the nucleotide sequence of SEQ ID NO: 7, 13, 19, 25, 31, 37, 43, 49, 55, 61, 67, 73, 79, 85, 91, 97, 103, 109, 115, 121, 127, 133, 139, 145, 151, 157, 163, 169, 175, 181, 187, 193, 199, 205, 211, 217, 223, 229, 235 or any other nucleotide sequence encoding the protein VP1 with the amino acid sequences of SEQ ID No: 8, 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230 or 236, and the proteins VP2 and VP3 from alternative reading frames of the nucleotide sequence being used, wherein
   VP2 may have any one of the amino acid sequences of SEQ ID No: 10, 16, 22, 28, 34, 40, 46, 52, 58, 64, 70, 76, 82, 88, 94, 100, 106, 112, 118, 124, 130, 136, 142, 148, 154, 160, 166, 172, 178, 184, 190, 196, 202, 208, 214, 220, 226, 232 or 238;
   and VP3 may have any one of the amino acid sequences of SEQ ID No: 12, 18, 24, 30, 36, 42, 48, 54, 60, 66, 72, 78, 84, 90, 96, 102, 108, 114, 120, 126, 132, 138, 144, 150, 156, 162, 168, 174, 180, 186, 192, 198, 204, 210, 216, 222, 228, 234 or 240;
3) With a plasmid comprising a heterologous rAAV particle genome encoding the target gene intended for delivery into patient's cells.

This set of genes provides the assembly of rAAV viral particles and encapsidation of the target genome therein within 72 hours. 72 hours following transfection, the producing cells were subjected to lysis (TAKARA BIO INC, Japan) to release rAAV particles, the resulting vectors were treated with DNAase I (Invitrogen, USA) for 2 hours at 37 °C, followed by another 2 hours of treatment with proteinase K (Invitrogen, USA) at 56 °C. The titer of the resulting rAAV particles was verified by quantitative PCR (TaqMan, Applied Biosystems, USA) using an oligonucleotide set consisting of a forward primer 5'- ACCACATGAAGCAGCACGAC -3', a reverse primer 5'-TCAGCTCGATGCGGTTCAC -3', and a probe 5'- HEX-CATGCCCGAAGGCTACGTCCAG-BHQ1 -3' specific for the GFP sequence.

The authors of the invention surprisingly found that the presence of one or more mutations selected from the group comprising G226A, D286E, L341Y, C387V, Q421H, P466T, S594Q, T614L, N679K, P723V, V431Y, A616D, W683R, S222A, R285L, Q340A, S420F, S680A, T721V in wild-type rAAV5 capsid protein VP1 or in rAAV5 capsid protein VP1 already comprising the mutations S2A and T711S caused a statistically reliable increase in the efficiency of transgene packaging by the rAAV vectors with the above mutations. For example, quantitative PCR showed a change in copy number of the packaged heterologous rAAV particle genome encoding the target GFP gene (Fig. 6, Fig. 7).

In the simultaneous presence of the mutations S2A and T711S (AAV5-01Mut-GFP), the number of packaged viral genomes increased by 7.1 times from 2.51E+09 vg/ml to 1.78E+10 vg/ml compared to the control AAV5 with the wild-type capsid protein VP1 (AAV5-NullMut-GFP).

In the simultaneous presence of the mutations S2A, G226A and T711S (AAV5-02Mut-GFP), the number of packaged viral genomes increased by 5.4 times from 2.51E+09 vg/ml to 1.36E+10 vg/ml compared to the control AAV5 with the wild-type capsid protein VP1 (AAV5-NullMut-GFP).

In the simultaneous presence of the mutations S2A, D286E and T711S (AAV5-03Mut-GFP), the number of packaged viral genomes increased by 3.5 times from 2.51E+09 vg/ml to 8.79E+09 vg/ml compared to the control AAV5 with the wild-type capsid protein VP1 (AAV5-NullMut-GFP).

In the simultaneous presence of the mutations S2A, L341Y and T711S (AAV5-04Mut-GFP), the number of packaged viral genomes increased by 5.8 times from 2.51E+09 vg/ml to 1.46E+10 vg/ml compared to the control AAV5 with the wild-type capsid protein VP1 (AAV5-NullMut-GFP).

In the simultaneous presence of the mutations S2A, C387V and T711S (AAV5-05Mut-GFP), the number of packaged viral genomes increased by 4.8 times from 2.51E+09 vg/ml to 1.21E+10 vg/ml compared to the control AAV5 with the wild-type capsid protein VP1 (AAV5-NullMut-GFP).

In the simultaneous presence of the mutations S2A, Q421H and T711S (AAV5-06Mut-GFP), the number of packaged viral genomes increased by 2.1 times from 2.51E+09 vg/ml to 5.27E+09 vg/ml compared to the control AAV5 with the wild-type capsid protein VP1 (AAV5-NullMut-GFP).

In the simultaneous presence of the mutations S2A, P466T and T711S (AAV5-07Mut-GFP), the number of packaged viral genomes increased by 6.2 times from 2.51E+09 vg/ml to 1.56E+10 vg/ml compared to the control AAV5 with the wild-type capsid protein VP1 (AAV5-NullMut-GFP).

In the simultaneous presence of the mutations S2A, S594Q and T711S (AAV5-08Mut-GFP), the number of packaged viral genomes increased by 5.9 times from 2.51E+09 vg/ml to 1.48E+10 vg/ml compared to the control AAV5 with the wild-type capsid protein VP1 (AAV5-NullMut-GFP).

In the simultaneous presence of the mutations S2A, T614L and T711S (AAV5-09Mut-GFP), the number of packaged viral genomes increased by 6.8 times from 2.51E+09 vg/ml to 1.71E+10 vg/ml compared to the control AAV5 with the wild-type capsid protein VP1 (AAV5-NullMut-GFP).

In the simultaneous presence of the mutations S2A, N679K and T711S (AAV5-10Mut-GFP), the number of packaged viral genomes increased by 3.3 times from 2.51E+09 vg/ml to 8.28E+09 vg/ml compared to the control AAV5 with the wild-type capsid protein VP1 (AAV5-NullMut-GFP).

In the simultaneous presence of the mutations S2A, P723V and T711S (AAV5-11Mut-GFP), the number of packaged viral genomes increased by 5.0 times from 2.51E+09 vg/ml to 1.26E+10 vg/ml compared to the control AAV5 with the wild-type capsid protein VP1 (AAV5-NullMut-GFP).

In the simultaneous presence of the mutations S2A, V431Y and T711S (AAV5-12Mut-GFP), the number of packaged viral genomes increased by 2.4 times from 2.51E+09 vg/ml to 6.03E+09 vg/ml compared to the control AAV5 with the wild-type capsid protein VP1 (AAV5-NullMut-GFP).

In the simultaneous presence of the mutations S2A, A616D and T711S (AAV5-13Mut-GFP), the number of packaged viral genomes increased by 3.6 times from 2.51E+09 vg/ml to 9.04E+09 vg/ml compared to the control AAV5 with the wild-type capsid protein VP1 (AAV5-NullMut-GFP).

In the simultaneous presence of the mutations S2A, W683R and T711S (AAV5-14Mut-GFP), the number of packaged viral genomes increased by 4.6 times from 2.51E+09 vg/ml to 1.15E+10 vg/ml compared to the control AAV5 with the wild-type capsid protein VP1 (AAV5-NullMut-GFP).

In the simultaneous presence of the mutations S2A, S222A and T711S (AAV5-15Mut-GFP), the number of packaged viral genomes increased by 1.5 times from 2.51E+09 vg/ml to 3.77E+09 vg/ml compared to the control AAV5 with the wild-type capsid protein VP1 (AAV5-NullMut-GFP).

In the simultaneous presence of the mutations S2A, R285L and T711S (AAV5-16Mut-GFP), the number of packaged viral genomes increased by 2.5 times from 2.51E+09 vg/ml to 6.28E+09 vg/ml compared to the control AAV5 with the wild-type capsid protein VP1 (AAV5-NullMut-GFP).

In the simultaneous presence of the mutations S2A, Q340A and T711S (AAV5-17Mut-GFP), the number of packaged viral genomes increased by 1.8 times from 2.51E+09 vg/ml to 4.52E+09 vg/ml compared to the control AAV5 with the wild-type capsid protein VP1 (AAV5-NullMut-GFP).

In the simultaneous presence of the mutations S2A, S420F and T711S (AAV5-18Mut-GFP), the number of packaged viral genomes increased by 4.4 times from 2.51E+09 vg/ml to 1. 10E+10 vg/ml compared to the control AAV5 with the wild-type capsid protein VP1 (AAV5-NullMut-GFP).

In the simultaneous presence of the mutations S2A, S680A and T711S (AAV5-19Mut-GFP), the number of packaged viral genomes increased by 3.0 times from 2.51E+09 vg/ml to 7.53E+09 vg/ml compared to the control AAV5 with the wild-type capsid protein VP1 (AAV5-NullMut-GFP).

In the simultaneous presence of the mutations S2A, T721V and T711S (AAV5-20Mut-GFP), the number of packaged viral genomes increased by 5.2 times from 2.51E+09 vg/ml to 1.31E+10 vg/ml compared to the control AAV5 with the wild-type capsid protein VP1 (AAV5-NullMut-GFP).

In the presence of the single mutation G226A (AAV5-21Mut-GFP), the amount of packaged viral genomes increased by 3,9 times from 2,74E+09 vg/ml to 1,07E+10 vg/ml as compared to control AAV5 with wild-type capsid protein VP1 (AAV5-NullMut-GFP).

In the presence of the single mutation D286E (AAV5-22Mut-GFP), the amount of packaged viral genomes increased by 2,4 times from 2,74E+09 vg/ml to 6,58E+09 vg/ml as compared to control AAV5 with wild-type capsid protein VP1 (AAV5-NullMut-GFP).

In the presence of the single mutation L341Y (AAV5-23Mut-GFP), the amount of packaged viral genomes increased by 2,7 times from 2,74E+09 vg/ml to 7,40E+09 vg/ml as compared to control AAV5 with wild-type capsid protein VP1 (AAV5-NullMut-GFP).

In the presence of the single mutation C387V (AAV5-24Mut-GFP), the amount of packaged viral genomes increased by 4,8 times from 2,74E+09 vg/ml to 1,32E+10 vg/ml as compared to control AAV5 with wild-type capsid protein VP1 (AAV5-NullMut-GFP).

In the presence of the single mutation Q421H (AAV5-25Mut-GFP), the amount of packaged viral genomes increased by 3,2 times from 2,74E+09 vg/ml to 8,77E+09 vg/ml as compared to control AAV5 with wild-type capsid protein VP1 (AAV5-NullMut-GFP).

In the presence of the single mutation P466T (AAV5-26Mut-GFP), the amount of packaged viral genomes increased by 1,7 times from 2,74E+09 vg/ml to 4,66E+09 vg/ml as compared to control AAV5 with wild-type capsid protein VP1 (AAV5-NullMut-GFP).

In the presence of the single mutation S594Q (AAV5-27Mut-GFP), the amount of packaged viral genomes increased by 3,1 times from 2,74E+09 vg/ml to 8,50E+09 vg/ml as compared to control AAV5 with wild-type capsid protein VP1 (AAV5-NullMut-GFP).

In the presence of the single mutation T614L (AAV5-28Mut-GFP), the amount of packaged viral genomes increased by 1,5 times from 2,74E+09 vg/ml to 4,11E+09 vg/ml as compared to control AAV5 with wild-type capsid protein VP1 (AAV5-NullMut-GFP).

In the presence of the single mutation N679K (AAV5-29Mut-GFP), the amount of packaged viral genomes increased by 2,5 times from 2,74E+09 vg/ml to 6,85E+09 vg/ml as compared to control AAV5 with wild-type capsid protein VP1 (AAV5-NullMut-GFP).

In the presence of the single mutation P723V (AAV5-30Mut-GFP), the amount of packaged viral genomes increased by 3,2 times from 2,74E+09 vg/ml to 8,77E+09 vg/ml as compared to control AAV5 with wild-type capsid protein VP1 (AAV5-NullMut-GFP).

In the presence of the single mutation V431Y (AAV5-31Mut-GFP), the amount of packaged viral genomes increased by 2,4 times from 2,74E+09 vg/ml to 6,58E+09 vg/ml as compared to control AAV5 with wild-type capsid protein VP1 (AAV5-NullMut-GFP).

In the presence of the single mutation A616D (AAV5-32Mut-GFP), the amount of packaged viral genomes increased by 1,6 times from 2,74E+09 vg/ml to 4,39E+09 vg/ml as compared to control AAV5 with wild-type capsid protein VP1 (AAV5-NullMut-GFP).

In the presence of the single mutation W683R (AAV5-33Mut-GFP), the amount of packaged viral genomes increased by 1,3 times from 2,74E+09 vg/ml to 3,56E+09 vg/ml as compared to control AAV5 with wild-type capsid protein VP1 (AAV5-NullMut-GFP).

In the presence of the single mutation S222A (AAV5-34Mut-GFP), the amount of packaged viral genomes increased by 1,8 times from 2,74E+09 vg/ml to 4,93E+09 vg/ml as compared to control AAV5 with wild-type capsid protein VP1 (AAV5-NullMut-GFP).

In the presence of the single mutation R285L (AAV5-35Mut-GFP), the amount of packaged viral genomes increased by 2,1 times from 2,74E+09 vg/ml to 5,76E+09 vg/ml as compared to control AAV5 with wild-type capsid protein VP1 (AAV5-NullMut-GFP).

In the presence of the single mutation Q340A (AAV5-36Mut-GFP), the amount of packaged viral genomes increased by 1,4 times from 2,74E+09 vg/ml to 3,84E+09 vg/ml as compared to control AAV5 with wild-type capsid protein VP1 (AAV5-NullMut-GFP).

In the presence of the single mutation S420F (AAV5-37Mut-GFP), the amount of packaged viral genomes increased by 3,5 times from 2,74E+09 vg/ml to 9,59E+09 vg/ml as compared to control AAV5 with wild-type capsid protein VP1 (AAV5-NullMut-GFP).

In the presence of the single mutation S680A (AAV5-38Mut-GFP), the amount of packaged viral genomes increased by 2,2 times from 2,74E+09 vg/ml to 6,03E+09 vg/ml as compared to control AAV5 with wild-type capsid protein VP1 (AAV5-NullMut-GFP).

In the presence of the single mutation T721V (AAV5-39Mut-GFP), the amount of packaged viral genomes increased by 5,1 times from 2,74E+09 vg/ml to 1,40E+10 vg/ml as compared to control AAV5 with wild-type capsid protein VP1 (AAV5-NullMut-GFP).

### Example 12. Efficiency of generation of vectors based on modified adeno-associated virus serotype 9 (rAAV9)

To produce rAAV particles with the modified serotype 9 capsid, HEK293 producer cells were transfected using polyethylenimine (PEI, linear, MW 25000, Polyscinces, Inc.) simultaneously with 3 plasmids as follows:
1) With a plasmid comprising adenovirus nucleotide sequences encoding proteins and RNAs required for assembly of rAAV particles (helper plasmid);
2) With a plasmid comprising the natural nucleotide sequence of the Rep gene of adeno-associated virus serotype 2, as well as the sequence of the modified Cap gene, which is selected from the group comprising: the nucleotide sequence of SEQ ID NO: 241, 247, 253, 259, 265, 271, 277, 283, 289, 295, 301, 307, 313, 319, 325, 331, 337, 343, 349, 355, 361 or 367 or any other nucleotide sequence encoding the protein VP1 with the amino acid sequences of SEQ ID No: 242, 248, 254, 260, 266, 272, 278, 284, 290, 296, 302, 308, 314, 320, 326, 332, 338, 344, 350, 356, 362 or 368 and the proteins VP2 and VP3 from alternative reading frames of the nucleotide sequence being used, wherein
   VP2 may have any one of the amino acid sequences of SEQ ID No: 244, 250, 256, 262, 268, 274, 280, 286, 292, 298, 304, 310, 316, 322, 328, 334, 340, 346, 352, 358, 364 or 370;
   and VP3 may have any one of the amino acid sequences of SEQ ID No: 246, 252, 258, 264, 270, 276, 282, 288, 294, 300, 306, 312, 318, 324, 330, 336, 342, 348, 354, 360, 366 or 372;
3) With a plasmid comprising a heterologous rAAV particle genome encoding the target gene intended for delivery into patient's cells.

This set of genes provides the assembly of rAAV viral particles and encapsidation of the target genome therein within 72 hours. 72 hours following transfection, the producing cells were subjected to lysis (TAKARA BIO INC, Japanese) to release rAAV particles, the resulting vectors were treated with DNAase I (Invitrogen, USA) for 2 hours at 37 °C, followed by another 2 hours of treatment with proteinase K (Invitrogen, USA) at 56 °C. The titer of the resulting rAAV particles was verified by quantitative PCR (TaqMan, Applied Biosystems, USA) using an oligonucleotide set consisting of a forward primer 5'- ACCACATGAAGCAGCACGAC -3', a reverse primer 5'-TCAGCTCGATGCGGTTCAC -3', and a probe 5'- HEX-CATGCCCGAAGGCTACGTCCAG-BHQ1 -3' specific for the GFP sequence.

The authors of the invention have surprisingly found that the presence of one or more mutations selected from the group comprising S232T, D297E, Q351K, Q351R, C396V, D433Y, L444R, Y478F, G604N, G627K, T625L, T625V, S692T, T733V, A427R, M635D, W695R, R296L, Q351A, Y395F, R434L, N691A in wild-type rAAV9 capsid protein VP1 caused a significant increase in the efficiency of transgene packaging by the rAAV vectors with the above mutations. For example, quantitative PCR showed a change in copy number of the packaged heterologous rAAV particle genome encoding the target GFP gene (Fig. 8).

In the presence of the mutation S232T (AAV9-01Mut-GFP), the amount of packaged viral genomes increased by 1,9 times from 6,32E+09 vg/ml to 1,19E+10 vg/ml as compared to control AAV9 with wild-type capsid protein VP1 (AAV9-NullMut-GFP).

In the presence of the mutation D297E (AAV9-02Mut-GFP), the amount of packaged viral genomes increased by 3,2 times from 6,32E+09 vg/ml to 2,03E+10 vg/ml as compared to control AAV9 with wild-type capsid protein VP1 (AAV9-NullMut-GFP).

In the presence of the mutation Q351K (AAV9-03Mut-GFP), the amount of packaged viral genomes increased by 1,5 times from 6,32E+09 vg/ml to 9,30E+09 vg/ml as compared to control AAV9 with wild-type capsid protein VP1 (AAV9-NullMut-GFP).

In the presence of the mutation Q351R (AAV9-04Mut-GFP), the amount of packaged viral genomes increased by 2,5 times from 6,32E+09 vg/ml to 1,60E+10 vg/ml as compared to control AAV9 with wild-type capsid protein VP1 (AAV9-NullMut-GFP).

In the presence of the mutation C396V (AAV9-05Mut-GFP), the amount of packaged viral genomes increased by 1,9 times from 6,32E+09 vg/ml to 1,17E+10 vg/ml as compared to control AAV9 with wild-type capsid protein VP1 (AAV9-NullMut-GFP).

In the presence of the mutation D433Y (AAV9-06Mut-GFP), the amount of packaged viral genomes increased by 2,9 times from 6,32E+09 vg/ml to 1,85E+ 10 vg/ml as compared to control AAV9 with wild-type capsid protein VP1 (AAV9-NullMut-GFP).

In the presence of the mutation L444R (AAV9-07Mut-GFP), the amount of packaged viral genomes increased by 1,9 times from 6,32E+09 vg/ml to 1,23E+10 vg/ml as compared to control AAV9 with wild-type capsid protein VP1 (AAV9-NullMut-GFP).

In the presence of the mutation Y478F (AAV9-08Mut-GFP), the amount of packaged viral genomes increased by 5,0 times from 6,32E+09 vg/ml to 3,14E+10 vg/ml as compared to control AAV9 with wild-type capsid protein VP1 (AAV9-NullMut-GFP).

In the presence of the mutation G604N (AAV9-09Mut-GFP), the amount of packaged viral genomes increased by 2,5 times from 6,32E+09 vg/ml to 1,61E+10 vg/ml as compared to control AAV9 with wild-type capsid protein VP1 (AAV9-NullMut-GFP).

In the presence of the mutation G627K (AAV9-10Mut-GFP), the amount of packaged viral genomes increased by 1,8 times from 6,32E+09 vg/ml to 1,14E+10 vg/ml as compared to control AAV9 with wild-type capsid protein VP1 (AAV9-NullMut-GFP).

In the presence of the mutation T625L (AAV9-11Mut-GFP), the amount of packaged viral genomes increased by 4,6 times from 6,32E+09 vg/ml to 2,91E+10 vg/ml as compared to control AAV9 with wild-type capsid protein VP1 (AAV9-NullMut-GFP).

In the presence of the mutation T625V (AAV9-12Mut-GFP), the amount of packaged viral genomes increased by 5,7 times from 6,32E+09 vg/ml to 3,62E+10 vg/ml as compared to control AAV9 with wild-type capsid protein VP1 (AAV9-NullMut-GFP).

In the presence of the mutation S692T (AAV9-13Mut-GFP), the amount of packaged viral genomes increased by 2,1 times from 6,32E+09 vg/ml to 1,32E+10 vg/ml as compared to control AAV9 with wild-type capsid protein VP1 (AAV9-NullMut-GFP).

In the presence of the mutation T733V (AAV9-14Mut-GFP), the amount of packaged viral genomes increased by 3,0 times from 6,32E+09 vg/ml to 1,89E+ 10 vg/ml as compared to control AAV9 with wild-type capsid protein VP1 (AAV9-NullMut-GFP).

In the presence of the mutation A427R (AAV9-15Mut-GFP), the amount of packaged viral genomes increased by 1,6 times from 6,32E+09 vg/ml to 9,86E+09 vg/ml as compared to control AAV9 with wild-type capsid protein VP1 (AAV9-NullMut-GFP).

In the presence of the mutation M635D (AAV9-16Mut-GFP), the amount of packaged viral genomes increased by 1,9 times from 6,32E+09 vg/ml to 1,21E+10 vg/ml as compared to control AAV9 with wild-type capsid protein VP1 (AAV9-NullMut-GFP).

In the presence of the mutation W695R (AAV9-17Mut-GFP), the amount of packaged viral genomes increased by 1,5 times from 6,32E+09 vg/ml to 9,41E+09 vg/ml as compared to control AAV9 with wild-type capsid protein VP1 (AAV9-NullMut-GFP).

In the presence of the mutation R296L (AAV9-18Mut-GFP), the amount of packaged viral genomes increased by 3,0 times from 6,32E+09 vg/ml to 1,93E+10 vg/ml as compared to control AAV9 with wild-type capsid protein VP1 (AAV9-NullMut-GFP).

In the presence of the mutation Q351A (AAV9-19Mut-GFP), the amount of packaged viral genomes increased by 2,0 times from 6,32E+09 vg/ml to 1,29E+10 vg/ml as compared to control AAV9 with wild-type capsid protein VP1 (AAV9-NullMut-GFP).

In the presence of the mutation Y395F (AAV9-20Mut-GFP), the amount of packaged viral genomes increased by 4,6 times from 6,32E+09 vg/ml to 2,91E+10 vg/ml as compared to control AAV9 with wild-type capsid protein VP1 (AAV9-NullMut-GFP).

In the presence of the mutation R434L (AAV9-21Mut-GFP), the amount of packaged viral genomes increased by 4,0 times from 6,32E+09 vg/ml to 2,50E+10 vg/ml as compared to control AAV9 with wild-type capsid protein VP1 (AAV9-NullMut-GFP).

In the presence of the mutation N691A (AAV9-22Mut-GFP), the amount of packaged viral genomes increased by 4,5 times from 6,32E+09 vg/ml to 2,85E+10 vg/ml as compared to control AAV9 with wild-type capsid protein VP1 (AAV9-NullMut-GFP).

### Example 13. Increased efficiency of cell transduction with rAAV5-based products comprising point mutations in wild-type rAAV5 capsid protein VP1 or in rAAV5 capsid protein VP1 already comprising the mutations S2A and T711S.

Experimental design:
SK-Hep1 cells were plated into the wells of 12-well plates. The cells were plated into a EMEM growth medium supplemented with glutamine, 10% bovine serum. Cell seeding density was 10,000 cell/cm². During the transduction run, pre-prepared cells were transduced at MOI of 1,250 vg/cell. All samples were run in triplicates. Intact cells were used as a negative control.

Transduction efficiency was measured by intensity of the GFP reporter protein signal using a Guava EasyCyte flow cytometer and GuavaSoft software.

The authors of the invention surprisingly found that the presence of one or more mutations selected from the group comprising G226A, D286E, L341Y, C387V, Q421H, P466T, S594Q, T614L, N679K, P723V, V431Y, A616D, W683R, S222A, R285L, Q340A, S420F, S680A, T721V in wild-type rAAV5 capsid protein VP1 or in rAAV5 capsid protein VP1 already comprising the mutations S2A and T711S caused a statistically significant increase in the efficiency of transgene delivery by the rAAV vectors with the above mutations. For example, flow cytometry revealed a change in the amount of GFP positive cells 48 hours following the transduction of the SK-Hep1 line with rAAV-based products with wild-type AAV5 capsid protein VP1 or wild-type AAV5 capsid protein VP1 bearing one or more mutations selected from the group: G226A, D286E, L341Y, C387V, Q421H, P466T, S594Q, T614L, N679K, P723V, V431Y, A616D, W683R, S222A, R285L, Q340A, S420F, S680A, T721V in wild-type rAAV5 capsid protein VP1 or in rAAV5 capsid protein VP1 already comprising the mutations S2A and T711S (Fig. 9, Fig. 10).

In the simultaneous presence of the mutations S2A and T711S (AAV5-01Mut-GFP), the amount of GFP-expressing cells increased by 2.16 times from 20.11% to 43.44% compared to the control AAV5 with the wild-type capsid protein VP1 (AAV5-NullMut-GFP).

In the simultaneous presence of the mutations S2A, G226A and T711S (AAV5-02Mut-GFP), the amount of GFP-expressing cells increased by 2.02 times from 20.11% to 40.69% compared to the control AAV5 with the wild-type capsid protein VP1 (AAV5-NullMut-GFP).

In the simultaneous presence of the mutations S2A, D286E and T711S (AAV5-03Mut-GFP), the amount of GFP-expressing cells increased by 1.90 times from 20.11% to 38.17% compared to the control AAV5 with the wild-type capsid protein VP1 (AAV5-NullMut-GFP).

In the simultaneous presence of the mutations S2A, L341Y and T711S (AAV5-04Mut-GFP), the amount of GFP-expressing cells increased by 1.75 times from 20.11% to 35.27% compared to the control AAV5 with the wild-type capsid protein VP1 (AAV5-NullMut-GFP).

In the simultaneous presence of the mutations S2A, C387V and T711S (AAV5-05Mut-GFP), the amount of GFP-expressing cells increased by 2.10 times from 20.11% to 42.22% compared to the control AAV5 with the wild-type capsid protein VP1 (AAV5-NullMut-GFP).

In the simultaneous presence of the mutations S2A, Q421H and T711S (AAV5-06Mut-GFP), the amount of GFP-expressing cells increased by 1.64 times from 20.11% to 32.97% compared to the control AAV5 with the wild-type capsid protein VP1 (AAV5-NullMut-GFP).

In the simultaneous presence of the mutations S2A, P466T and T711S (AAV5-07Mut-GFP), the amount of GFP-expressing cells increased by 1.41 times from 20.11% to 28.35% compared to the control AAV5 with the wild-type capsid protein VP1 (AAV5-NullMut-GFP).

In the simultaneous presence of the mutations S2A, S594Q and T711S (AAV5-08Mut-GFP), the amount of GFP-expressing cells increased by 2.08 times from 20.11% to 41.82% compared to the control AAV5 with the wild-type capsid protein VP1 (AAV5-NullMut-GFP).

In the simultaneous presence of the mutations S2A, T614L and T711S (AAV5-09Mut-GFP), the amount of GFP-expressing cells increased by 2.36 times from 20.11% to 47.50% compared to the control AAV5 with the wild-type capsid protein VP1 (AAV5-NullMut-GFP).

In the simultaneous presence of the mutations S2A, N679K and T711S (AAV5-10Mut-GFP), the amount of GFP-expressing cells increased by 1.67 times from 20.11% to 33.55% compared to the control AAV5 with the wild-type capsid protein VP1 (AAV5-NullMut-GFP).

In the simultaneous presence of the mutations S2A, P723V and T711S (AAV5-11Mut-GFP), the amount of GFP-expressing cells increased by 1.87 times from 20.11% to 37.60% compared to the control AAV5 with the wild-type capsid protein VP1 (AAV5-NullMut-GFP).

In the simultaneous presence of the mutations S2A, V431Y and T711S (AAV5-12Mut-GFP), the amount of GFP-expressing cells increased by 1.27 times from 20.11% to 25.54% compared to the control AAV5 with the wild-type capsid protein VP1 (AAV5-NullMut-GFP).

In the simultaneous presence of the mutations S2A, A616D and T711S (AAV5-13Mut-GFP), the amount of GFP-expressing cells increased by 2.12 times from 20.11% to 42.63% compared to the control AAV5 with the wild-type capsid protein VP1 (AAV5-NullMut-GFP).

In the simultaneous presence of the mutations S2A, W683R and T711S (AAV5-14Mut-GFP), the amount of GFP-expressing cells increased by 1.26 times from 20.11% to 25.33% compared to the control AAV5 with the wild-type capsid protein VP1 (AAV5-NullMut-GFP).

In the simultaneous presence of the mutations S2A, S222A and T711S (AAV5-15Mut-GFP), the amount of GFP-expressing cells increased by 1.57 times from 20.11% to 31.57% compared to the control AAV5 with the wild-type capsid protein VP1 (AAV5-NullMut-GFP).

In the simultaneous presence of the mutations S2A, R285L and T711S (AAV5-16Mut-GFP), the amount of GFP-expressing cells increased by 1.38 times from 20.11% to 27.75% compared to the control AAV5 with the wild-type capsid protein VP1 (AAV5-NullMut-GFP).

In the simultaneous presence of the mutations S2A, Q340A and T711S (AAV5-17Mut-GFP), the amount of GFP-expressing cells increased by 1.45 times from 20.11% to 29.15% compared to the control AAV5 with the wild-type capsid protein VP1 (AAV5-NullMut-GFP).

In the simultaneous presence of the mutations S2A, S420F and T711S (AAV5-18Mut-GFP), the amount of GFP-expressing cells increased by 1.57 times from 20.11% to 31.57% compared to the control AAV5 with the wild-type capsid protein VP1 (AAV5-NullMut-GFP).

In the simultaneous presence of the mutations S2A, S680A and T711S (AAV5-19Mut-GFP), the amount of GFP-expressing cells increased by 1.68 times from 20.11% to 33.78% compared to the control AAV5 with the wild-type capsid protein VP1 (AAV5-NullMut-GFP).

In the simultaneous presence of the mutations S2A, T721V and T711S (AAV5-20Mut-GFP), the amount of GFP-expressing cells increased by 2.01 times from 20.11% to 40.41% compared to the control AAV5 with the wild-type capsid protein VP1 (AAV5-NullMut-GFP).

In the presence of the single mutation G226A (AAV5-21Mut-GFP), the amount of GFP-expressing cells increased by 1,29 times from 20,11% to 34,10% as compared to control AAV5 with wild-type capsid protein VP1 (AAV5-NullMut-GFP).

In the presence of the single mutation D286E (AAV5-22Mut-GFP), the amount of GFP-expressing cells increased by 1,27 times from 20,11% to 33,57% as compared to control AAV5 with wild-type capsid protein VP1 (AAV5-NullMut-GFP).

In the presence of the single mutation L341Y (AAV5-23Mut-GFP), the amount of GFP-expressing cells increased by 1,41 times from 20,11% to 37,08% as compared to control AAV5 with wild-type capsid protein VP1 (AAV5-NullMut-GFP).

In the presence of the single mutation C387V (AAV5-24Mut-GFP), the amount of GFP-expressing cells increased by 1,85 times from 20,11% to 48,62% as compared to control AAV5 with wild-type capsid protein VP1 (AAV5-NullMut-GFP).

In the presence of the single mutation Q421H (AAV5-25Mut-GFP), the amount of GFP-expressing cells increased by 1,52 times from 20,11% to 40,05% as compared to control AAV5 with wild-type capsid protein VP1 (AAV5-NullMut-GFP).

In the presence of the single mutation P466T (AAV5-26Mut-GFP), the amount of GFP-expressing cells increased by 1,33 times from 20,11% to 34,94% as compared to control AAV5 with wild-type capsid protein VP1 (AAV5-NullMut-GFP).

In the presence of the single mutation S594Q (AAV5-27Mut-GFP), the amount of GFP-expressing cells increased by 1,60 times from 20,11% to 42,26% as compared to control AAV5 with wild-type capsid protein VP1 (AAV5-NullMut-GFP).

In the presence of the single mutation T614L (AAV5-28Mut-GFP), the amount of GFP-expressing cells increased by 1,26 times from 20,11% to 33,16% as compared to control AAV5 with wild-type capsid protein VP1 (AAV5-NullMut-GFP).

In the presence of the single mutation N679K (AAV5-29Mut-GFP), the amount of GFP-expressing cells increased by 1,24 times from 20,11% to 32,78% as compared to control AAV5 with wild-type capsid protein VP1 (AAV5-NullMut-GFP).

In the presence of the single mutation P723V (AAV5-30Mut-GFP), the amount of GFP-expressing cells increased by 1,58 times from 20,11% to 41,67% as compared to control AAV5 with wild-type capsid protein VP1 (AAV5-NullMut-GFP).

In the presence of the single mutation V431Y (AAV5-31Mut-GFP), the amount of GFP-expressing cells increased by 1,27 times from 20,11% to 33,46% as compared to control AAV5 with wild-type capsid protein VP1 (AAV5-NullMut-GFP).

In the presence of the single mutation A616D (AAV5-32Mut-GFP), the amount of GFP-expressing cells increased by 1,46 times from 20,11% to 38,47% as compared to control AAV5 with wild-type capsid protein VP1 (AAV5-NullMut-GFP).

In the presence of the single mutation W683R (AAV5-33Mut-GFP), the amount of GFP-expressing cells increased by 1,52 times from 20,11% to 40,05% as compared to control AAV5 with wild-type capsid protein VP1 (AAV5-NullMut-GFP).

In the presence of the single mutation S222A (AAV5-34Mut-GFP), the amount of GFP-expressing cells increased by 1,34 times from 20,11% to 35,31% as compared to control AAV5 with wild-type capsid protein VP1 (AAV5-NullMut-GFP).

In the presence of the single mutation R285L (AAV5-35Mut-GFP), the amount of GFP-expressing cells increased by 1,55 times from 20,11% to 40,84% as compared to control AAV5 with wild-type capsid protein VP1 (AAV5-NullMut-GFP).

In the presence of the single mutation Q340A (AAV5-36Mut-GFP), the amount of GFP-expressing cells increased by 1,44 times from 20,11% to 37,94% as compared to control AAV5 with wild-type capsid protein VP1 (AAV5-NullMut-GFP).

In the presence of the single mutation S420F (AAV5-37Mut-GFP), the amount of GFP-expressing cells increased by 1,63 times from 20,11% to 42,95% as compared to control AAV5 with wild-type capsid protein VP1 (AAV5-NullMut-GFP).

In the presence of the single mutation S680A (AAV5-38Mut-GFP), the amount of GFP-expressing cells increased by 1,71 times from 20,11% to 45,06% as compared to control AAV5 with wild-type capsid protein VP1 (AAV5-NullMut-GFP).

In the presence of the single mutation T721V (AAV5-39Mut-GFP), the amount of GFP-expressing cells increased by 2,06 times from 20,11% to 54,40% as compared to control AAV5 with wild-type capsid protein VP1 (AAV5-NullMut-GFP).

### Example 14. Increasing the efficiency of cell transduction with rAAV9-based products comprising point mutations in wild-type rAAV9 capsid protein VP1.

Experimental design:
HeLa cells were plated into the wells of 12-well plates. The cells were plated into a EMEM growth medium supplemented with glutamine, 10% bovine serum. Cell seeding density was 10,000 cell/cm². During the transduction run, pre-prepared cells were transduced at MOI of 100,000 vg/cell. All samples were run in triplicates. Intact cells were used as a negative control.

Transduction efficiency was measured by intensity of the GFP reporter protein signal using a Guava EasyCyte flow cytometer and GuavaSoft software.

The authors of the invention have surprisingly found that the presence of one or more mutations selected from the group comprising S232T, D297E, Q351K, Q351R, C396V, D433Y, L444R, Y478F, G604N, G627K, T625L, T625V, S692T, T733V, A427R, M635D, W695R, R296L, Q351A, Y395F, R434L, N691A in wild-type rAAV9 capsid protein VP1 caused a significant increase in the efficiency of transgene delivery by the rAAV vectors with the above mutations. For example, flow cytometry revealed a change in the amount of GFP positive cells 48 hours following HeLa line transduction with rAAV-based products with wild-type AAV9 capsid protein VP1 or wild-type AAV9 capsid protein VP1 bearing one or more mutations selected from the group: S232T, D297E, Q351K, Q351R, C396V, D433Y, L444R, Y478F, G604N, G627K, T625L, T625V, S692T, T733V, A427R, M635D, W695R, R296L, Q351A, Y395F, R434L, N691A in wild-type rAAV9 capsid protein VP1 (Fig. 11).

In the presence of the mutation S232T (AAV9-01Mut-GFP), the amount of GFP-expressing cells increased by 1,21 times from 27,53% to 33,31% as compared to control AAV9 with wild-type capsid protein VP1 (AAV9-NullMut-GFP).

In the presence of the mutation D297E (AAV9-02Mut-GFP), the amount of GFP-expressing cells increased by 1,25 times from 27,53% to 34,41% as compared to control AAV9 with wild-type capsid protein VP1 (AAV9-NullMut-GFP).

In the presence of the mutation Q351K (AAV9-03Mut-GFP), the amount of GFP-expressing cells increased by 1,30 times from 27,53% to 35,87% as compared to control AAV9 with wild-type capsid protein VP1 (AAV9-NullMut-GFP).

In the presence of the mutation Q351R (AAV9-04Mut-GFP), the amount of GFP-expressing cells increased by 1,16 times from 27,53% to 31,93% as compared to control AAV9 with wild-type capsid protein VP1 (AAV9-NullMut-GFP).

In the presence of the mutation C396V (AAV9-05Mut-GFP), the amount of GFP-expressing cells increased by 1,37 times from 27,53% to 37,74% as compared to control AAV9 with wild-type capsid protein VP1 (AAV9-NullMut-GFP).

In the presence of the mutation D433Y (AAV9-06Mut-GFP), the amount of GFP-expressing cells increased by 1,10 times from 27,53% to 30,28% as compared to control AAV9 with wild-type capsid protein VP1 (AAV9-NullMut-GFP).

In the presence of the mutation L444R (AAV9-07Mut-GFP), the amount of GFP-expressing cells increased by 1,40 times from 27,53% to 38,45% as compared to control AAV9 with wild-type capsid protein VP1 (AAV9-NullMut-GFP).

In the presence of the mutation Y478F (AAV9-08Mut-GFP), the amount of GFP-expressing cells increased by 1,56 times from 27,53% to 42,95% as compared to control AAV9 with wild-type capsid protein VP1 (AAV9-NullMut-GFP).

In the presence of the mutation G604N (AAV9-09Mut-GFP), the amount of GFP-expressing cells increased by 1,25 times from 27,53% to 34,32% as compared to control AAV9 with wild-type capsid protein VP1 (AAV9-NullMut-GFP).

In the presence of the mutation G627K (AAV9-10Mut-GFP), the amount of GFP-expressing cells increased by 1,24 times from 27,53% to 34,14% as compared to control AAV9 with wild-type capsid protein VP1 (AAV9-NullMut-GFP).

In the presence of the mutation T625L (AAV9-11Mut-GFP), the amount of GFP-expressing cells increased by 1,45 times from 27,53% to 39,92% as compared to control AAV9 with wild-type capsid protein VP1 (AAV9-NullMut-GFP).

In the presence of the mutation T625V (AAV9-12Mut-GFP), the amount of GFP-expressing cells increased by 1,49 times from 27,53% to 41,02% as compared to control AAV9 with wild-type capsid protein VP1 (AAV9-NullMut-GFP).

In the presence of the mutation S692T (AAV9-13Mut-GFP), the amount of GFP-expressing cells increased by 1,38 times from 27,53% to 37,99% as compared to control AAV9 with wild-type capsid protein VP1 (AAV9-NullMut-GFP).

In the presence of the mutation T733V (AAV9-14Mut-GFP), the amount of GFP-expressing cells increased by 1,44 times from 27,53% to 39,73% as compared to control AAV9 with wild-type capsid protein VP1 (AAV9-NullMut-GFP).

In the presence of the mutation A427R (AAV9-15Mut-GFP), the amount of GFP-expressing cells increased by 1,23 times from 27,53% to 33,86% as compared to control AAV9 with wild-type capsid protein VP1 (AAV9-NullMut-GFP).

In the presence of the mutation M635D (AAV9-16Mut-GFP), the amount of GFP-expressing cells increased by 1,22 times from 27,53% to 33,59% as compared to control AAV9 with wild-type capsid protein VP1 (AAV9-NullMut-GFP).

In the presence of the mutation W695R (AAV9-17Mut-GFP), the amount of GFP-expressing cells increased by 1,47 times from 27,53% to 40,47% as compared to control AAV9 with wild-type capsid protein VP1 (AAV9-NullMut-GFP).

In the presence of the mutation R296L (AAV9-18Mut-GFP), the amount of GFP-expressing cells increased by 1,26 times from 27,53% to 34,69% as compared to control AAV9 with wild-type capsid protein VP1 (AAV9-NullMut-GFP).

In the presence of the mutation Q351A (AAV9-19Mut-GFP), the amount of GFP-expressing cells increased by 1,35 times from 27,53% to 37,17% as compared to control AAV9 with wild-type capsid protein VP1 (AAV9-NullMut-GFP).

In the presence of the mutation Y395F (AAV9-20Mut-GFP), the amount of GFP-expressing cells increased by 1,48 times from 27,53% to 40,74% as compared to control AAV9 with wild-type capsid protein VP1 (AAV9-NullMut-GFP).

In the presence of the mutation R434L (AAV9-21Mut-GFP), the amount of GFP-expressing cells increased by 1,67 times from 27,53% to 45,91% as compared to control AAV9 with wild-type capsid protein VP1 (AAV9-NullMut-GFP).

In the presence of the mutation N691A (AAV9-22Mut-GFP), the amount of GFP-expressing cells increased by 1,72 times from 27,53% to 47,47% as compared to control AAV9 with wild-type capsid protein VP1 (AAV9-NullMut-GFP).

### Example 15. Increasing the production of target protein encoded by transgene following cell transduction with rAAV5-based products comprising single mutations in wild-type rAAV5 capsid protein VP1 or in rAAV5 capsid protein VP1 already comprising the mutations S2A and T711S.

Experimental design:
SK-Hep1 cells were plated into the wells of 12-well plates. The cells were plated into a EMEM growth medium supplemented with glutamine, 10% bovine serum. Cell seeding density was 10,000 cell/cm². During the transduction run, pre-prepared cells were transduced at MOI of 10,000 vg/cell. All samples were run in triplicates. Intact cells were used as a negative control.

The amount of FIX protein in the culture liquid 7 days post transduction was assessed using the Human Factor IX ELISA Kit. We used a 1:50 dilution of the samples. The procedure was carried out according to the manufacturer's instructions.

The authors of the invention surprisingly found that the presence of one or more mutations selected from the group comprising G226A, D286E, L341Y, C387V, Q421H, P466T, S594Q, T614L, N679K, P723V, V431Y, A616D, W683R, S222A, R285L, Q340A, S420F, S680A, T721V in wild-type rAAV5 capsid protein VP1 or in rAAV5 capsid protein VP1 already comprising the mutations S2A and T711S caused significantly increased production of FIX protein following transduction of SK-Hep1 cells with rAAV5 vectors with the above mutations. For example, enzyme-linked immunoassay (EIA) revealed increased amount of FIX protein in the culture medium 7 days following the transduction of the SK-Hep1 cells with rAAV-based products with wild-type AAV5 capsid protein VP1 or wild-type AAV5 capsid protein VP1 bearing one or more mutations selected from the group: G226A, D286E, L341Y, C387V, Q421H, P466T, S594Q, T614L, N679K, P723V, V431Y, A616D, W683R, S222A, R285L, Q340A, S420F, S680A, T721V in wild-type rAAV5 capsid protein VP1 or in rAAV5 capsid protein VP1 already comprising the mutations S2A and T711S (Fig. 12, Fig. 13).

In the simultaneous presence of the mutations S2A, T711S (AAV5-01Mut-FIX), the amount of protein produced increased by 2.76 times from 628.67 ng/ml to 1736.67 ng/ml compared to the control AAV5 with the wild-type capsid protein VP1 (AAV5-NullMut- FIX).

In the simultaneous presence of the mutations S2A, G226A and T711S (AAV5-02Mut-FIX), the amount of protein produced increased by 1.85 times from 628.67 ng/ml to 1161.83 ng/ml compared to the control AAV5 with the wild-type capsid protein VP1 (AAV5-NullMut- FIX).

In the simultaneous presence of the mutations S2A, D286E and T711S (AAV5-03Mut-FIX), the amount of protein produced increased by 1.71 times from 628.67 ng/ml to 1075.02 ng/ml compared to the control AAV5 with the wild-type capsid protein VP1 (AAV5-NullMut- FIX).

In the simultaneous presence of the mutations S2A, L341Y and T711S (AAV5-04Mut-FIX), the amount of protein produced increased by 1.65 times from 628.67 ng/ml to 1037.30 ng/ml compared to the control AAV5 with the wild-type capsid protein VP1 (AAV5-NullMut- FIX).

In the simultaneous presence of the mutations S2A, C387V and T711S (AAV5-05Mut-FIX), the amount of protein produced increased by 2.28 times from 628.67 ng/ml to 1433.36 ng/ml compared to the control AAV5 with the wild-type capsid protein VP1 (AAV5-NullMut- FIX).

In the simultaneous presence of the mutations S2A, Q421H and T711S (AAV5-06Mut-FIX), the amount of protein produced increased by 1.67 times from 628.67 ng/ml to 1049.87 ng/ml compared to the control AAV5 with the wild-type capsid protein VP1 (AAV5-NullMut- FIX).

In the simultaneous presence of the mutations S2A, P466T and T711S (AAV5-07Mut-FIX), the amount of protein produced increased by 1.21 times from 628.67 ng/ml to 763.33 ng/ml compared to the control AAV5 with the wild-type capsid protein VP1 (AAV5-NullMut- FIX).

In the simultaneous presence of the mutations S2A, S594Q and T711S (AAV5-08Mut-FIX), the amount of protein produced increased by 1.26 times from 628.67 ng/ml to 792.12 ng/ml compared to the control AAV5 with the wild-type capsid protein VP1 (AAV5-NullMut- FIX).

In the simultaneous presence of the mutations S2A, T614L and T711S (AAV5-09Mut-FIX), the amount of protein produced increased by 2.17 times from 628.67 ng/ml to 1362.67 ng/ml compared to the control AAV5 with the wild-type capsid protein VP1 (AAV5-NullMut- FIX).

In the simultaneous presence of the mutations S2A, N679K and T711S (AAV5-10Mut-FIX), the amount of protein produced increased by 1.98 times from 628.67 ng/ml to 1244.76 ng/ml compared to the control AAV5 with the wild-type capsid protein VP1 (AAV5-NullMut- FIX).

In the simultaneous presence of the mutations S2A, P723V and T711S (AAV5-11mut-FIX), the amount of protein produced increased by 1.69 times from 628.67 ng/ml to 1062.45 ng/ml compared to the control AAV5 with the wild-type capsid protein VP1 (AAV5-NullMut- FIX).

In the simultaneous presence of the mutations S2A, V431Y and T711S (AAV5-12Mut-FIX), the amount of protein produced increased by 1.12 times from 628.67 ng/ml to 704.11 ng/ml compared to the control AAV5 with the wild-type capsid protein VP1 (AAV5-NullMut- FIX).

In the simultaneous presence of the mutations S2A, A616D and T711S (AAV5-13Mut-FIX), the amount of protein produced increased by 2.15 times from 628.67 ng/ml to 1351.63 ng/ml compared to the control AAV5 with the wild-type capsid protein VP1 (AAV5-NullMut- FIX).

In the simultaneous presence of the mutations S2A, W683R and T711S (AAV5-14Mut-FIX), the amount of protein produced increased by 1.08 times from 628.67 ng/ml to 678.96 ng/ml compared to the control AAV5 with the wild-type capsid protein VP1 (AAV5-NullMut- FIX).

In the simultaneous presence of the mutations S2A, S222A and T711S (AAV5-15Mut-FIX), the amount of protein produced increased by 1.30 times from 628.67 ng/ml to 817.27 ng/ml compared to the control AAV5 with the wild-type capsid protein VP1 (AAV5-NullMut- FIX).

In the simultaneous presence of the mutations S2A, R285L and T711S (AAV5-16Mut-FIX), the amount of protein produced increased by 1.16 times from 628.67 ng/ml to 729.25 ng/ml compared to the control AAV5 with the wild-type capsid protein VP1 (AAV5-NullMut- FIX).

In the simultaneous presence of the mutations S2A, Q340A and T711S (AAV5-17Mut-FIX), the amount of protein produced increased by 1.36 times from 628.67 ng/ml to 854.99 ng/ml compared to the control AAV5 with the wild-type capsid protein VP1 (AAV5-NullMut- FIX).

In the simultaneous presence of the mutations S2A, S420F and T711S (AAV5-18Mut-FIX), the amount of protein produced increased by 1.19 times from 628.67 ng/ml to 745.00 ng/ml compared to the control AAV5 with the wild-type capsid protein VP1 (AAV5-NullMut- FIX).

In the simultaneous presence of the mutations S2A, S680A and T711S (AAV5-19Mut-FIX), the amount of protein produced increased by 1.52 times from 628.67 ng/ml to 955.57 ng/ml compared to the control AAV5 with the wild-type capsid protein VP1 (AAV5-NullMut- FIX).

In the simultaneous presence of the mutations S2A, T721V and T711S (AAV5-20Mut-FIX), the amount of protein produced increased by 1.79 times from 628.67 ng/ml to 1125.00 ng/ml compared to the control AAV5 with the wild-type capsid protein VP1 (AAV5-NullMut- FIX).

In the presence of the single mutation G226A (AAV5-21Mut-FIX), the amount of protein being produced increased by 1,56 times from 653,19 ng/ml to 1020,59 ng/ml as compared to control AAV5 with wild-type capsid protein VP1 (AAV5-NullMut- FIX).

In the presence of the single mutation D286E (AAV5-22Mut-FIX), the amount of protein being produced increased by 1,63 times from 653,19 ng/ml to 1063,78 ng/ml as compared to control AAV5 with wild-type capsid protein VP1 (AAV5-NullMut- FIX).

In the presence of the single mutation L341Y (AAV5-23Mut-FIX), the amount of protein being produced increased by 1,95 times from 653,19 ng/ml to 1271,00 ng/ml as compared to control AAV5 with wild-type capsid protein VP1 (AAV5-NullMut- FIX).

In the presence of the single mutation C387V (AAV5-24Mut-FIX), the amount of protein being produced increased by 1,81 times from 653,19 ng/ml to 1184,04 ng/ml as compared to control AAV5 with wild-type capsid protein VP1 (AAV5-NullMut- FIX).

In the presence of the single mutation Q421H (AAV5-25Mut-FIX), the amount of protein being produced increased by 1,92 times from 653,19 ng/ml to 1254,12 ng/ml as compared to control AAV5 with wild-type capsid protein VP1 (AAV5-NullMut- FIX).

In the presence of the single mutation P466T (AAV5-26Mut-FIX), the amount of protein being produced increased by 1,73 times from 653,19 ng/ml to 1130,02 ng/ml as compared to control AAV5 with wild-type capsid protein VP1 (AAV5-NullMut- FIX).

In the presence of the single mutation S594Q (AAV5-27Mut-FIX), the amount of protein being produced increased by 1,88 times from 653,19 ng/ml to 1228,00 ng/ml as compared to control AAV5 with wild-type capsid protein VP1 (AAV5-NullMut- FIX).

In the presence of the single mutation T614L (AAV5-28Mut-FIX), the amount of protein being produced increased by 1,40 times from 653,19 ng/ml to 915,81 ng/ml as compared to control AAV5 with wild-type capsid protein VP1 (AAV5-NullMut- FIX).

In the presence of the single mutation N679K (AAV5-29Mut-FIX), the amount of protein being produced increased by 1,21 times from 653,19 ng/ml to 790,36 ng/ml as compared to control AAV5 with wild-type capsid protein VP1 (AAV5-NullMut- FIX).

In the presence of the single mutation P723V (AAV5-30Mut-FIX), the amount of protein being produced increased by 1,35 times from 653,19 ng/ml to 881,81 ng/ml as compared to control AAV5 with wild-type capsid protein VP1 (AAV5-NullMut- FIX).

In the presence of the single mutation V431Y (AAV5-31Mut-FIX), the amount of protein being produced increased by 1,34 times from 653,19 ng/ml to 875,27 ng/ml as compared to control AAV5 with wild-type capsid protein VP1 (AAV5-NullMut- FIX).

In the presence of the single mutation A616D (AAV5-32Mut-FIX), the amount of protein being produced increased by 1,11 times from 653,19 ng/ml to 727,75 ng/ml as compared to control AAV5 with wild-type capsid protein VP1 (AAV5-NullMut- FIX).

In the presence of the single mutation W683R (AAV5-33Mut-FIX), the amount of protein being produced increased by 1,57 times from 653,19 ng/ml to 1025,51 ng/ml as compared to control AAV5 with wild-type capsid protein VP1 (AAV5-NullMut- FIX).

In the presence of the single mutation S222A (AAV5-34Mut-FIX), the amount of protein being produced increased by 1,78 times from 653,19 ng/ml to 1162,68 ng/ml as compared to control AAV5 with wild-type capsid protein VP1 (AAV5-NullMut- FIX).

In the presence of the single mutation R285L (AAV5-35Mut-FIX), the amount of protein being produced increased by 1,47 times from 653,19 ng/ml to 960,19 ng/ml as compared to control AAV5 with wild-type capsid protein VP1 (AAV5-NullMut- FIX).

In the presence of the single mutation Q340A (AAV5-36Mut-FIX), the amount of protein being produced increased by 1,64 times from 653,19 ng/ml to 1071,23 ng/ml as compared to control AAV5 with wild-type capsid protein VP1 (AAV5-NullMut- FIX).

In the presence of the single mutation S420F (AAV5-37Mut-FIX), the amount of protein being produced increased by 1,24 times from 653,19 ng/ml to 809,96 ng/ml as compared to control AAV5 with wild-type capsid protein VP1 (AAV5-NullMut- FIX).

In the presence of the single mutation S680A (AAV5-38Mut-FIX), the amount of protein being produced increased by 1,37 times from 653,19 ng/ml to 894,87 ng/ml as compared to control AAV5 with wild-type capsid protein VP1 (AAV5-NullMut- FIX).

In the presence of the single mutation T721V (AAV5-39Mut-FIX), the amount of protein being produced increased by 1,96 times from 653,19 ng/ml to 1280,25 ng/ml as compared to control AAV5 with wild-type capsid protein VP1 (AAV5-NullMut- FIX).

### Example 16. Increasing the production of target protein encoded by transgene following cell transduction with rAAV9-based products comprising mutations in wild-type rAAV9 capsid protein VP1.

Experimental design:
The CHO-K1-S cells were plated into the wells of 12-well plates. Seeding was made into the following growth medium: DMEM/F12 supplemented with glutamine, glucose content was 4.5 g/l, 5% bovine serum. Cell seeding density was 10,000 cell/cm2. During the transduction run, pre-prepared cells were transduced at MOI of 200,000 vg/cell. All samples were run in triplicates. Intact cells were used as a negative control.

The amount of FIX protein in the culture liquid 7 days post transduction was assessed using the Human Factor IX ELISA Kit. We used a 1:25 dilution of the samples. The procedure was carried out according to the manufacturer's instructions.

The authors of the invention have surprisingly found that the presence of one or more mutations selected from the group: S232T, D297E, Q351K, Q351R, C396V, D433Y, L444R, Y478F, G604N, G627K, T625L, T625V, S692T, T733V, A427R, M635D, W695R, R296L, Q351A, Y395F, R434L, N691A in wild-type rAAV9 capsid protein VP1 caused a significant increase in the production of FIX protein following transduction of CHO-K1-S cells with the rAAV vectors with the above mutations. For example, enzyme-linked immunoassay (EIA) revealed increased amount of FIX protein in the culture medium 7 days following the transduction of CHO-K1-S cells with rAAV products with wild-type AAV9 capsid protein VP1 bearing one or more mutations selected from the group: S232T, D297E, Q351K, Q351R, C396V, D433Y, L444R, Y478F, G604N, G627K, T625L, T625V, S692T, T733V, A427R, M635D, W695R, R296L, Q351A, Y395F, R434L, N691A (Fig. 14).

In the presence of the mutation S232T (AAV9-01Mut- FIX), the amount of protein produced increased by 1,18 times from 947,5 ng/ml to 1118,05 ng/ml as compared to control AAV9 with wild-type capsid protein VP1 (AAV9-NullMut- FIX).

In the presence of the mutation D297E (AAV9-02Mut- FIX), the amount of protein produced increased by 1,20 times from 947,5 ng/ml to 1137,00 ng/ml as compared to control AAV9 with wild-type capsid protein VP1 (AAV9-NullMut- FIX).

In the presence of the mutation Q351K (AAV9-03Mut- FIX), the amount of protein produced increased by 1,26 times from 947,5 ng/ml to 1195,00 ng/ml as compared to control AAV9 with wild-type capsid protein VP1 (AAV9-NullMut- FIX).

In the presence of the mutation Q351R (AAV9-04Mut- FIX), the amount of protein produced increased by 1,22 times from 947,5 ng/ml to 1157,17 ng/ml as compared to control AAV9 with wild-type capsid protein VP1 (AAV9-NullMut- FIX).

In the presence of the mutation C396V (AAV9-05Mut- FIX), the amount of protein produced increased by 1,64 times from 947,5 ng/ml to 1554,17 ng/ml as compared to control AAV9 with wild-type capsid protein VP1 (AAV9-NullMut- FIX).

In the presence of the mutation D433Y (AAV9-06Mut- FIX), the amount of protein produced increased by 1,17 times from 947,5 ng/ml to 1110,50 ng/ml as compared to control AAV9 with wild-type capsid protein VP1 (AAV9-NullMut- FIX).

In the presence of the mutation L444R (AAV9-07Mut- FIX), the amount of protein produced increased by 1,20 times from 947,5 ng/ml to 1141,50 ng/ml as compared to control AAV9 with wild-type capsid protein VP1 (AAV9-NullMut- FIX).

In the presence of the mutation Y478F (AAV9-08Mut- FIX), the amount of protein produced increased by 2,01 times from 947,5 ng/ml to 1908,50 ng/ml as compared to control AAV9 with wild-type capsid protein VP1 (AAV9-NullMut- FIX).

In the presence of the mutation G604N (AAV9-09Mut- FIX), the amount of protein produced increased by 1,26 times from 947,5 ng/ml to 1197,67 ng/ml as compared to control AAV9 with wild-type capsid protein VP1 (AAV9-NullMut- FIX).

In the presence of the mutation G627K (AAV9-10Mut- FIX), the amount of protein produced increased by 1,23 times from 947,5 ng/ml to 1166,67 ng/ml as compared to control AAV9 with wild-type capsid protein VP1 (AAV9-NullMut- FIX).

In the presence of the mutation T625L (AAV9-11Mut- FIX), the amount of protein produced increased by 1,77 times from 947,5 ng/ml to 1677,50 ng/ml as compared to control AAV9 with wild-type capsid protein VP1 (AAV9-NullMut- FIX).

In the presence of the mutation T625V (AAV9-12Mut- FIX), the amount of protein produced increased by 1,83 times from 947,5 ng/ml to 1736,67 ng/ml as compared to control AAV9 with wild-type capsid protein VP1 (AAV9-NullMut- FIX).

In the presence of the mutation S692T (AAV9-13Mut- FIX), the amount of protein produced increased by 1,55 times from 947,5 ng/ml to 1464,83 ng/ml as compared to control AAV9 with wild-type capsid protein VP1 (AAV9-NullMut- FIX).

In the presence of the mutation T733V (AAV9-14Mut- FIX), the amount of protein produced increased by 1,55 times from 947,5 ng/ml to 1469,50 ng/ml as compared to control AAV9 with wild-type capsid protein VP1 (AAV9-NullMut- FIX).

In the presence of the mutation A427R (AAV9-15Mut- FIX), the amount of protein produced increased by 1,65 times from 947,5 ng/ml to 1563,38 ng/ml as compared to control AAV9 with wild-type capsid protein VP1 (AAV9-NullMut- FIX).

In the presence of the mutation M635D (AAV9-16Mut- FIX), the amount of protein produced increased by 1,70 times from 947,5 ng/ml to 1607,00 ng/ml as compared to control AAV9 with wild-type capsid protein VP1 (AAV9-NullMut- FIX).

In the presence of the mutation W695R (AAV9-17Mut- FIX), the amount of protein produced increased by 2,32 times from 947,5 ng/ml to 2201,67 ng/ml as compared to control AAV9 with wild-type capsid protein VP1 (AAV9-NullMut- FIX).

In the presence of the mutation R296L (AAV9-18Mut- FIX), the amount of protein produced increased by 1,23 times from 947,5 ng/ml to 1161,83 ng/ml as compared to control AAV9 with wild-type capsid protein VP1 (AAV9-NullMut- FIX).

In the presence of the mutation Q351A (AAV9-19Mut- FIX), the amount of protein produced increased by 1,48 times from 947,5 ng/ml to 1402,30 ng/ml as compared to control AAV9 with wild-type capsid protein VP1 (AAV9-NullMut- FIX).

In the presence of the mutation Y395F (AAV9-20Mut- FIX), the amount of protein produced increased by 1,21 times from 947,5 ng/ml to 1145,00 ng/ml as compared to control AAV9 with wild-type capsid protein VP1 (AAV9-NullMut- FIX).

In the presence of the mutation R434L (AAV9-21Mut- FIX), the amount of protein produced increased by 1,54 times from 947,5 ng/ml to 1456,83 ng/ml as compared to control AAV9 with wild-type capsid protein VP1 (AAV9-NullMut- FIX).

In the presence of the mutation N691A (AAV9-22Mut- FIX), the amount of protein produced increased by 2,33 times from 947,5 ng/ml to 2203,33 ng/ml as compared to control AAV9 with wild-type capsid protein VP1 (AAV9-NullMut- FIX).

## Claims

1. A method for producing a modified AAV capsid, comprising:
a) determining amino acids in capsomere protein of the modified AAV, which are located in the interaction interface region of adjacent pentameric subunits of the capsid;
b) aligning structurally the modified AAV capsid to the template capsid to determine the pairwise correspondence between each amino acid of the modified AAV from the interaction interface region between adjacent pentameric subunits and the amino acid closest thereto of the template capsid, as a structural analogue of the initial amino acid that is considered a potential substitution,
wherein the template capsid is understood to mean a structurally similar capsid of a virus selected from the family Parvoviridae, which is other than AAV;
c) comparing pairwisely amino acid residues, determined in step b), of the modified AAV capsid and the template capsid to identify structural differences between the modified AAV capsid and the template capsid in the interaction interface region of adjacent pentameric subunits of the capsid;
d) selecting positions for mutagenesis, wherein the amino acid residues for mutagenesis are located in the interaction interface region of adj acent pentameric subunits of the modified AAV capsid and have a structural difference between the modified AAV capsid and the template capsid in the interaction interface region of adjacent pentameric subunits of the capsid;
e) selecting an amino acid residue for mutagenesis at position selected in step d) using one of the following principles:
- substitution of the initial amino acid in the capsomere protein of the modified AAV for an amino acid that has a greater volume, other than cysteine and methionine;
- substitution of the initial amino acid in the capsomere protein of the modified AAV for an amino acid that has an increased or decreased number of polar contacts between the capsomeres, other than cysteine and methionine;
f) introducing one or more amino acid substitutions selected in steps d)-e) in the interaction interface region of adj acent pentameric subunits of the modified AAV capsid.

2. The method for producing the modified AAV capsid according to claim 1, further comprising checking the modified AAV capsids produced in step f) with one or more amino acid substitutions for the presence of one or more improved properties as compared to AAV capsid free of these modifications, wherein the one or more improved properties are selected from the group:
- increased efficiency of cell transduction,
- increased production of target protein,
- increased efficiency of generation due to highly efficient production (assembly) of encapsidated viral vectors based on recombinant adeno-associated virus (rAAV).

3. The method for producing the modified AAV capsid according to claim 1, further comprising checking the modified AAV capsids produced in step f) for the presence of one or more improved properties as compared to AAV capsid free of these modifications and selecting modified AAV capsids that have one or more improved properties as compared to AAV capsid free of these modifications, wherein the one or more improved properties are selected from the group:
- increased efficiency of cell transduction,
- increased production of target protein,
- increased efficiency of generation due to highly efficient production (assembly) of encapsidated viral vectors based on recombinant adeno-associated virus (rAAV).

4. The method for producing an AAV capsid according to claim 1, wherein the modified AAV capsid is selected from the group comprising: human AAV, simian AAV, or avian AAV.

5. The method for producing an AAV capsid according to claim 1, wherein the modified AAV capsid is selected from the group comprising the following AAV serotypes: AAV1, AAV2, AAV3, AAV4, AAV5, AAV6, AAV7, AAV8, AAV9, AAV10 AAV11, AAV12, AAV13, AAV14, AAV15, or AAV16.

6. The method for producing an AAV capsid according to claim 1, wherein the structurally similar template capsid is selected from the group comprising: parvovirus B19, human bocavirus 1 (HBoV1), bovine parvovirus (BVP).

7. A modified AAV capsid for producing viral vectors based on recombinant adeno-associated virus, said modified AAV capsid being produced by a method comprising:
a) determining amino acids in capsomere protein of the modified AAV, which are located in the interaction interface region of adjacent pentameric subunits of the capsid;
b) aligning structurally the modified AAV capsid with the template capsid to determine the pairwise correspondence between each amino acid of the modified AAV from the interaction interface region between adjacent pentameric subunits and the nearest amino acid of the template capsid, as a structural analogue of the original amino acid that is considered a potential substitution,
wherein the template capsid is understood to mean a structurally similar capsid of a virus selected from the family Parvoviridae, which is other than AAV;
c) comparing pairwisely amino acid residues, determined in step b), of the AAV capsid and the template capsid to identify structural differences between the modified AAV capsid and the template capsid in the interaction interface region of adjacent pentameric subunits of the capsid;
d) selecting positions for mutagenesis, wherein the amino acid residues for mutagenesis are located in the interaction interface region of adj acent pentameric subunits of the modified AAV capsid and have a structural difference between the modified AAV capsid and the template capsid in the interaction interface region of adjacent pentameric subunits of the capsid;
e) selecting an amino acid residue for mutagenesis at position selected in step d) using one of the following principles:
- substitution of the initial amino acid in the capsomere protein of the modified AAV for an amino acid that has a greater volume, other than cysteine and methionine;
- substitution of the initial amino acid in the capsomere protein of the modified AAV for an amino acid that has an increased or decreased number of polar contacts between the capsomeres, other than cysteine and methionine;
f) introducing one or more amino acid substitutions selected in steps d)-e) in the interaction interface region of adj acent pentameric subunits of the modified AAV capsid;
and, if necessary, further comprising
g) checking the modified AAV capsids produced in step f) for the presence of one or more improved properties as compared to AAV capsid free of these modifications and selecting modified AAV capsids that have one or more improved properties as compared to AAV capsid free of these modifications, wherein the one or more improved properties are selected from the group:
- increased efficiency of cell transduction,
- increased production of target protein,
- increased efficiency of generation due to highly efficient production (assembly) of encapsidated viral vectors based on recombinant adeno-associated virus (rAAV).

8. The modified AAV capsid according to claim 7, wherein the modified AAV capsid is selected from the group comprising: human AAV, simian AAV, or avian AAV.

9. The modified AAV capsid according to claim 7, wherein the modified AAV capsid is selected from the group comprising the following AAV serotypes: AAV1, AAV2, AAV3, AAV4, AAV5, AAV6, AAV7, AAV8, AAV9, AA V10 AAV11, AAV12, AAV13, AAV14, AAV15, or AAV16.

10. A modified AAV capsid for producing viral vectors based on recombinant adeno-associated virus, comprising a modified AAV capsid protein VP1 having an amino acid sequence that is selected from the group: SEQ ID No: 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, or 368.

11. The modified AAV capsid according to claim 10, comprising:
a) a modified AAV capsid protein VP1 having an amino acid sequence that is selected from the group: SEQ ID No: 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, or 368;
b) a modified AAV capsid protein VP2 corresponding to protein VP1 thereof, said modified AAV capsid protein VP2 having an amino acid sequence that is selected from the group: SEQ ID No: 16, 22, 28, 34, 40, 46, 52, 58, 64, 70, 76, 82, 88, 94, 100, 106, 112, 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, or 370;
c) a modified AAV capsid protein VP3 corresponding to protein VP1 thereof, said modified AAV capsid protein VP3 having an amino acid sequence that is selected from the group: SEQ ID No: 18, 24, 30, 36, 42, 48, 54, 60, 66, 72, 78, 84, 90, 96, 102, 108, 114, 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, or 372.

12. An isolated nucleic acid encoding the modified capsid according to claims 7 to 11.

13. A vector based on recombinant adeno-associated virus for delivering to a subject a heterologous nucleic acid sequence, said vector comprising:
1) the modified AAV capsid according to any one of claims 7 to 11, and
2) a heterologous nucleic acid sequence comprising regulatory sequences that promote the expression of the product encoded by the heterologous nucleic acid sequence, in the target cells.

14. The vector based on recombinant adeno-associated virus according to claim 13, wherein the product of expression of the heterologous nucleic acid sequence is a therapeutic polypeptide or reporter polypeptide.
